(19) Europäisches Patentamt
European Patent Office
Office européen des brevets

(11) Publication number: **0 231 622 B1**

# (12) EUROPEAN PATENT SPECIFICATION

(45) Date of publication of patent specification: **24.08.94**

(51) Int. Cl.5: **C07D 513/04**, C07D 471/04, C07D 487/04, A61K 31/425, A61K 31/415, A61K 31/435, A61K 31/54, C07D 207/22, C07D 211/72, C07D 277/18, C07D 279/06

(21) Application number: **86309672.3**

(22) Date of filing: **11.12.86**

## (54) Inhibition of the 5-lipoxygenase pathway.

(30) Priority: **12.12.85 US 808407**
**12.12.85 US 808595**
**28.04.86 US 856875**
**28.04.86 US 856928**

(43) Date of publication of application:
**12.08.87 Bulletin 87/33**

(45) Publication of the grant of the patent:
**24.08.94 Bulletin 94/34**

(84) Designated Contracting States:
**AT BE CH DE ES FR GB GR IT LI LU NL SE**

(56) References cited:
**EP-A- 0 000 353**
**EP-A- 0 011 111**
**EP-A- 0 019 688**
**EP-A- 0 020 858**
**EP-A- 0 203 787**
**DE-A- 2 709 639**
**DE-A- 2 742 725**
**DE-B- 1 900 974**
**FR-A- 2 365 572**
**US-A- 3 364 112**
**US-A- 4 153 706**
**US-A- 4 175 127**
**US-A- 4 186 205**

(73) Proprietor: **SMITHKLINE BEECHAM CORPORATION**
**One Franklin Plaza**
**P.O. Box 7929**
**Philadelphia Pennsylvania 19103 (US)**

(72) Inventor: **Bender, Paul Elliot**
**504 Lilac Lane**
**Cherry Hill, NJ 08003 (US)**
Inventor: **Hanna, Nabil**
**507 Kent Place**
**Berwyn, PA 19312 (US)**
Inventor: **Sarau, Henry Martin**
**1880 Cindy Lane**
**Hatfield, PA 19440 (US)**
Inventor: **Gleason, John Gerald**
**8 Heron Hill Drive**
**Downingtown, PA 19335 (US)**
Inventor: **Lantos, Ivan**
**1447 Boxwood Drive**
**Blackwood, NJ 08012 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 231 622 B1

**CHEMICAL ABSTRACTS, vol. 100, no. 3, 16th January 1984, page 26, column 2, abstract no. 17343v, Columbus, Ohio, US; I. LANTOS et al.: "Antiinflammatory activity of 5,6-diaryl-2,3-dihydroimidazo(2,1-b)thiazoles. Isomeric 4-pyridyl and 4-substituted-phenyl derivatives", & J. MED. DHEM. 1984, 27(1), 72-5**

**CHEMICAL ABSTRACTS, vol. 97, no. 19, 8th November 1982, page 708, abstract no. 162885x, Columbus, Ohio, US; A. ANDREANI et al.: "5,6-Disubstituted imidazo(2,1-b)thiazoles as potential antiinflammatory agents", & EUR. J. MED. CHEM. - CHIM. THER. 1982, 17(3), 271-4**

Inventor: **Griswold, Don Edgar**
**205 Lower Valley Road**
**North Wales, PA 19454 (US)**
Inventor: **Razgaitis, Kazys Arunas**
**7 Arthur road**
**Rosemont, PA 19010 (US)**
Inventor: **Shilcrat, Susan Cathy**
**2 East Amherst Road**
**Bala Cynwyd, PA 19004 (US)**

(74) Representative: **Waters, David Martin et al**
**SMITHKLINE BEECHAM,**
**Corporate Patents,**
**Mundells**
**Welwyn Garden City, Hertfordshire AL7 1EY**
**(GB)**

## Description

This invention relates to novel compounds, pharmaceutical compositions and their use in the manufacture of a medicament for inhibiting the 5-lipoxygenase pathway of arachidonic acid metabolism in an animal in need thereof.

This invention relates to compounds wherein a diaryl-substituted imidazole is fused to a thiazole, pyrrole, thiazine, or pyridine ring. Related compounds have been disclosed in EP-A-19668, FR-A-2365572, US-A-4175127, EP-A-353, DE-A-2742725, DE-A-2709639, Chem. Abs. 100 : 17343v, EP-A-11117, US-A-4186205, US-A-4153706, DE-B-1900974, US-A-3364112 and EP-A-203787.

The compounds designated herein as Formula (I) include all of the compounds of Formulae (IA), (IB), and (IC). The designation of Formula (I) is employed herein primarily in disclosure of processes which can be used in the preparation of any of the compounds of Formulae (IA), (IB), and (IC).

Those compounds designated herein as Formula (IA) are active as inhibitors of the 5-lipoxygenase pathway of arachidonic acid metabolism, and some are also useful as intermediates in the preparation of such active compounds. These compounds have chemical structures which are not necessarily novel. Some of the compounds of Formula (IA) are known in the art, but their utility for inhibiting the 5-lipoxygenase pathway is not known.

Those compounds designated as Formula (IB) are novel compounds. Some of the compounds of Formula (IB) are active as 5-lipoxygenase inhibitors, some are useful as intermediates in the preparation of such compounds, and some have both of the above uses.

All of those compounds designated as Formula (IC) are both novel compounds and active as 5-lipoxygenase inhibitors. Some of the compounds of Formula (IC) are also useful as intermediates in the preparation of other compounds which are active as 5-lipoxygenase pathway inhibitors.

The compounds designated herein as Formulae (E), (F), (G), (H), (J) and (K), are novel compounds which are intermediates useful in the preparation of the compounds of Formula (I).

This invention relates to novel compounds of Formulae (IB), (J), (K), (E), (F), (G), and (H). This invention further relates to pharmaceutical compositions of the compounds of Formula (IC), and compounds of Formula (IC) for use in treating arthritis. This invention further relates to use of the compounds of Formula (IA) in the manufacature of a medicament for treating 5-lipoxygenase mediated diseases.

More particularly, this invention relates to novel compounds of the Formula (IB) represented by the structure:

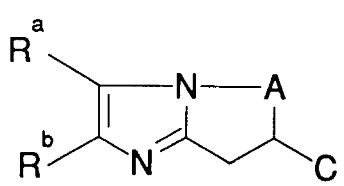

**Formula (IB)**

wherein:
A is methylene or 1,2-ethanediyl optionally substituted by methyl, ethyl or gem-dimethyl,
C is H, methyl, ethyl or gem-dimethyl; and

I. one of $R^a$ or $R^b$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, disubstituted phenyl wherein said substituents are independently selected from $C_{1-4}$alkyl or $C_{1-3}$alkoxy or the disubstituents together form a methylenedioxy group; and the other of $R^a$ or $R^b$ is selected from:

1) pyridyl;
2) phenyl;
3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);
4) disubstituted phenyl wherein said substituents are independently selected from $C_{1-4}$alkyl or $C_{1-3}$alkoxy or the disubstituents together form a methylenedioxy group; or

5) 3,4,5-trimethoxyphenyl;

provided that:

(a) when either $R^a$ or $R^b$ is 4-fluorophenyl, the other must be selected from pyridyl or phenyl substituted in the 4-position with 2,2,2-trihaloethoxy, or prop-2-en-1-oxy;

(b) $R^a$ and $R^b$ are not both phenyl substituted in the 2,3,5 or 6 position with $C_{1-3}$alkylamino; $C_{1-3}$dialkylamino; or 4-N-(azacyclo $C_{5-6}$alkyl);

(c) when either of $R^a$ or $R^b$ is disubstituted phenyl the other must be 4-pyridyl;

(d) when either of $R^a$ or $R^b$ is cyanophenyl the other must be cyanophenyl or 4-pyridyl;

(e) $R^a$ and $R^b$ are not both 3-pyridyl or 2-pyridyl, or

II. one of $R^a$ or $R^b$ is pyridyl and the other is selected from:

(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-($C_{1-3}$alkyl)-($C_{1-3}$alkanamido), N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or hydroxy, or the disubstituents together form a methylenedioxy group; or

(c) disubstituted phenyl wherein said substituents are not the same and are independently selected from halo, $C_{1-3}$alkylamino, nitro, N-($C_{1-3}$alkyl)-N-($C_{1-3}$alkanamido), $C_{1-3}$dialkylamino, amino or N-(azacyclo $C_{5-6}$alkyl); or

substituents must be $C_{1-3}$alkoxy, hydroxy, 2,2,2-trihaloethoxy or prop-2-en-1-oxy and the other substitutent is independently selected from halo, $C_{1-3}$alkylamino, N-($C_{1-3}$alkyl)-$C_{1-3}$alkanamido), $C_{1-3}$dialkylamino, amino, or N-(azacyclo $C_{5-6}$alkyl);

provided that:

(1) when $R^a$ is 2 or 3-pyridyl and $R^b$ is monosubstituted phenyl, the substituent is selected from other than bromo, iodo, N-($C_{1-3}$alkyl)-($C_{1-3}$alkanamido), $C_{1-3}$alkylthio, $C_{1-3}$alkylsulfinyl or $C_{1-3}$alkylsulfonyl;

(2) when $R^a$ is 2- or 3-pyridyl and $R^b$ is disubstituted phenyl, the disubstituents are both selected from other than bromo, iodo, amino, nitro, hydroxy, or ($C_{1-3}$alkyl)-($C_{1-3}$alkanamido);

(3) when $R^b$ is 2-, 3- or 4-pyridyl and $R^a$ is monosubstituted phenyl, the substituents are selected from other than bromo, iodo, N-($C_{1-3}$alkyl)-($C_{1-3}$alkanamido), $C_{1-3}$alkylthio, $C_{1-3}$alkylsulfinyl, or $C_{1-3}$alkylsulfonyl; and

(4) when $R^b$ is 2, 3 or 4-pyridyl and $R^a$ is disubstituted phenyl, the substituents are both selected from other than bromo, iodo, amino, nitro, hydroxy, N-($C_{1-3}$alkyl)-N-($C_{1-3}$alkanamido),

or a pharmaceutically acceptable salt thereof.

This invention also relates to a compound of the formula (IC) and to a pharmaceutical composition comprising a pharmaceutically acceptable carrier or diluent and an effective, non-toxic 5-lipoxygenase pathway inhibiting amount of a compound of Formula (IC) represented by the structure:

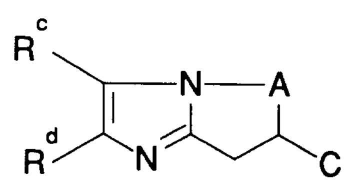

**Formula (IC)**

wherein

A is methylene or 1,2-ethanediyl optionally substituted by methyl, ethyl or gem-dimethyl, and C is H, methyl, ethyl or gem-dimethyl;

I. one of $R^c$ or $R^d$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, or disubstituted phenyl wherein said substituents are independently selected from $C_{1-3}$alkyl or $C_{1-3}$alkoxy or the disubstituents together form a methylendioxy group; and the other of $R^c$ or $R^d$ is selected from :

1) pyridyl;

2) phenyl;

3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);

4) disubstituted phenyl wherein said substituents are independently selected from $C_{1-4}$alkyl or $C_{1-3}$alkoxy or the disubstituents together form a methylenedioxy group; or

5) 3,4,5-trimethoxyphenyl;

provided that:

(a) when either $R^c$ or $R^d$ is 4-fluorophenyl, the other must be selected from pyridyl or phenyl substituted in the 4-position with 2,2,2-trihaloethoxy, or prop-2-en-1-oxy;

(b) $R^c$ and $R^d$ are both not phenyl substituted in the 2,3,5 or 6 position with $C_{1-3}$alkylamino; $C_{1-3}$dialkylamino; or 4-N-(azacyclo $C_{5-6}$alkyl);

(c) when either of $R^c$ or $R^d$ is disubstituted phenyl the other must be 4-pyridyl;

(d) when either of $R^c$ or $R^d$ is cyanophenyl the other must be cyanophenyl or 4-pyridyl;

(e) when $R^d$ is $C_{1-3}$alkanamido or N-($C_{1-3}$alkyl)-($C_{1-3}$alkanamido), $R^c$ must be 4-pyridyl;

(f) $R^c$ and $R^d$ are not both 3-pyridyl or 2-pyridyl; or

II. when one of $R^c$ or $R^d$ is pyridyl, the other is selected from:

(a) monosubstituted phenyl wherein said substitutent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or

(c) disubstituted phenyl wherein said substituents are not the same and are independently selected from $C_{1-3}$alkylamino, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo-$C_{5-6}$alkyl);

provided that:

(1) when $R^c$ is 2 or 3-pyridyl and $R^d$ is monosubstituted phenyl, the substituent is selected from other than bromo, iodo, $C_{1-3}$alkylthio, $C_{1-3}$alkylsulfinyl, or $C_{1-3}$alkylsulfonyl;

(2) when $R^c$ is 2- or 3-pyridyl and $R^d$ is disubstituted phenyl, the disubstituents are both selected from other than bromo or iodo;

(3) when $R^d$ is 2-, 3- or 4-pyridyl and $R^c$ is monosubstituted phenyl, the substituents are selected from other than bromo, iodo, $C_{1-3}$alkylthio, $C_{1-3}$alkylsulfinyl, or $C_{1-3}$alkylsulfonyl; and

(4) when $R^d$ is 2-, 3- or 4-pyridyl and $R^c$ is disubstituted phenyl, the substituents are both selected from other than bromo or iodo;

or a pharmaceutically acceptable salt thereof.

This invention also relates to a compound of (IC) or a pharmaceutically acceptable salt thereof for use in the treatment of rheumatoid arthritis in an animal.

This invention also relates to the use of a compound in the preparation of a medicament for the treatment of a 5-lipoxygenase pathway mediated disease other than, or in addition to, rheumatoid arthritis, rheumatism, inflammation, or any other cyclooxygenase mediated disease state, said compound being of the formula (IA) :

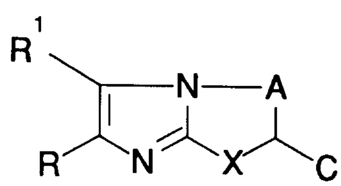

**Formula (IA)**

wherein :

A and C are as defined in Claim 1;

I. when X is $CH_2$ or $S(O)_n$ and n is 0, 1, or 2;

$R^1$ and R are independently selected from

(a) pyridyl, provided that when either or both of R and $R^1$ are pyridyl X is other then $CH_2$;

(b) phenyl;

(c) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);

(d) disubstituted phenyl wherein said substituents are independently selected from $C_{1-4}$alkyl or $C_{1-3}$alkoxy or the disubstituents together form a methylenedioxy group ;

(e) 3,4,5-trimethoxyphenyl;

or a pharmaceutically acceptable salt thereof provided that :

(1) when X is $CH_2$, both of R and $R^1$ are other than phenyl substituted in the 2 or 6 position with $C_{1-3}$alkylamino; $C_{1-3}$dialkylamino or N-(azacyclo $C_{5-6}$alkyl;

(2) when either of R or $R^1$ is cyanophenyl the other must be cyanophenyl or 4-pyridyl;

(3) when X is $S(O)_n$ and R or $R^1$ is phenyl substituted in the 2,3,5 or 6 position with $C_{1-3}$alkylamino; $C_{1-3}$dialkylamino or N-(azacyclo $C_{5-6}$alkyl, the other must be 4-pyridyl; or

II. when X is $CH_2$ and one of R or $R^1$ is pyridyl, the other is selected from :

(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or

(c) disubstituted phenyl wherein said substituents are not the same and are independently selected from $C_{1-3}$alkylamino, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that :

(1) when $R^1$ is 2- or 3-pyridyl and R is monosubstituted phenyl, the substituent is selected from other than bromo, iodo, $C_{1-3}$alkylthio, $C_{1-3}$alkylsulfinyl, or $C_{1-3}$alkylsulfonyl;

(2) when $R^1$ is 2- or 3-pyridyl and R is disubstituted phenyl, the disubstituents are both selected from other than bromo or iodo;

(3) when R is 2-, 3-, or 4-pyridyl and $R^1$ is monosubstituted phenyl, the substituents are selected from other than bromo, iodo, $C_{1-3}$alkylthio, $C_{1-3}$alkylsulfinyl, or $C_{1-3}$alkylsulfonyl; and

(4) when R is 2-, 3- or 4-pyridyl and $R^1$ is disubstituted phenyl, the substituents are both selected from other than bromo or iodo;

or a hydrate thereof having a hydroxy group attached to the carbon atom to which R is attached wherein R is other than pyridyl, $R^1$ is 2- or 4-halo, $CF_3$ or cyano substituted phenyl and X is S;

or a pharmaceutically acceptable salt thereof.

Compounds of the Formula (J) are represented by the structure:

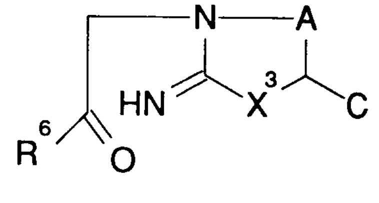

**Formula (J)**

wherein:

$X^3$ is S or $CH_2$;

A and C are as hereinbefore defined;

$R^6$ is selected from:

(a) phenyl or monosubstituted phenyl wherein said substituent is selected from $C_{1-3}$ alkanamido, $C_{1-3}$ dialkylamino, N-(azacyclo $C_{5-6}$alkyl), halo, cyano, $C_{1-3}$ alkoxy, $C_{1-3}$ alkylthio, $C_{1-4}$ alkyl, or $CF_3$;
(b) disubstituted phenyl wherein said substituents are independently selected from $C_{1-4}$ alkyl or $C_{1-3}$ alkoxy, or the disubstituents together form a methylenedioxy group; or
(c) 3,4,5-trimethoxyphenyl;

provided that when A is $CH_2$ and C is H, $R^6$ is other than phenyl or monosubstituted phenyl wherein said substituent is halo, $C_{1-3}$ alkoxy, $C_{1-4}$ alkyl, $CF_3$ or $C_{1-3}$ alkylthio,
or a pharmaceutically acceptable salt thereof.

Compounds of the Formula (K) are represented by the structure:

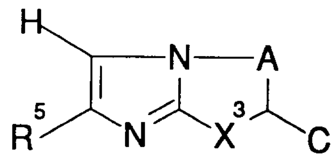

**Formula (K)**

wherein:

$X^3$ is $CH_2$ or S;
A and C are as hereinbefore defined,
$R^5$ is selected from :

(a) monosubstituted phenyl wherein said substituent is selected from $C_{1-3}$ alkanamido, N-($C_{1-3}$ alkyl)-($C_{1-3}$ alkanamido), cyano, $C_{1-3}$ dialkylamino, N-(azacyclo $C_{5-6}$ alkyl), halo, $C_{1-3}$ alkoxy, $C_{1-3}$ alkylthio, $C_{1-4}$ alkyl, $CF_3$, prop-2-en-1-oxy, 2,2,2-trihaloethoxy;
(b) disubstituted phenyl wherein said substituents are independently selected from $C_{1-4}$ alkyl or $C_{1-3}$ alkoxy, or the disubstituents together form a methylenedioxy group; or
(c) 3,4,5-trimethoxyphenyl;

provided that:

(i) when A is $CH_2$, C is H, and $X^3$ is S, $R^5$ is other than 2-, 3- or 4-methoxyphenyl, 2,4-dimethoxyphenyl, 4-hydroxyphenyl, 4-methylphenyl, 4-butylphenyl, 4-chlorophenyl or 4-bromophenyl; and
(ii) when A is $CH_2CH_2$, C is H, and $X^3$ is S, $R^5$ is other than 4-bromophenyl, 4-chlorophenyl or 4-methylphenyl; or a pharmaceutically acceptable salt thereof.

This invention also relates to a compound of the Formula (E) represented by the structure:

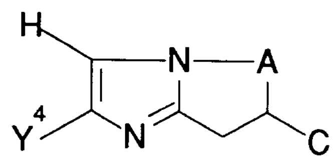

**Formula (E)**

wherein
A and C are as hereinbefore defined;
$Y^4$ is selected from:

(a) phenyl;
(b) monosubstituted phenyl, wherein said substituent is selected from halo, $C_{1-3}$alkoxy, $C_{1-3}$alkylthio, $C_{1-4}$alkyl, cyano, N-($C_{1-3}$alkyl)-($C_{1-3}$alkanamido), $C_{1-3}$alkanamido, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;
(c) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, hydroxy, or the disubstituents together form a methylenedioxy group;

(d) disubstituted phenyl wherein said substituents are not the same and are independently selected from halo, $C_{1-3}$alkylamino, $C_{1-3}$dialkylamino, amino, N-(azacyclo $C_{5-6}$alkyl), nitro, or N-($C_{1-3}$alkyl)-$C_{1-3}$alkanamido), or

(e) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that when A is $CH_2CH_2$, C is H, and $Y^4$ is other than 2,4-dimethoxyphenyl or 4-aminophenyl; or a pharmaceutically acceptable salt thereof.

This invention also relates to a compound of the Formula (F) represented by the structure :

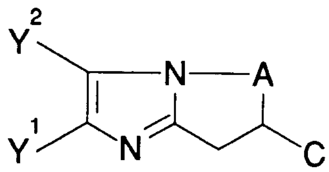

**Formula (F)**

wherein:
A and C are as hereibefore defined; and
$Y^2$ is 4-(1,4-dihydro)pyridyl substituted with N-($C_{1-8}$ alkanoyl), N-($C_{1-8}$alkoxycarbonyl), N-(benzoyl), N-(phenoxycarbonyl), N-(phenylacetyl) or N-(benzyloxycarbonyl);
$Y^1$ is selected from

(a) phenyl or monosubstituted phenyl wherein said substituent is selected from halo, $C_{1-3}$alkoxy, $C_{1-3}$alkylthio, $C_{1-4}$alkyl, $CF_3$, N-($C_{1-3}$alkyl)-$C_{1-3}$alkanamido, $C_{1-3}$dialkylamino, N-(azacyclo $C_{5-6}$alkyl), prop-2-en-1-oxy, 2,2,2-trihaloethoxy, $C_{1-3}$alkanamido or $C_{1-3}$dialkylamino;

(b) disubstituted phenyl wherein said substituents are the same and are selected from halo, $C_{1-3}$alkoxy, $C_{1-3}$dialkylamino, N-(azacyclo $C_{5-6}$alkyl), $C_{1-3}$alkylamino, 2,2,2-trihaloethoxy, or prop-2-en-1-oxy, hydroxy or the disubstituents together form a methylenedioxy group;

(c) disubstituted phenyl wherein said substituents are not the same and are independently selected from halo, nitro, $C_{1-3}$dialkylamino or N-($C_{1-3}$alkyl)-($C_{1-3}$alkanamido), N-(azacyclo $C_{5-6}$alkyl); or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

or a pharmaceutically acceptable salt thereof.

This invention also relates to a compound of the Formula (G) represented by the structure :

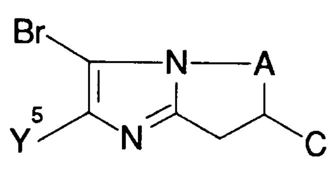

**Formula (G)**

wherein:
A and C are as hereinbefore defined;
$Y^5$ is selected from

(a) phenyl or monosubstituted phenyl wherein said substituent is selected from fluoro, chloro, $C_{1-3}$alkoxy, $C_{1-4}$alkyl, $C_{1-3}$dialkylamino, $CF_3$, $C_{1-3}$alkylamino, N-(azacyclo $C_{5-6}$alkyl), prop-2-en-1-oxy or 2,2,2-trihaloethoxy;

(b) disubstituted phenyl wherein said substituents are the same and are selected from fluoro, chloro, $C_{1-3}$alkoxy, $C_{1-3}$dialkylamino, N-(azacyclo $C_{5-6}$alkyl), 2,2,2-trihaloethoxy, or prop-2-en-1-oxy, or the

disubstituents together form a methylenedioxy group; or

(c) disubstituted phenyl wherein said substituents are not the same and are independently selected from fluoro, chloro, $C_{1-3}$alkylamino, $C_{1-3}$dialkylamino, or N-(azacyclo $C_{5-6}$alkyl); or

(d) disubstituted phenyl wherein one of said substituents must be $C_{1-3}$alkoxy, 2,2,2-trihaloethoxy or prop-2-en-1-oxy and the other substituent is independently selected from fluoro, chloro, $C_{1-3}$alkylamino, $C_{1-3}$dialkylamino, or N-(azacyclo $C_{5-6}$alkyl);

or a pharmaceutically acceptable salt thereof.

This invention relates to compounds of the Formula (H) represented by the structure:

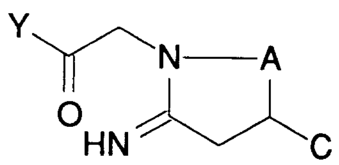

**Formula (H)**

wherein :

A and C are as hereinbefore defined;

Y is selected from :

(a) phenyl or monosubstituted phenyl, wherein said substituent is selected from halo, $C_{1-3}$alkoxy, $C_{1-3}$alkylthio, $C_{1-4}$alkyl, $C_{1-3}$alkanamido, $C_{1-3}$dialkylamino, $CF_3$, $C_{1-3}$alkylamino, N-(azacyclo $C_{5-6}$alkyl) or N-($C_{1-3}$alkyl)-($C_{1-3}$alkanamido);

(b) disubstituted phenyl wherein said substituents are the same and are selected from halo, $C_{1-3}$dialkylamino, N-(azacyclo $C_{5-6}$alkyl), or $C_{1-3}$alkoxy, or the disubstituents together form a methylenedioxy group;

(c) disubstituted phenyl wherein said substituents are not the same and are independently selected from halo, nitro, $C_{1-3}$alkanamido, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, N-($C_{1-3}$alkyl)-($C_{1-3}$alkanamido), $C_{1-3}$dialkylamino, or N-(azacyclo $C_{5-6}$alkyl);

provided that when A is $CH_2CH_2$ and C is H, Y is other than 2,4-dimethoxyphenyl or 4-aminophenyl;

or a pharmaceutically acceptable salt thereof.

As used herein, the term "Formula (I)" will refer to compounds represented by the structure:

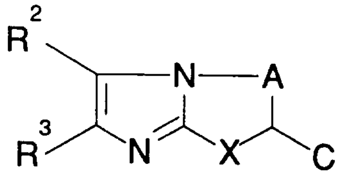

**Formula (I)**

wherein:

A and C are as hereinbefore defined;

X is $CH_2$ or $S(O)_n$ and n is 0, 1, or 2; $R^2$ and $R^3$ are independently selected from

(a) pyridyl,

(b) phenyl or monosubstituted phenyl wherein said substituent is selected from $C_{1-3}$ dialkylamino, $C_{1-3}$ alkylamino, N-(azacyclo $C_{5-6}$ alkyl), cyano, 2,2,2-trihaloethoxy, prop-2-en-1-oxy, N-($C_{1-3}$ alkyl)-($C_{1-3}$ alkanamido), $C_{1-3}$ alkanamido, amino, hydroxy, $C_{1-3}$ alkylthio, $C_{1-4}$ alkyl, halo, $CF_3$, $C_{1-3}$alkoxy, $C_{1-3}$ alkylsulfinyl, or $C_{1-3}$ alkylsulfonyl;

(c) disubstituted phenyl wherein said substituents are independently selected from $C_{1-4}$ alkyl, $C_{1-3}$ alkoxy, hydroxy, nitro, amino, halo, $C_{1-3}$ alkylamino, $C_{1-3}$ dialkylamino, N-(azacyclo $C_{5-6}$ alkyl), 2,2,2-trihaloethoxy, prop-2-en-1-oxy, or N-($C_{1-3}$ alkyl)-($C_{1-3}$alkanamido), or the disubstituents together form a methylenedioxy group; or

(d) 3,4,5-trimethoxyphenyl;

provided that:

(1) when $R^3$ is cyanophenyl, $R^2$ must be either cyanophenyl or 4-pyridyl;

or a pharmaceutically acceptable salt thereof.

All the compounds of Formula (I) can be prepared by the following synthetic routes 1 or 2.

## SYNTHETIC ROUTE 1

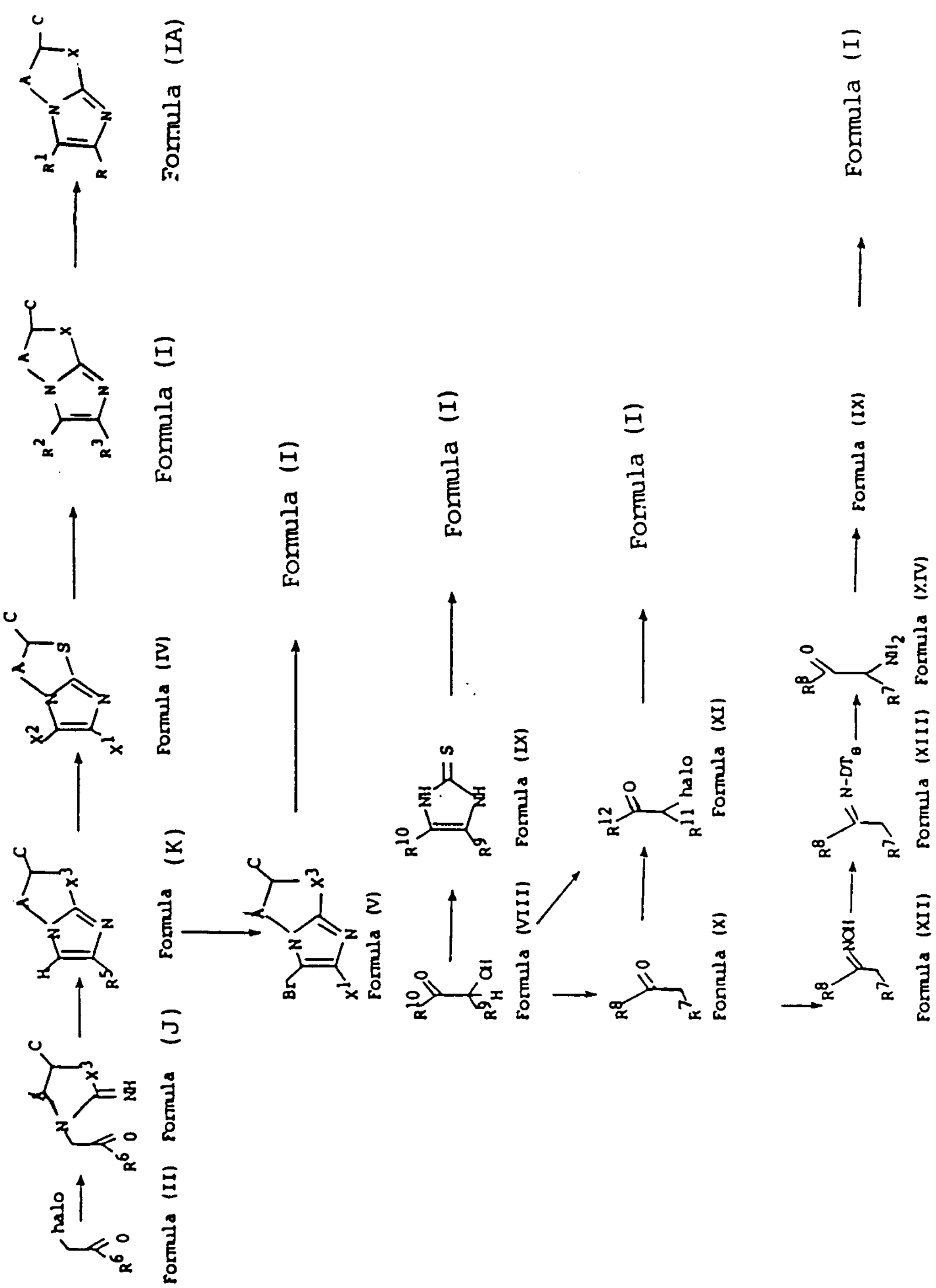

Formula (II) Formula (J) Formula (K) Formula (IV) Formula (I) Formula (IA)

Formula (V) Formula (I)

Formula (VIII) Formula (IX) Formula (I)

Formula (X) Formula (XI) Formula (I)

Formula (XII) Formula (XIII) Formula (XIV) Formula (IX) Formula (I)

SYNTHETIC ROUTE 2

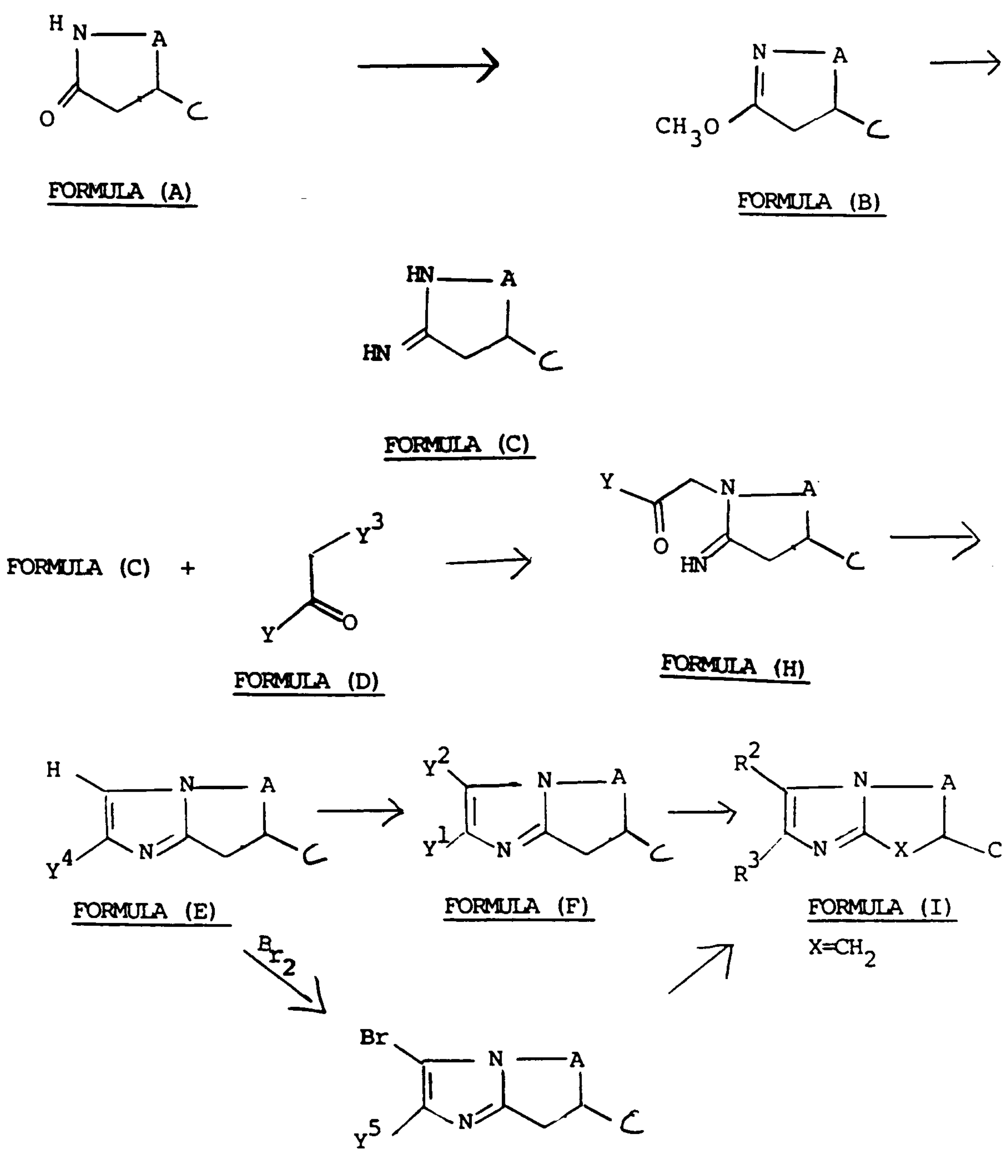


FORMULA (A)

FORMULA (B)

FORMULA (C)

FORMULA (C) +

FORMULA (D)

FORMULA (H)

FORMULA (E)

FORMULA (F)

FORMULA (I)

$X=CH_2$

FORMULA (G)

It will be apparent to one of skill in the art that all the compounds of Formula (IA), Formula (IB) and Formula (IC) are encompassed by the scope of Formula (I).

The required compounds necessary to produce the compounds of Formula (I) in accordance with synthetic route 1, i.e., the compounds of Formulas (II), (J), (K), (IV), (V), (VIII), (IX), (X), (XI), (XII), (XIII), (XIV) and (I), can be obtained from commercial sources or can be prepared by techniques described herein.

The compounds of Formula (I) can be prepared according to synthetic route 1 by means of the following reactions.

The compounds of Formula (K) are prepared in two steps from the corresponding Formula (II) and Formula (J) compounds. Formula (J) compounds are prepared by treatment of the appropriately substituted Formula (II) phenacyl halide with the correspondingly substituted 2-amino-3,4-dihydrothiazole or 2-amino-5,6-dihydro-4H-(1,3)thiazine in a chlorinated hydrocarbon or preferably an alcoholic solvent. It has been found that a polar solvent is also effective. The hydrohalide salt of the Formula (J) compound which can be isolated as a precipitate or oil on addition of a nonpolar cosolvent such as ether or hexane, is refluxed in water or aqueous alcohol until cyclodehydration is complete. Neutralization with aqueous base affords the corresponding Formula (K) compound. The substituted Formula (II) compounds are prepared from the corresponding acetophenones by treatment with bromine or alternatively prepared by acylating the corresponding monoor disubstituted benzene by Friedel Crafts reaction with 2-chloroacetyl chloride and $AlCl_3$.

The Formula (K) 6-arylimidazo[2,1-b]thiazole or thiazine analog is reacted with an excess of both the pyridine and the reactive acyl ester in a 2:1 ratio in an organic solvent in which the reactants are soluble and inert to prepare a compound of Formula (IV). One skilled in the art will recognize that the pyridine and acyl ester react to form the acylpyridinium reagent in situ. Optionally, the acylpyridinium reagent can be prepared separately in a solvent and then added to the solution of Formula (K) compound. Suitable solvents include methylene chloride, ethylene chloride, chloroform, carbon tetrachloride, tetrahydrofuran, ethyl ether, dioxane, toluene, or excess pyridine.

The reaction mixture is cooled during mixing of the reactants, preferably to a temperature between 5-20 °C, by use of an ice/water bath. The mixture is then allowed to stand briefly at ambient temperature, followed by refluxing until the reaction is complete. The reaction mixture is continually assayed using high pressure liquid chromatography (HPLC) or thin layer chromatography (TLC) on aliquots to ascertain if unreacted compound is present. If so, additional acyl pyridinium salt is introduced. Following the reaction, the resultant Formula (IV) compounds can be recovered from the reaction mixture and isolated by standard techniques.

The compound of Formula (K) in a nonpolar organic solvent is treated with bromine at room temperature. The resulting hydrobromide salt of Formula (V) is treated with aqueous base to afford the Formula (V) compound.

The Grignard reagent derived from a compound of Formula (V) is prepared by reacting a compound of Formula (V) with at least an equimolar amount of a $C_{1-5}$ alkyllithium reagent at a temperature of from about -80 °C to about -30 °C. Following the lithium-halogen exchange, from which the organolithium compound results, the magnesium halide etherate is added to the mixture in excess and reacted with the organolithium compound during which the reaction temperature can be permitted to rise to a temperature of about 0 °C, although a reaction temperature below about -10 °C is preferred.

Compounds of Formula (IV) can also be prepared by reacting the Grignard reagent derived from a Formula (V) compound with an excess of both pyridine and the selected reactive acyl ester in a solvent such as ethyl ether, or preferably, tetrahydrofuran. The Grignard reagent can be combined with the pyridine and the acyl ester in sequence, or the pyridinium salt can be prepared separately and then added to the solution of Grignard reagent. Preferably, the reaction is conducted by the addition of 2-6 moles of pyridine to each mole of the Grignard reagent, followed by addition of at least an equimolar amount of the acyl compound.

This reaction is conducted at a temperature of -10 °C or below. A minimum of about 5 mole percent of cuprous iodide (CuI), based on the molar quantity of pyridine present, is introduced to the reaction mixture to insure the ultimate addition of the Grignard reagent at the 4-position of the N-acyl-1,4-dihydropyridine compound.

The reaction mixture can be permitted to warm to a temperature of about 20 °C, but preferably no higher than about 15 °C. Aliquots of the reaction mixture are removed at intervals and assayed using HPLC or TLC to determine the presence of unreacted imidazo[2,1-b]thiazole or thiazine. The conditions of the reaction are otherwise standard for those pertaining to Grignard synthesis in general. Recovery and isolation can be effected by standard techniques.

The Formula (K) starting materials for the Formula (IV) compounds in which A is $CH_2CH_2$ (thiazine) can be prepared in an analogous manner by heating the corresponding 2-amino-5,6-dihydro-4H-1,3-thiazine with a 4-substituted bromoacetophenone of Formula (II) in a non-polar solvent such as benzene or chloroform, to form an intermediate which is then refluxed in water. It has also been found that a polar solvent can be used as well, and that the intermediate of Formula (J) may or may not be isolated prior to the refluxing step. The amino-thiazine starting material can be prepared by treating the appropriate 3-bromopropylamine with t-butyl isothiocyanate, followed by reflux with hydrobromic or hydrochloric acid.

Compounds of Formula (I) wherein X is S; A and C are as hereinbefore defined and $R^3$ and $R^2$ are independently mono-, dior tri-substituted phenyl, are prepared from either (a) the corresponding Formula (IX) compounds wherein $R^9$ or $R^{10}$ are the same as $R^a$ or $R^b$ in Formula (IB); or (b) the corresponding Formula (XI) compounds wherein $R^{11}$ or $R^{12}$ are the same as $R^a$ or $R^b$ in Formula (IB).

In accordance with synthetic route 1, in the first procedure, the Formula (IX) compound is treated with one equivalent of the corresponding 1,2-dihaloethane or 1,3-dihalopropane and potassium carbonate. The reactants are refluxed in N,N-dimethylformamide for about 3 hours, cooled, water is added, and the pH is adjusted to 11 with a base such as 10% aqueous sodium hydroxide. The precipitate is washed with water and dissolved in a solvent such as methylene chloride. The organic solution is washed with water, dried, and treated with charcoal. The solvent is removed and the crude product recrystallized or alternatively, it is chromatographed and the solvent evaporated in vacuo.

The requisite Formula (IX) compounds wherein $R^9$ and $R^{10}$ are independently selected from (a) phenyl or monosubstituted phenyl where said substituent is selected from $C_{1-3}$ dialkylamino, N-(azacyclo $C_{5-6}$ alkyl), $C_{1-3}$ alkoxy, 2,2,2-trihaloethoxy, prop-2-en-1-oxy, $C_{1-4}$ alkyl, $C_{1-3}$ alkylthio, N-$C_{1-3}$ alkanamido, N-($C_{1-3}$ alkyl)-($C_{1-3}$ alkanamido), halo, or $CF_3$, (b) disubstituted phenyl wherein said substitutents are independently selected from $C_{1-4}$ alkyl or $C_{1-3}$ alkoxy or the disubstitutents together form a methylenedioxy group, or (c) 3,4,5-trimethoxyphenyl are prepared by refluxing the corresponding Formula (VIII) compound with thiourea in dimethylformamide. The Formula (VIII) compounds are prepared from the condensation of two molecules of the corresponding aryl carboxaldehydes catalyzed by cyanide, usually in refluxing aqueous ethanolic solution (benzoin condensation).

Alternatively, compounds of Formula (IX) may be prepared from the corresponding ethanones of Formula (X) by reaction with hydroxylamine to afford the oximes of Formula (XII). Subsequent treatment of the Formula (XII) compounds with tosyl chloride and pyridine gives the oxime tosylates of Formula (XIII). Reaction of the Formula (XIII) compounds with strong base affords the 2-amino-ethan-1-ones of Formula (XIV). These compounds are treated with hydrochloric acid, and the resulting Formula (XIV) hydrochloride salt is refluxed in an aqueous solution of sodium thiocyanate to afford conversion of the Formula (XIV) compound into the diaryl-2-mercaptoimidazole Formula (IX) compound which can be filtered from the reaction mixture and recrystallized in alcohol or dimethylformamide-water. The required ethanones of Formula (X) wherein $R^7$ and $R^8$ are independently selected from (a) phenyl or monosubstituted phenyl where said substitutent is selected from $C_{1-3}$ alkoxy, 2,2,2-trihaloethoxy, prop-2-ene-1-oxy, $C_{1-4}$ alkyl, $C_{1-3}$ alkylthio, $C_{1-3}$ alkanamido, halo, or $CF_3$ (b) disubstituted phenyl wherein said substituents are independently selected from $C_{1-4}$ alkyl or $C_{1-3}$ alkoxy or the disubstituents together form a methylenedioxy group (c) 3,4,5-trimethoxyphenyl or (d) $R^7$ and $R^8$ are both CN, are obtained by (a) Friedel-Crafts acylation by a substituted phenylacetyl chloride on the corresponding benzene, (b) Curtius rearrangement of the substituted stilbenecarbonyl azides derived from Perkin condensation of a substituted benzaldehyde with the substituted phenylacetic acid ester, (c) reduction of the corresponding benzoin with zinc or tin in glacial acetic acid by boiling for 4-5 hours, and (d) Claisen condensation of a substituted phenylacetonitrile with a substituted aryl carboxylic acid ester.

In the second procedure of synthetic route 1 for the preparation of Formula (I) compounds wherein X is S; A and C are as hereinbefore defined and $R^3$ and $R^2$ are independently mono-, di- or tri-substituted phenyl, one equivalent of a 2-halo-ethan-1-one of Formula (XI) wherein $R^{11}$ and $R^{12}$ are independently selected from (a) phenyl or monosubstituted phenyl and said substitutent is selected from $C_{1-4}$ alkyl, halo (preferably chloro or bromo), $CF_3$, $C_{1-3}$ alkoxy, $C_{1-3}$ alkylthio, 2,2,2-trihaloethoxy, prop-2-en-1-oxy, $C_{1-3}$ alkanamido, N-($C_{1-3}$ alkyl)-($C_{1-3}$alkanamido), or (b) disubstituted phenyl wherein said substituents are independently selected from $C_{1-4}$ alkyl or $C_{1-3}$ alkoxy or the disubstituents together form a methylenedioxy group, is treated with two to five equivalents of the correspondingly substituted 2-amino-thiazoline or 2-amino-5,6-dihydro-4H-[1,3]thiazine and potassium carbonate in a polar solvent such as dry acetonitrile at room temperature for 18 hours to 5 days. The product is then purified, for example the solvent is evaporated and the residue is dissolved in methylene chloride, is washed with 5% aqueous sodium carbonate and the organic phase concentrated in vacuo and recrystallized or chromatographed to yield the desired Formula (I) compound. The Formula (XI) compounds where halo is Br are prepared preferably by bromination of the corresponding Formula (X) compounds. Alternately the alpha-chloro Formula (XI) compounds are prepared from the corresponding Formula (VIII) compounds by heating of 4 g of the benzoin with 4 ml of thionyl chloride.

Alternatively, when X is $CH_2$ or S, one equivalent of 2-haloethanone of Formula (XI) is treated with one equivalent of the corresponding substituted 2-amino-thiazoline or 2-amino-4,5-dihydro-[1,3]thiazine (where X is S) or 2-iminopyrrolidine or 2-imino-piperidine (where X is $CH_2$) in a nonpolar solvent, such as chloroform

or toluene, at room temperature for about 4 to 24 hours. The solvent is evaporated in vacuo and the residue refluxed in a polar solvent such as water or aqueous alcohol for up to about 15 hours. The solution is treated with 5% aqueous sodium carbonate and extracted with methylene chloride. The organic phase is concentrated in vacuo and recrystallized or chromatographed to yield the desired Formula (I) product.

Compounds of Formula (I) wherein X is S; A and C are as hereinbefore defined and one of $R^2$ or $R^3$ is pyridyl and the other is mono-, di-, or trisubstituted phenyl are prepared from the corresponding Formula (IX) compounds by alkylation with the appropriate $C_{2-3}$ dihaloalkane and one equivalent of sodium hydride in dimethylformamide followed by addition of potassium carbonate, subsequent cyclization upon heating, and precipitation by addition of ice water. The resulting two isomeric 6-aryl-5-pyridyl and 5-aryl-6-pyridyl Formula (I) compounds are separated chromatographically. The Formula (IX) compounds are prepared from either the corresponding (a) Formula (VIII) 2-hydroxyethanones or (b) Formula (XIV) 2-aminoethanones. Formula (IX) compounds where at least one of $R^9$ or $R^{10}$ is pyridyl and the other is selected from (a) phenyl or monosubstituted phenyl where said substituent is selected from $C_{1-3}$ alkoxy, 2,2,2-trihaloethoxy, prop-2-en-1-oxy, $C_{1-4}$ alkyl, $C_{1-3}$ alkylthio, $C_{1-3}$ alkanamido, halo, or $CF_3$, (b) disubstituted phenyl wherein said substitutents are independently selected from $C_{1-4}$ alkyl or $C_{1-3}$ alkoxy or the disubstitutents together form a methylenedioxy group, (c) 3,4,5-trimethoxyphenyl, or (d) pyridyl are prepared from the corresponding Formula (VIII) compounds by the same procedure as described above for the substituted diphenyl Formula (IX) compounds. Formula (VIII) compounds where one of $R^9$ or $R^{10}$ is 4-pyridyl are prepared by treatment of 4-pyridine carboxaldehyde cyanohydrin benzoate and a substituted benzaldehyde in t-butanol with sodium or potassium hydride.

Alternatively, pyridyl-containing Formula (IX) compounds may be prepared from the corresponding Formula (X) ethanones in 4 steps by successive conversion of the Formula (X) compounds to the corresponding Formula (XII), Formula (XIII), and Formula (XIV) compounds by the method previously described. The requisite Formula (X) ethanones wherein at least one of $R^7$ and $R^8$ is pyridyl and the other is independently selected from (a) phenyl or mono-substituted phenyl where said substituent is selected from $C_{1-3}$ alkoxy, 2,2,2-trihaloethoxy, prop-2-en-1-oxy, $C_{1-4}$ alkyl, $C_{1-3}$ alkylthio, $C_{1-3}$-alkanamido, halo, or $CF_3$, (b) disubstituted phenyl wherein said substituents are independently selected from $C_{1-4}$ alkyl or $C_{1-3}$ alkoxy or the disubstituents together form a methylenedioxy group, or (c) 3,4,5-trimethoxyphenyl, are preferably prepared by Claisen condensation of a substituted phenylacetonitrile with a 2-, 3-, or 4-picolinic acid ester or alternatively by reaction of the picolyl sodium or lithium and the appropriately substituted benzoic acid ester.

The Formula (I) compounds where both $R^2$ and $R^3$ are pyridyl and X is S are prepared in two steps from the corresponding Formula (VIII) 2-hydroxy-ethan-1-ones and the corresponding Formula (IX) compounds by the methods described above for the corresponding Formula (VIII), Formula (IX) and Formula (I) compounds where the aryl groups are both substituted phenyl. The precursor dipyridyl Formula (VIII) compounds are prepared by the benzoin condensation as described above for the corresponding diphenyl Formula (VIII) compounds with the exception that thiourea must be added in the condensation of 4-pyridine carboxaldehyde.

Compounds of Formula (I) where $R^2$ is 4-pyridyl and $R^3$ is substituted phenyl are preferably prepared in two steps from their corresponding Formula (K) and Formula (IV) compounds. The Formula (IV) compounds wherein A and C are as hereinbefore defined; $X^2$ is N-Z-carbonyl-1,4-dihydro-4-pyridyl, Z is $C_{1-8}$ alkyl, $C_{1-8}$ alkoxy, phenyl, phenoxy, benzyl or benzyloxy, and $X^1$ is (a) phenyl or monosubstituted phenyl and said substituent is selected from $C_{1-3}$ alkoxy, halo, $CF_3$, $C_{1-3}$ alkylthio, $C_{1-4}$ alkyl, N-(azacyclo $C_{5-6}$ alkyl), N-($C_{1-3}$ alkyl)-($C_{1-3}$ alkanamido), $C_{1-3}$ dialkylamino, cyano, 2,2,2-trihaloethoxy prop-2-en-1-oxy or disubstituted phenyl wherein said substituents are independently selected from $C_{1-4}$ alkyl or $C_{1-3}$ alkoxy or the disubstituents together form a methylenedioxy group, are prepared from the corresponding Formula (K) compounds. In the first step, the Formula (K) compound is treated at 5-25°C with pyridine and an acyl halide such as an alkyl chloroformate (preferably ethyl chloroformate) or an arylcarbonyl halide such as benzoyl chloride in a solvent in which the reactants are soluble and inert, such as methylene chloride, to form the corresponding Formula (IV) compound. In the second step, the Formula (IV) compound, an N-acyldihydropyridine product, is deacylated and aromatized either with a mild oxidizing agent such as sulfur in refluxing decalin, tetralin or p-cymene or preferably with oxygen and excess potassium t-butoxide in t-butanol, to afford the compounds of Formula (I).

The 2,2,2-trihaloethoxyphenyl and prop-2-ene-1-oxyphenyl substituted Formula (I), Formula (K), and Formula (V) compounds are prepared by alkylation of the corresponding Formula (I), Formula (K), and Formula (V) hydroxyphenyl compounds with a 2,2,2-trihaloalkylester of trifluoromethane sulfonic acid and 2-propenyl bromide respectively as follows. One equivalent of the hydroxyphenyl compound is added to sodium hydride in tetrahydrofuran under nitrogen below 0°C. After 1/2 hour three equivalents of the ester of

the trifluoromethane sulfonic acid is added dropwise. The suspension is poured into ice water and extracted into methylene chloride followed by washing, drying, and evaporation of the solvent. To one equivalent of the hydroxyphenyl compound is added about one equivalent of the 2-propenyl bromide in dimethylformamide Sodium hydride is added maintaining the temperature below 55 °C. A second portion of the bromide is added and the reaction is heated to 60-75 °C for 1 hour. The reaction mixture is cooled, is added to water, and the pH is adjusted to 11 with 10% sodium hydroxide. It is then extracted into methylene chloride and purified.

The hydroxyphenyl Formula (I), Formula (K), and Formula (V) compounds are prepared by treatment of the corresponding methoxyphenyl compounds with HBr in refluxing acetic acid or alternatively with $BBr_3$ in methylene chloride.

$C_{1-3}$ alkanamido and N-($C_{1-3}$ alkyl)-($C_{1-3}$ alkanamido) phenyl substituted acetophenones, and in some cases the Formula (K), and Formula (I) compounds, are prepared by acylation of the corresponding amino and $C_{1-3}$ alkylamino compounds with the alkanoic acid anhydride or chloride in pyridine. Another alternative preparation of the ($C_{1-3}$ alkyl)-($C_{1-3}$ alkanamido) phenyl carboxaldehydes, substituted Formula (K) and Formula (I) compounds employs the alkylation of the corresponding ($C_{1-3}$ alkanamido) phenyl substituted carboxaldehydes, Formula (K) and Formula (I) compounds with sodium hydride and a $C_{1-3}$ alkyl bromide or iodide in dimethylformamide.

Aminophenyl substituted Formula (K) and Formula (I) compounds are prepared by hydrolysis of the corresponding $C_{1-3}$ alkanamido compounds in refluxing 6N mineral acid.

N-($C_{1-3}$ alkylamino) phenyl substituted Formula (K), Formula (V), and Formula (I) compounds are preferably prepared by acid catalyzed hydrolysis of the corresponding N-($C_{1-3}$ alkyl)-($C_{1-3}$ alkanamido) compounds as described above for the aminophenyl substituted compounds, or alternatively by reduction of the corresponding ($C_{1-3}$ alkanamido) phenyl Formula (K), Formula (V) or Formula (I) compounds with borane or borane dimethylsulfide complex in tetrahydrofuran.

N,N-($C_{1-3}$ dialkylamino) phenyl substituted Formula (K) and Formula (I) compounds are alternatively prepared by reduction of the corresponding N-($C_{1-3}$ alkyl)-($C_{1-3}$ alkanamido) compounds with borane as described above for the N-($C_{1-3}$ alkylamino) phenyl substituted compounds.

N-(azacyclo $C_{5-6}$ alkyl) phenyl substituted Formula (K) and Formula (I) compounds are alternatively prepared by cyclodialkylation of the corresponding aminophenyl compounds with dibromobutane or dibromopentane and anhydrous potassium carbonate in an inert solvent such as dimethylformamide.

Compounds of Formula (I) where X is $S(O)_n$ and n is 1 or 2 are prepared by oxidation with one or two equivalents of an organic peracid.

Compounds of Formula (I) where at least one of $R^3$ and $R^2$ is pyridyl, X is $S(O)_n$ and n is 2 are preferably prepared by oxidation of 1 equivalent of an acid salt of a Formula (I) compound where X is $S(O)_n$, and n is 1, with 2/3 equivalent of an aqueous solution of potassium permanganate followed by dissolution of the resulting manganese dioxide with sulfur dioxide.

Compounds of Formula (I) where X is $S(O)_n$, n is 1 or 2, and where at least one of $R^2$ and $R^3$ is $C_{1-3}$ alkylaminophenyl, $C_{1-3}$ dialkylaminophenyl, or N-(azacyclo$C_{5-6}$ alkyl)phenyl are preferably prepared by treating the immediate precursor alkanamidophenyl Formula (I) compound with an oxidizing agent (as described above for preparation of compounds of Formula (IA) where X is $S(O)_n$ and n is 1 or 2) followed by hydrolysis of the alkanamide to the primary or secondary amine. The primary or secondary amine may then be further alkylated as described above to afford the tertiary amine $S(O)_n$ compounds where n is 1 or 2.

The compounds of Formula (I) can also be prepared according to the alternate synthetic route 2.

All the compounds of Formula (E), Formula (F), Formula (G) and Formula (H) are useful as intermediates in the preparation of compounds of Formula (I) wherein X is $CH_2$. All of the necessary compounds of Formula (A), Formula (B), Formula (C) and Formula (D) can be obtained from commercial sources or are preparable by conventional techniques such as those set out herein.

Compounds of Formula (B), wherein A and C are as hereinbefore defined, H can be prepared by 0-alkylation of the corresponding 2-piperidone or 2-pyrrolidone of Formula (A), wherein A and C are as defined above, with dimethylsulfate. The necessary compounds of Formula (A) are commercially available or are prepared by known techniques. Compounds of Formula (C) wherein A and C are as defined above, can be prepared by treatment of the corresponding compound of Formula (B) with ammonium chloride in absolute ethanol. Compounds of Formula (C) wherein A and C are H are preferably prepared as their hydrohalide salts and liberated to the bases with concentrated aqueous sodium hydroxide or preferably with one equivalent of sodium $C_{1-2}$ alkoxide in the $C_{1-2}$ alcohol, followed by evaporation of the solvent in vacuo. Compounds of Formula (D), wherein $Y^3$ is Br and Y is the same as defined above in Formula (H), are commercially available or are prepared by treatment of the correspondingly substituted acetophenone in methylene chloride, chloroform or acetic acid with one equivalent of bromine, or alternatively, by reaction in

chloroform-ethyl acetate with a suspension of copper (II) bromide. The necessary acetophenones are commercially available or preparable by known techniques. Alternatively the Formula (D) compounds, wherein $Y^3$ is chloro and Y is (a) phenyl or 4-mono-substituted phenyl where the substituent is selected from halo, $C_{1-4}$ alkyl, $C_{1-3}$ alkylthio, $C_{1-3}$ alkoxy, or (b) 3,4-disubstituted phenyl wherein the substituents are the same and are selected from $C_{1-3}$ alkoxy, methylenedioxy, or where the substituents are independently selected from halo or $C_{1-3}$ alkoxy, can be prepared by acylating the corresponding mono- or disubstituted benzene by Friedel Crafts reaction with 2-chloroacetyl chloride and aluminium chloride.

Preferably, compounds of Formula (E) are prepared from their corresponding compound of Formula (H). Compounds of Formula (H) serve as intermediates in the preparation of compounds of Formula (E). Compounds of Formula (H) are prepared by treatment of a solution of a substituted Formula (D) compound, such as a 2-haloacetophenone, or a 2,3, or 4-bromoacetylpyridine, in a neutral, preferably nonpolar solvent with one molar equivalent of the corresponding Formula (C) compound, maintaining the temperature at or below 25°C. The resulting crystalline Formula (H) hydrohalide salts are converted to Formula (E) compounds by refluxing in water. Compounds of Formula (E) serve as intermediates in the preparation of the compounds of Formula (I) when X is $CH_2$. Alternatively, compounds of Formula (E) are prepared by treatment of a solution of the 2-iminopyrrolidine or 2-iminopiperidine with a substituted 2-bromoacetophenone of Formula (D), either in a polar organic solvent, such as dimethylformamide or ethanol, or in a nonpolar chlorinated hydrocarbon, followed by removing all or most of the solvent and refluxing the residue in aqueous solution.

Compounds of Formula (I) where X is $CH_2$, $R^3$ is phenyl or substituted phenyl, and $R^2$ is 4-pyridyl are preferably prepared in two steps. In the first step, the corresponding compound of Formula (E) is treated, preferably at 20-25°C, with an excess of both pyridine and an acyl halide or a haloacyl ester such as acetyl bromide, benzoylchloride, benzyl chloroformate, or preferably ethyl chloroformate, in an organic solvent in which the reactants are soluble and inert to form the compound of Formula (F). The acyl group can contain $C_{1-8}$ alkyl, $C_{1-8}$ alkoxy, phenyl, phenoxy, benzyl, or benzyloxy. One skilled in the art will recognize that the pyridine and acyl ester react to form the acylpyridinium reagent in situ. Optionally, the acylpyridinium reagent can be prepared separately in a solvent and then added to the solution of Formula (E) compound. Suitable solvents include methylene chloride, ethylene chloride, chloroform, carbon tetrachloride, tetrahydrofuran, ethyl ether, dioxane, toluene, or excess pyridine.

The reaction mixture is cooled as necessary by use of an ice/water bath during the mixing of the reactants, in order to maintain ambient temperature. The mixture is then allowed to stir at ambient temperature for up to 48 hours until reaction is complete. The reaction mixture is continually assayed using high pressure liquid chromatography (HPLC) or thin layer chromatography (TLC) on aliquots to ascertain if unreacted Formula (E) compound is present. If so, additional acyl pyridinium salt is introduced. Other conditions of the reaction are standard to the art. Following the reaction, the resultant compounds can be recovered from the reaction mixture and isolated by standard techniques. Compounds of Formula (F) serve as intermediates in the preparation of the compounds of Formula (I). In the second step, the Formula (F) compound, a dihydropyridine product, is deacylated and aromatized with sulfur in refluxing decalin, tetralin, p-cymene or xylene, or preferably with potassium tert-butoxide in tert-butanol with oxygen gas at reflux for 15 minutes to the afford the corresponding compound of Formula (I).

The same Formula (E) compounds used to prepare the 4-pyridyl Formula (I) compounds where X is $CH_2$ are employed to prepare the analogous 2-pyridyl and 3-pyridyl Formula (I) compounds. Treatment of one equivalent Formula (E) compound with one equivalent of bromine in methylene chloride at 20-25°C for about 1/2 hour, followed by addition of 5% potassium carbonate and concentration of the organic phase in vacuo results in 3-bromination to afford the 3-bromo-2-(substituted phenyl)-6,7-dihydro-(5H)-pyrrolo[1,2-a]-imidazoles and 3-bromo-2-(substituted phenyl)-5,6,7,8-tetrahydro-imidazo[1,2-a]pyridines compounds of Formula (G). The compounds of Formula (G) serve as intermediates in the preparation of compounds of Formula (I). These Formula (G) compounds are treated with n-butyl lithium (n-BuLi) in tetrahydrofuran to afford their 3-lithio derivatives by halogen-metal interchange. Transmetallation of the 3-lithio compounds with $MgBr_2$ or $ZnCl_2$ to the corresponding magnesium or zinc compounds, provides good coupling to 2- or 3- bromo-pyridine in the presence of $PdCl_2$(1,4-bis(diphenylphosphino)butane) catalyst, a bidentate Pd (II) catalyst. Alternatively the Formula (G) compounds may be coupled to the 2 or 3-metalated pyridine employing this bidentate Pd (II) catalyst, or the corresponding Ni(II)$Cl_2$- (1,2-bis(diphenylphosphino)ethane) catalyst. By either of these routes, Formula (I) compounds are obtained where $R^2$ is 2-pyridyl or 3-pyridyl.

Compounds of Formula (F) can be prepared by addition of a Grignard reagent prepared from a Formula (G) compound to an N-acylpyridium salt by the method previously described for the reaction of Grignard reagents prepared from Formula (V) compounds with N-acylpyridium salt. The Grignard reagent is prepared from a compound of Formula (G) by the method previously described for the preparation of the analogous

Grignard reagent from a Formula (V) compound.

Regioisomers of Formula (I) compounds where X is $CH_2$, $R^2$ is substituted phenyl, or 2-, 3-, or 4-pyridyl and $R^3$ is 2-, 3-, and 4-pyridyl are obtained from compounds of Formula (E) where $Y^4$ is 2-, 3-, or 4-pyridyl. Compounds of Formula (E) where $Y^4$ is 2-, 3-, or 4-pyridyl are prepared by treatment of a 2-, 3-, or 4-bromoacetylpyridine hydrobromide salt of Formula (D), wherein R is 2-, 3- or 4-pyridyl with 2-3 equivalents of the 2-iminopyrrolidine or 2-iminopiperidine by the procedure used to prepare the other compounds of Formula (E) described above. 3-Bromination affords the corresponding Formula (G) compounds. Metalation of the Formula (G) compounds via halogen-metal interchange with n-BuLi, transmetallation with $MgBr_2$ and coupling to the substituted bromobenzene or 2-, 3-, or 4-bromopyridine in the presence of the bidentate phosphine-palladium or nickel complex as described above affords the desired regioisomers of Formula (I) and the bis(pyridyl) compounds of Formula (I). Alternatively the metalated pyridine or substituted benzene may be coupled to the Formula (G) compounds employing the catalysts as described above.

Compounds of Formula (I) where X is $CH_2$, $R^3$ or $R^2$ are $C_{1-3}$ alkylsulfinyl substituted phenyl are prepared by treatment of one equivalent of the corresponding compound of Formula (I) where $R^3$ or $R^2$ are $C_{1-3}$ alkylmercaptophenyl, with one equivalent of an oxidizing agent (preferably, 3-chloroperbenzoic acid) per mercapto function, in an inert solvent. Compounds of Formula (I) wherein X is $CH_2$, $R^3$ or $R^2$ are $C_{1-3}$ alkylsulfonyl substituted phenyl are prepared by treatment of one equivalent of the corresponding $C_{1-3}$ sulfinyl Formula (I) compound with two-thirds equivalent of potassium permanganate per sulfinyl function in aqueous solution. After addition of all potassium permanganate, sulfur dioxide is passed through the solution to dissolve any precipitated manganese dioxide. The resulting liquor is extracted with chloroform, dried, evaporated, and the residue recrystallized from 50% aqueous alcohol to yield the desired product.

$C_{1-3}$ alkanamido) and N-($C_{1-3}$ alkyl)-($C_{1-3}$ alkanamido)-phenyl substituted acetophenones, and in some cases the Formula (E) and Formula (I) compounds, are prepared by acylation of the corresponding amino and $C_{1-3}$ alkylamino) compounds with the alkanoic acid anhydride or chloride in pyridine. Another alternative preparation of the N-($C_{1-3}$ alkyl)-($C_{1-3}$ alkanamido) phenyl substituted Formula (E) and Formula (I) compounds is the alkylation of the corresponding $C_{1-3}$ alkan-amido substituted compounds with sodium hydride and a $C_{1-3}$ alkyl bromide or iodide in dimethyl formamide.

Aminophenyl substituted Formula (E) and Formula (I) compounds are prepared either by hydrolysis of the corresponding $C_{1-3}$ alkanamido compounds in refluxing 6 N mineral acid or by catalytic reduction of the corresponding nitro compounds.

N-($C_{1-3}$ alkylamino)phenyl substituted Formula (E), Formula (G), and Formula (I) compounds are preferably prepared by acid catalyzed hydrolysis of the corresponding N-($C_{1-3}$ alkyl)-($C_{1-3}$ alkanamido) compounds of Formula (E), Formula (G) and Formula (I), respectively, prepared as described above for the aminophenyl substituted compounds, or alternatively, either by (a) reduction of the corresponding $C_{1-3}$ alkanamido compounds with borane or borane dimethylsulfide complex in tetrahydrofuran, or (b) by cleavage of the corresponding N,N-($C_{1-3}$ dialkylamino)phenyl substituted Formula (E) and Formula (I) compounds with cyanogen bromide in the Von Braun reaction.

N,N-($C_{1-3}$ dialkylamino)phenyl substituted Formula (E) and Formula (I) compounds are alternatively prepared either by reduction of the corresponding N-($C_{1-3}$ alkyl)-($C_{1-3}$ alkanamido) compounds of Formula (E) and Formula (I) with borane as described above for the N-($C_{1-3}$ alkylamino)phenyl substituted compounds.

N-(azacyclo $C_{5-6}$ alkyl)phenyl substituted Formula (E) and Formula (I) compounds are alternatively prepared by cyclodialkylation of the corresponding aminophenyl compounds with dibromobutane or dibromo-pentane and anhydrous potassium carbonate in an inert solvent such as dimethylformamide.

Compounds of Formula (E) where $Y^4$ is 2,2,2-trihaloethoxy or prop-2-ene-1-oxy substituted phenyl are prepared by alkylation of the appropriate phenols of Formula (E) with trifluoromethylsulfonic acid 2,2,2-trifluoroethyl ester or allyl bromide respectively as previously described for the Formula (K) compounds. Appropriately substituted mono and dihydroxy phenyl compounds of Formula (E) and Formula (I) are obtained by treatment of their respective correspondingly substituted methoxy derivatives with HBr in acetic acid, or preferably with $BBr_3$ in methylene chloride at -60 °C followed by a return to room temperature, addition of water, and filtering of the crude product.

Pharmaceutically acceptable salts and their preparation are well known to those skilled in pharmaceuticals. Pharmaceutically acceptable salts of the compounds of Formulae (I), (K), (J), and (V) which are useful in the present invention include, but are not limited to, maleate, fumarate, lactate, oxalate, methanesulfonate, ethanesulfonate, benzenesulfonate, tartrate, citrate, hydrochloride, hydrobromide, sulfate and phosphate salts. Preferred pharmaceutically acceptable salts of the compounds of Formula (I) include hydrochloride and hydrobromide salts, and such salts can be prepared by known techniques.

Some of the compounds of Formula (I) can form covalent hydrates i.e., the compounds of Formula (I) where $R^3$ is other than pyridyl, $R^2$ is 2- or 4-halo, $CF_3$ or cyano substituted phenyl and X is S will form covalent hydrates where an OH group is attached to the carbon atom attached to $R^3$ and an H is attached to the carbon atom attached to $R^2$. The preparation of such hydrates is disclosed by Bender et al., U.S. Patent 4,153,706, issued May 8, 1979, which claims compounds having the structure:

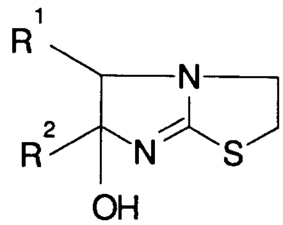

wherein $R^1$ is 4-substituted phenyl wherein said substituent is selected from lower alkoxy, lower alkylthio, fluoro, chloro, bromo or trifluoro methyl; and $R^2$ is 4-substituted phenyl wherein said substituent is an electron withdrawing group, in particular, fluoro, chloro, bromo or trifluoromethyl. At least some of the hydrates disclosed by Bender et al. have 5-lipoxygenase inhibiting activity as determined by the assays set forth in the Examples. Therefore, the scope of the compounds of Formula (I) includes such hydrates, described in Bender et al., which have 5-lipoxygenase pathway inhibiting activity or which are useful to prepare other compounds of Formula (I) which have 5-lipoxygenase pathway inhibiting activity.

It is known that some of the compounds of Formula (IA) are useful for inhibiting the cyclooxygenase pathway of arachidonic acid metabolism. It has now been discovered that all the compounds of Formula (IA) are useful for treating disease states mediated by the 5-lipoxygenase pathway of arachidonic acid metabolism by inhibiting such pathway. Formula (IA) includes all compounds active as 5-lipoxygenase inhibitors whether or not such compounds are novel. The discovery that the compounds of Formula (IA) are inhibitors of the 5-lipoxygenase pathway or are dual inhibitors of the cyclooxygenase and 5-lipoxygenase pathways is based on the effects of the compounds of Formula (IA) on tissue inflammation in vivo and on the production of cyclooxygenase products and 5-lipoxygenase products by inflammatory cells in vitro in assays which are described in the Examples. In summary, such assays reveal that the compounds of Formula (IA) inhibit the infiltration of polymorphonuclear leukocytes into inflammatory lesions in mice (carrageenan-induced peritonitis) and rats (air pouch inflammation induced by arachidonic acid). In addition, the compounds of Formula (IA) display anti-inflammatory activity in arachidonic acid-induced inflammation in the mouse ear and rat paw models. The cyclooxygenase inhibitor, indomethacin, did not reduce inflammation or cell infiltration in these assays. These data, together with previous observations on the anti-edematous effects of the compounds of Formula (IA) in inflammatory lesions caused by cyclooxygenase-generated products, reveal that the compounds of Formula (IA) inhibit either the 5-lipoxygenase pathway or both the 5-lipoxygenase and cyclooxygenase pathways of arachidonic acid metabolism. The 5-lipoxygenase pathway inhibitory action of the compounds of Formula (IA) was confirmed by showing that such compounds (a) impaired the production of 5-lipoxygenase products such as leukotriene $B_4$ (di-HETE) and 5-HETE production by RBL-1 cells, (b) impaired the production of $LTC_4$ by human monocytes, and (c) that peritoneal exudate cells harvested from Formula (IA) compound treated mice exhibited a reduced capacity to produce $LTB_4$ in vitro.

The pathophysiological role of arachidonic acid metabolites has been the focus of recent intensive studies. In addition to the well-described phlogistic activity (i.e. general inflammatory activity) of prostaglandins, the more recent description of similar activity for lipxygenase products of arachidonic acid has broadened the interest in these products as mediators of inflammation. The reported discovery of potent chemotactic and algesic activity for $LTB_4$, together with known $LTC_4$ and $LTD_4$-mediated increase in capillary permeability, has led to their consideration as targets for pharmacological intervention in both the fluid and cellular phases of inflammatory diseases.

The pharmacology of several inflammatory model systems has attested to the effectiveness of corticosteroids in reducing the cellular infiltration. These results, and the observation that corticosteroids inhibit the generation of both cyclooxygenase and lipoxygenase products, suggest that such dual inhibitors may effectively reduce both the fluid and cellular phases of the inflammatory response since selective cyclooxygenase inhibitors do not reliably inhibit cell influx into inflammatory sites The observations outlined above cogently argue that a dual inhibitor of arachidonic acid metabolism would be a more effective anti-inflammatory agent than an inhibitor of cyclooxygenase only. Under optimal conditions, it is likely that an

agent with preferential lipoxygenase inhibitory activity would not share the ulcerogenic liability of cyclooxygenase inhibitors or the toxicity of corticosteroids.

Recent clinical data also support the utility of dual inhibitors of arachidonic acid metabolism in a variety of inflammatory diseases. These include rheumatoid arthritis, inflammatory bowel disease, psoriasis, gout, myocardial infarction, organ transplant rejection, tissue trauma, asthma, and inflammation reactions in the central nervous system such as multiple sclerosis.

Compounds of Formula (IA) which are preferred because of their potent 5-lipoxygenase pathway inhibiting activity, as evidenced by their ability to inhibit the 5-lipoxygenase products known as 5-HETE, $LTB_4$ and/or $LTC_4$, are listed in Table A, below. For all compounds on Table A and C are hydrogen.

TABLE A

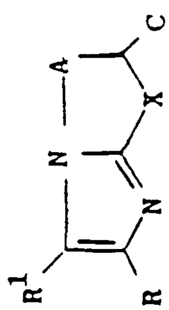

Formula (IA)

| Compound Number | R' | R | X | A | n |
|---|---|---|---|---|---|
| 3 | 4-(pyrrolidin-1-yl)phenyl | 4-(pyrrolidin-1-yl)phenyl | S(O)n | $CH_2$ | 0 |
| 6 | 4-fluorophenyl | 4-dimethylaminophenyl | S(O)n | $CH_2$ | 0 |
| 9 | 4-diethylaminophenyl | 4-diethylaminophenyl | S(O)n | $CH_2$ | 0 |
| 14 | 4-fluorophenyl | 4-methoxyphenyl | S(O)n | $CH_2$ | 0 |
| 17 | 4-ethylaminophenyl | 4-ethylaminophenyl | S(O)n | $CH_2$ | 0 |
| 24 | 4-pyridyl | 4-(pyrrolidin-1-yl)phenyl | S(O)n | $CH_2$ | 0 |
| 27 | 4-pyridyl | 4-fluorophenyl | S(O)n | $CH_2$ | 1 |
| 28 | 4-pyridyl | 4-fluorophenyl | S(O)n | $CH_2$ | 2 |
| 1 | 4-pyridyl | 4-fluorophenyl | S(O)n | $CH_2$ | 0 |
| 2 | 4-pyridyl | 4-(1-propylamino)phenyl | S(O)n | $CH_2$ | 0 |
| 4 | 4-(piperidin-1-yl)phenyl | 4-(piperidin-1-yl)phenyl | S(O)n | $CH_2$ | 0 |
| 7 | 3,4-(methylenedioxy)phenyl | 4-pyridyl | S(O)n | $CH_2$ | 0 |
| 15 | 4-trifluoromethylphenyl | 4-trifluoromethylphenyl | S(O)n | $CH_2$ | 0 |
| 16 | 3,4-(methylenedioxy)phenyl | 3,4-(methylenedioxy)phenyl | S(O)n | $CH_2$ | 0 |
| 17 | 4-ethylaminophenyl | 4-ethylaminophenyl | S(O)n | $CH_2$ | 0 |
| 18 | 4-pyridyl | 4-methoxyphenyl | S(O)n | $CH_2$ | 0 |
| 19 | 4-fluorophenyl | 4-fluorophenyl | S(O)n | $CH_2$ | 0 |
| 21 | 4-methoxyphenyl | 4-methoxyphenyl | S(O)n | $CH_2$ | 0 |
| 20 | 4-methoxyphenyl | 4-methoxyphenyl | S(O)n | $CH_2CH_2$ | 0 |
| 22 | 4-methoxyphenyl | 4-methoxyphenyl | $CH_2$ | $CH_2$ | - |
| 11 | 4-pyridyl | 4-pyridyl | S(O)n | $CH_2$ | 0 |
| 39 | 4-pyridyl | 4-fluorophenyl | $CH_2$ | $CH_2$ | 0 |
| 40 | 4-pyridyl | 4-methoxyphenyl | $CH_2$ | $CH_2$ | 0 |

This invention also relates to a pharmaceutical composition comprising a pharmaceutically acceptable carrier or diluent and an effective, 5-lipoxygenase pathway inhibiting amount of a compound of Formula (IC), or pharmaceutically acceptable salts thereof.

Active compounds of Formula (I), are administered in conventional dosage forms prepared by combining a therapeutically effective amount (i.e., a 5-lipoxygenase pathway inhibiting amount) of a compound of Formula (I) ("active ingredient") with standard pharmaceutical carriers or diluents according to conventional procedures. These procedures may involve mixing, granulating and compressing or dissolving the ingredients as appropriate to the desired preparation.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25 mg. to about 1 g. If a liquid carrier is used, the preparation will be in the form of a syrup, emulsion, soft gelatin capsule, sterile injectable liquid such as an ampule or nonaqueous liquid suspension.

To obtain a stable water soluble dose form, a pharmaceutically acceptable salt of a compound of Formula (I) is dissolved in an aqueous solution of an organic or inorganic acid, such as a 0.3M solution of succinic acid, or, preferably, citric acid.

Preferably, each parenteral dosage unit will contain the active ingredient in an amount of from about 50 mg. to about 500 mg. Preferably each oral dosage unit will contain the active ingredient in an amount of from about 100 mg to about 1,000 mg.

The compounds of Formula (IA) can be used in treating a disease state which is mediated by the 5-lipoxygenase pathway in an animal in need thereof, including humans and other mammals, provided that such animal is in need of treatment of a 5-lipoxygenase pathway mediated disease other than, or in addition to, rheumatoid arthritis, rheumatism, inflammation or any other cyclooxygenase mediated disease state, which comprises administering to such animal an effective, 5-lipoxygenase pathway inhibiting amount of a Formula (IA) compound or a pharmaceutically acceptable salt thereof. By the term "treating" is meant prophylactic or therapeutic therapy. By the term "mediated" is meant caused by or exacerbated by. This invention also relates to a compound of Formula (IC) for use in treating rheumatoid arthritis in a animal in need thereof, including humans and other mammals. The Formula (IA) compound is administered to an animal in need of treatment of a 5-lipoxygenase pathway mediated disease state, other than or in addition to rheumatoid arthritis, rheumatism, inflammation or any other cyclooxygenase mediated disease state, in an amount sufficient to inhibit the 5-lipoxygenase pathway. The Formula (IC) compound is administered to an animal in need of treatment of rheumatoid arthritis in an amount sufficient to inhibit the 5-lipoxygenase pathway. Such Formula (IA) or (IC) compound can be administered to such animal in a conventional dosage form prepared by combining the Formula (IA) or (IC) compound with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables.

As stated above, it will be apparent to one of skill in the art that all the compounds of Formula (IC) are embraced by the scope of Formula (IA), and as such, all the following disclosure regarding appropriate pharmaceutical composition dosage forms and preferred dosage ranges are applicable to both Formula (IA) and Formula (IC) compounds which will hereafter be collectively referred to as "Formula (IA)" compound(s). The route of administration of the Formula (IA) compound may be oral, parenteral, by inhalation or topical. The term parenteral as used herein includes intravenous, intramuscular, subcutaneous, rectal, vaginal or intraperitoneal administration. The subcutaneous and intramuscular forms of parenteral administration are generally preferred. The daily parenteral dosage regimen for a compound will preferably be from about 50 mg to about 1,000 mg per day. The daily oral dosage regimen will preferably be from about 150 mg to about 2,000 mg.

The compounds for Formula (IA) may also be administered by inhalation. By "inhalation" is meant intranasal and oral inhalation administration. Appropriate dosage forms for such administration, such as an aerosol formulation or a metered dose inhaler, may be prepared by conventional techniques. The preferred daily dosage amount of a compound of Formula (IA) administered by inhalation is from about 10 mg to about 100 mg per day.

The compounds of Formula (IA) may also be administered topically to a mammal in need of the inhibition of the 5-lipoxygenase pathway of arachidonic acid metabolism. Thus, the compounds of Formula (IA) may be administered topically in the treatment of inflammation in an animal, including man and other

mammals, and may be used in the relief of rheumatoid arthritis, rheumatoid spondylitis, osteoarthritis, gouty arthritis and other arthritic conditions, inflammed joints, eczema, psoriasis or other inflammatory skin conditions such as burns; inflammatory eye conditions including conjunctivitis; pyresis, pain and other conditions associated with inflammation.

The amount of a compound of Formula (IA) (hereinafter referred to as the active ingredient) required for therapeutic effect on topical administration will, of course, vary with the compound chosen, the nature and severity of the inflammatory condition and the animal undergoing treatment, and is ultimately at the discretion of the physician. A suitable anti-inflammatory dose of a compound of Formula (IA) is 1.5 µg to 500 mg of base per kilogram bodyweight for topical administration, the most preferred dosage being 1 µg to 50 mg/kg of animal bodyweight, for example 5 µg to 25 mg/kg; administered two or three times daily. For application to the skin, from 1 µg to several mg of active ingredient may be applied per application, preferably from 10 to 100 µg per application.

By topical administration is meant non-systemic administration and includes the application of a compound of Formula (IA) externally to the epidermis, to the buccal cavity and instillation of such a compound into the ear, eye and nose, and where the compound does not significantly enter the blood stream. By systemic administration is meant oral, intravenous, intraperitoneal and intramuscular administration.

While it is possible for an active ingredient to be administered alone as the raw chemical, it is preferable to present it as a pharmaceutical formulation. The active ingredient may comprise, for topical administration, from 0.001% to 10% w/w, e.g. from 1% to 2% by weight of the formulation although it may comprise as much as 10% w/w but preferably not in excess of 5% w/w and more preferably from 0.1% to 1% w/w of the formulation.

The topical formulations of the present invention, both for veterinary and for human medical use, comprise an active ingredient together with one or more acceptable carrier(s) therefore and optionally any other therapeutic ingredient(s). The carrier(s) must be 'acceptable' in the sense of being compatible with the other ingredients of the formulation and not deleterious to the recipient thereof.

Formulations suitable for topical administration include liquid or semi-liquid preparations suitable for penetration through the skin to the site of inflammation such as: liniments, lotions, creams, ointments or pastes, and drops suitable for administration to the eye, ear or nose.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100°C for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powered form, alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as steric or oleic acid together with an alcohol such as prolylene glycol or macrogols. The formulation may incorporate any suitable surface active agent such as an anionic, cationic or non-ionic sulfactant such as sorbitan esters or polyoxyethylene derivatives thereof. Suspending agents such as natural gums, cellulose derivatives or inorganic materials such as silicaceous silicas, and other ingredients such as lanolin, may also be included.

It will be recognized by one of skill in the art that the optimal quantity and spacing of individual dosages of the Formula (IA) compound will be determined by the nature and extent of the condition being treated, the form, route and site of administration, and the particular animal being treated, and that such optimums can be determined by conventional techniques. It will also be appreciated by one of skill in the art that the optimal course of treatment, i.e., the number of doses of the Formula (IA) compound given per day for a

defined number of days, can be ascertained by those skilled in the art using conventional course of treatment determination tests.

Without further elaboration, it is believed that one skilled in the art can, using the preceding description, utilize the present invention to its fullest extent. The following examples are, therefore, to be construed as merely illustrative and not a limitation of the scope of the present invention in any way.

As used herein, the term "Compound 1" refers to the compound of Formula (IA) wherein $R^1$ is 4-pyridyl, R is 4-fluorophenyl, A is $CH_2$, C is H, X is $S(O)_n$, and n is 0.

Temperature is in degrees Centigrade (°C).

**EXAMPLE 1**

**5-(4-N,N-Dimethylaminophenyl)-6-(4-fluorophenyl)-2,3-dihydroimidazo[2,1-b]thiazole and 6-(4-N,N-dimethylaminophenyl)-5-(4-fluorophenyl)-2,3-dihydroimidazo[2,1-b]thiazole. Formula (IC) compounds.**

**a) Mixed Benzoin Product of 4-Fluorobenzaldehyde and 4-N,N-dimethylaminobenzaldehyde. Formula (VII) compound.**

A stirred solution of 10.0 g (0.068 mole) of 4-N,N-dimethylaminobenzaldehyde, 4.16 g (0.068 mole) of 4-fluorobenzaldehyde, and 4.0 g (0.061 mole) of potassium cyanide in 100 ml of 50% ethanol was refluxed for 3 hours and allowed to cool to ambient temperature overnight. The precipitate which formed was filtered, washed with 20% ethanol and recrystallized from absolute ethanol to give 3.0 g of yellow crystals, mp 133-138°C.

**b) 4-(4-N,N-dimethylaminophenyl)-5-(4-fluorophenyl)-2-mercaptoimidazole. Formula (IX) compound.**

A stirred mixture of 1.0 g (3.66 mmoles) of the mixed benzoin of 4-fluorobenzaldehyde and 4-N,N-dimethylaminobenzaldehyde, prepared as described in Example 1a), and 0.56 g (7.32 mmoles) of powdered thiourea in 9 ml of dry dimethylformamide was refluxed under argon for 3 hours. Addition of water to the cooled mixture gave a solid which was triturated with cold methanol. Filtration and washing of this precipitate with a small volume of cold methanol gave 0.65 g of the Formula (IX) compound as an orange-yellow solid. This was dried in vacuo and used immediately in part c.

**c) 5-(4-N,N-dimethylaminophenyl)-6-(4-fluorophenyl)-2,3-dihydroimidazo[2,1-b]thiazole and 6-(4-N,N-dimethylaminophenyl)-5-(4-fluorophenyl)-2,3-dihydroimidazo[2,1-b]thiazole. Formula (IC) compounds.**

A solution of 0.40 g (1.28 moles) of 4-(4-N,N-dimethylaminophenyl)-5-(4-fluorophenyl)-2-mercaptoimidazole, prepared as described in Example 1b), in 15 ml of dry dimethylformamide was treated with 0.061 g (1.28 mmoles) of a 50% sodium hydride dispersion. 0.18 g (1.28 mmoles) of 1-bromo-2-chloroethane was added after 0.5 hours stirring at ambient temperature. After an additional 12 hours stirring, 0.18 g (1.28 mmoles) of powdered potassium carbonate was added, and the mixture heated to 150°C for 2 hours. The solvent was removed in vacuo and the residue extracted with chloroform. The solution was washed with water, dried over magnesium sulfate and concentrated in vacuo. Column chromatography on silica afforded two isomers eluting with 10% and 20% acetonitrile in methylene chloride. Evaporation of the solvents followed by recrystallization from methanol gave 5-(4-dimethylaminophenyl)-6-(4-fluorophenyl)-2,3-dihydroimidazo[2,1-b)thiazole, mp 163-164.5°C, (TLC, silica, 10% $CH_3CN$ in methylene chloride, Rf 0.17) and 6-(4-dimethylaminophenyl)-5-(4-fluorophenyl)-2,3-dihydroimidazo[2,1-b]thiazole, mp 195-201°C, (TLC, silica, 10% $CH_3CN$ in methylene chloride, Rf 0.3).

**EXAMPLE 2**

**5,6-bis(4-N,N-diethylaminophenyl)-2,3-dihydroimidazo [2,1-b]thiazole. Formula (IC) compound**

**a) 5,6-Bis[4-(acetamido)phenyl]-2,3-dihydroimidazo[2,1-b]thiazole**

1,2-Dibromoethane and 4,5-bis[4-(acetamido)phenyl]-2-mercaptoimidazole,prepared according to the method of Example 1b) were combined to yield the desired 5,6-bis [4-(acetamido)phenyl]-2,3-dihydroimidazo[2,1-b]thiazole using the method of Example 1c).

**b) 5,6-Bis[4-(N-ethylacetamido)phenyl]-2,3-dihydroimidazo[2,1-b]thiazole**

A stirred solution of the compound of Example 2a) (18.6 g, 0.0470 mol) in dry dimethylformamide under nitrogen was chilled to -10 °C, treated with sodium hydride (50% oil dispersion, 6.8 g, 0.142 mol), and then warmed to room temperature to complete hydrogen evolution. This mixture was chilled to -10 °C, treated dropwise with a solution of bromoethane (10.9 g, 0.10 mol) in dry dimethylformamide (10 ml), and allowed to warm to room temperature. Additional bromoethane (0.51 g, 4.7 mmol) in dimethylformamide (2 ml) was added and the mixture stirred 1 hour. The reaction mixture was quenched portionwise under nitrogen with ice water (1 L) and extracted with methylene chloride. The extract was washed with water and dried with magnesium sulfate. Evaporation of the solvent gave a residue that was chromatographed on alumina with ethyl acetate as eluant and crystallized from ethyl acetate to afford 10.6 g (48%) of the desired product mp 170.5-171.5 °C.

c) **5,6-bis[4-N,N-diethylaminophenyl]-2,3-dihydroimidazo[2,1-b]thiazole. Formula (IC) compound**

A stirred solution of 0.85 g (1.9 mmoles) of 5,6-bis [4-(N-ethylacetamido)phenyl]-2,3-dihydroimidazo[2,1-b]thiazole of Example 1b) in 75 ml of dry tetrahydrofuran under argon was treated with 0.80 ml (8.0 mmoles) of a 10 M solution of borane-dimethylsulfide complex. After 72 hours at ambient temperature, the reaction mixture was refluxed for one hour and 50 ml of methanol added slowly to the cooled mixture. The solvent was removed in vacuo, and the residual complex suspended in methanol and removed in vacuo, and the residual complex suspended in methanol and 6N hydrochloric acid added to acidify. The mixture was heated under argon for several minutes on a steam bath, cooled to ice bath temperature and made basic with dilute aqueous sodium hydroxide. This mixture was extracted with methylene chloride and the extract dried over anhydrous potassium carbonate and concentrated in vacuo. The residue was chromatographed on alumina, eluted with methylene chloride-chloroform (1:1) and the major fraction recrystallized twice from methanol to give 0.27 g of the titled Formula (IC) compound. It had a melting point of 168-169 °C.

**EXAMPLE 3**

**5,6-bis(4-(1-piperidinyl)phenyl)-2,3-dihydroimidazo[2,1-b] thiazole. Formula (IC) compound.**

A mixture of 1.3 g (4.2 mmoles) of 5,6-bis(4-aminophenyl)-2,3-dihydroimidazo[2,1-b]thiazole, (prepared by refluxing the compound prepared in Example 2a with 6N hydrochloric acid followed by neutralization), 2.2 g (9.7 mmoles) of 1,5-dibromopentane, and 2.7 g (19.4 mmoles) of powdered potassium carbonate in 25 ml of dry dimethylformamide was heated to reflux for 1 hour. Another 2.7 g of powdered potassium carbonate and 2.2 g of 1,5-dibromopentane were added and refluxed for an additional 2 hours. Water was added and the mixture acidified and washed with methylene chloride. The aqueous phase was made alkaline and the product extracted into methylene chloride. The organic phase was dried over potassium carbonate and concentrated in vacuo. The residue was column chromatographed on silica and the product eluted with acetonitrile:methylene chloride (1:1). The solvent was removed in vacuo, and the residue was dissolved in methanol. Addition of ethereal hydrochloric acid gave the title Formula (IC) compound, mp 280-285 °C.

**EXAMPLE 4**

**5,6-bis(4-(1-pyrrolidinyl)phenyl)-2,3-dihydroimidazo[2,1-b] thiazole. Formula (IC) compound.**

A mixture of 1.3 g (4.2 mmoles) of 5,6-bis(4-aminophenyl)-2,3-dihydroimidazo(2,1-b)thiazole, prepared as in Example 3, 2.1 g (9.7 mmoles) of 1,4-dibro-mobutane, and 2.7 g (19.4 mmoles) of powdered potassium carbonate in 25 ml of dry dimethylformamide was refluxed for 2 hours under argon. The reaction mixture was poured into water, acidified and washed with methylene chloride. The aqueous phase was made basic and extracted with methylene chloride. The organic layer was dried over potassium carbonate, concentrated and column chromatographed on silica, eluting the product with ethyl acetate/methylene chloride (2:10). The solvent was evaporated and the solid residue recrystallized from methylene chloride-$CH_3OH$ to afford the title Formula (IC) compound, mp 235-237 ° C.

**EXAMPLE 5**

**5-(2-pyridinyl)-6-(4-fluorophenyl)-2,3-dihydroimidazo[2,1-b]thiazole and 5-(4-fluorophenyl)-6-(2-pyridinyl)-2,3-dihydroimidazo[2,1-b]thiazole. Formula (IC) compounds.**

**a) 1-(2-Pyridinyl)-2-(4-fluorophenyl)ethanone. Formula (X) compound.**

To a solution of 40.8 g (0.60 mol) of sodium ethoxide in 200 ml absolute ethanol was added a mixture of 60.5 g (0.40 mol) of ethyl picolinate and 54.1 g (0.40 mol) of 4-fluorophenylacetonitrile. The solution was refluxed 10 minutes, then cooled to ambient temperature. The solids were dissolved by the addition of 330 ml of water. Then, 50 ml 12N hydrochloric acid was added dropwise. The solid was collected by filtration, washed with water, and dried overnight (30 ° C in vacuo) to give 1-(2-pyridinyl)-2-cyano-2-(4-fluorophenyl)-ethen-1-ol. This compound was dissolved in 250 ml 48% hydrobormic acid and refluxed for 17 hours. Upon cooling, aqueous ammonium hydroxide was added until the reaction mixture was basic. The aqueous solution was extracted three times with chloroform. The combined organic extracts were washed with water, dried using magnesium sulfate, and concentrated. The resulting solid was chromatographed on silica gel with ether-petroleum ether (1:3) as eluant, and recrystallized from 2-propanol-hexane to give the title Formula (X) compound, m.p. 56-58 ° C.

**b) 1-(2-Pyridinyl)-2-(4-fluorophenyl)ethanone oxime (Formula (XII) compound).**

A solution of 18.5 g (0.086 mol) of 1-(2-pyridinyl)-2-(4-fluorophenyl)ethanone, prepared as described in part a, 52.7 g (0.387 mol) of sodium acetate trihydrate, and 19.6 g (0.282 mol) of hydroxylamine hydrochloride in 280 ml methanol-water (1:1) was refluxed for 1 hour. Upon cooling to 5 ° C, the precipitate was collected by filtration, washed with water, and dried overnight (30 ° C in vacuo). The title Formula (XII) compound was recrystallized from methanol-water, m.p. 106 ° C.

**c) 1-(2-Pyridinyl)-2-(4-fluorophenyl)ethanone, 0-[(4-methylphenyl)sulfonyl]oxime. (Formula (XIII) compound.**

To a solution of 19.6 g (0.085 mol) of 1-(4-pyridinyl)-2-(4-fluorophenyl)ethanone, oxime, prepared as described in part b, in 100 ml of dry pyridine at 0 ° C under argon was added 20.3 g (0.106 mol) of p-toluenesulfonyl chloride. The mixture was stirred at 25 ° C for 20 hours, and then poured into ice/water. The solid was collected by filtration and dried (25 ° C in vacuo to give the title Formula (XIII) compound, m.p. 120-122 ° C.

**d) 4-(2-Pyridinyl)-5-(4-fluorophenyl)imidazole-2-thione. (Formula (IX) compound).**

A suspension of 30.7 g (0.08 mol) of 1-(2-pyridinyl)-2-(4-fluorophenyl)ethanone, 0-[(4-methylphenyl) sulfonyl]oxime, prepared as described in Example 5c), in 170 ml absolute ethanol at 5 ° C under argon was treated with a solution of 8.6 g (0.10 mol) of potassium ethoxide in 90 ml of absolute ethanol. The suspension was stirred at 5 ° C for 1 hour. Then, 260 ml of ether was added, and the reaction mixture was stirred for an additional 90 minutes. The suspension was then filtered and washed with ether. The ethereal solution was washed four times with 10% hydrochloric acid. The combined aqueous acid extracts were concentrated in vacuo, then redissolved in 150 ml water. Then 15.5 g (0.16 mol) of potassium thiocyanate

was added, and the reaction was refluxed for 1 hour. After cooling, the reaction mixture was poured into 5% sodium bicarbonate solution. The solid was collected by filtration and dried overnight (30 ° C in vacuo) to give the title Formula (IX) compound, m.p. 248-250 ° C.

**e) 5-(2-Pyridinyl)-6-(4-fluorophenyl)-2,3-dihydroimidazo [2,1-b)thiazole and 5-(4-fluorophenyl)-6-(2-pyridinyl)-2,3-dihydroimidazo[2,1-b]thiazole. (Formula (IC) compounds.**

A solution of 9.76 g (0.036 mol) of 4-(2-pyridinyl)-5-(4-fluorophenyl)-imidazole-2-thione, prepared as described in part d, in 250 ml of dry N,N-dimethyl-formamide was treated with 3.52 g (0.073 mol) of a 50% oil dispersion of sodium hydride. The solution was stirred for 30 minutes, followed by the addition of 13.95 g (0.074 mol) of 1,2-dibromoethane. The reaction mixture was stirred for 3 hours, and then poured into ice/water. The aqueous suspension was extracted several times with methylene chloride. The combined organic layers were washed with water, dried using magnesium sulfate, and concentrated. The products were separated by chromatography on silica gel with 10% acetonitrile in methylene chloride and 25% acetonitrile in methylene chloride as eluants. Each product was recrystallized from acetonitrile-hexane to give the title Formula (IA) compounds 5-(2-pyridinyl)-6-(4-fluorophenyl)-2,3-dihydroimidazo[2,1-b]thiazole, m.p. 152-154 ° C, and 5-(4-fluorophenyl)-6-(2-pyridinyl)-2,3-dihydroimidazo[2,1-b]thiazole, m.p. 164-166 ° C.

**EXAMPLE 6**

**6-(4-pyridyl)-5-(3,4-methylenedioxyphenyl)-2,3-dihydroimidazo[2,1-b]thiazole and 5-(4-pyridyl)-6-(3,4-methylenedioxyphenyl)-2,3-dihydroimidazo[2,1-b]thiazole. Formula (IC) compounds.**

**a) Isonicotinaldehyde-0-benzoylcyanohydrin**

To an aqueous solution (375 ml) of sodium cyanide (91.5 g, 1.86 mmole) and benzyltriethyl ammonium chloride (15 g, 65 mmole) was added isonicotinaldehyde (50 g, 467 mmole) in 500 ml of methylene chloride at 0 ° C. Vigorous stirring was maintained for 15 minutes and a methylene chloride (250 ml) solution of benzoyl chloride (70 g, 500 mmole) was slowly added. Cooling was discontinued after one-half hour reaction and the mixture was allowed to reach ambient temperature. The organic layer was separated and washed with 5% sodium carbonate and brine, dried and evaporated to an oil which was thoroughly extracted with ether. The ethereal solution was concentrated to approximately 100 ml and allowed to crystallize to give 32.0 g of isonicotinaldehyde-0-benzoylcyanohydrin.

**b) 6-(4-pyridyl)-5-(3,4-methylenedioxyphenyl)-2,3-dihydroimidazo[2,1-b]thiazole and 5-(4-pyridyl)-6-(3,4-methylenedioxyphenyl)-2,3-dihydroimidazo[2,1-b]thiazole**

The cyanohydrin (8.3 g, 33 mmole) prepared in part a) above was stirred in 150 ml of tert-butyl alcohol with 3,4-methylenedioxybenzaldehyde (5.0 g, 33 mmole) and sodium hydride (33 mmole) was added. Stirring continued for 1-1/2 hours at room temperature and a mineral oil suspension of potassium hydride (24% suspension, 11.1 ml, 63 mmole) was added with caution. After 1-1/2 hours the reaction was quenched in 600 ml ice-water and extracted with chloroform. The chloroform extract was evaporated, and the resulting hydroxy ketone Formula VIII compound immediately dissolved in 150 ml dimethylformamide. To this was added thiourea (5.68 g, 75 mmole) and the solution was brought to reflux. After 4 hours the solvent was concentrated to half of the original volume then diluted with about 75 ml water to precipitate the mercaptoimidazole product, a Formula (IV) compound.

This mercaptoimidazole (0.25 g, 0.84 mmole) was suspended in 10 ml dimethylformamide and sodium hydride (0.88 mmole) was added. Salt formation was allowed to proceed a room temperature for 1/2 hour at which time 1-bromo-2-chloroethane was added from a syringe and the solution was stirred overnight under an argon atmosphere. Solid anhydrous potassium carbonate (0.185 g, 1.35 mmole) was added and the reaction mixture was refluxed for 3 hours. Dilution of the dimethylformamide solution with ice-water to 50 ml caused precipitation of the organic products which were separated by flash silica chromatography eluted with methanol:methylene chloride, (2:98) to afford the titled Formula (IC) compounds.

The 6-pyridyl isomer eluted first, and was recrystallized from methanol to give the 6-(4-pyridyl) product, mp 213.5-214.5 ° C. The 5-(4-pyridyl) product eluted next, and was also recrystallized from methanol, mp 177.5-178.5 ° C.

**EXAMPLE 7**

**5-(4-pyridyl)-6-(4-acetamidophenyl)-2,3-dihydroimidazo [(2,1-b)]thiazole and 6-(4-pyridyl)-5-(4-acetamidophenyl)-2,3-dihydroimidazo[2,1-b]thiazole. Formula (IC) compounds.**

The cyanohydrin prepared as in Example 6a (12.5 g, 50 mmole) was stirred in 225 ml of tert-butyl alcohol with 4-acetamidobenzaldehyde (8.0 g, 50 mmole) and sodium hydride (50 mmole) was added. Stirring continued for 1-1/2 hours at room temperature and a mineral oil suspension of potassium hydride (24% suspension, 16.7 ml, 94.5 mmole) was added with caution. After 1-1/2 hours the reaction was quenched in 1000 ml ice-water and extracted with chloroform. The chloroform extract was evaporated, and the resulting hydroxy ketone, a Formula VIII compound, immediately dissolved in 250 ml. dimethylformamide. To this was added thiourea (7.57 g, 100 mmole) and the solution was brought to reflux. After 4 hours the solvent was concentrated to half of the original volume then diluted with about 100 ml water to precipitate the thione product, a Formula (IX) compound.

The above prepared thione (0.79 g, 2.5 mmole) was suspended in 25 ml dimethylformamide and sodium hydride (2.6 mmole) was added. Salt formation was allowed to proceed at room temperature for 1/2 hour at which time 1-bromo-2-chloroethane was added from a syringe and the solution was stirred overnight under an argon atmosphere. Solid anhydrous potassium carbonate (0.55 g, 4.0 mmole) was added to the reaction mixture and the reflux was initiated for 3 hours. Dilution of the dimethylformamide solution with ice-water to 100 ml caused precipitation of the organic products to afford the titled Formula (IC) compounds which were separated by chromatography on silica eluted with methanol-methylene chloride (2:98), then a second column with isopropyl alcohol. The 5-(4-pyridyl) product (0.05 g) eluted before the 6-(4-pyridyl) product (0.03 g) in the second column.

**EXAMPLE 8**

**5-(4-pyridyl)-6-(4-(pyrrolidin-1-yl)phenyl)-2,3-dihydroimidazo[2,1-b]thiazole. Formula (IC) compound.**

**a) 6-(4-pyrrolidin-1-yl)phenyl)-2,3-dihydroimidazo[2,1-b] thiazole. Formula (K) compound.**

To 250 ml of pyridine dried over potassium hydroxide was added 50 g (0.37 mole) of p-aminoacetophenone and 39.6 g of acetic anhydride. After one and a half hours of stirring at room temperature, the solution had become a thick suspension of a white solid. Filtration and drying gave 44 g of p-acetamidoacetophenone. This solid (44 g, 0.25 mole) was then suspended in 500 ml methylene chloride and treated with 44 g of bromine (0.275 mole). The reaction was allowed to proceed overnight whereupon it was stripped and dried under high vacuum, then suspended in 200 ml absolute ethanol and treated with 60 g 2-aminothiazoline (0.59 mole). The reaction was stirred for 2 days then stripped, taken up in water and extracted with methylene chloride. The organic phase was washed with water, brine and was dried with sodium sulfate. Flash column chromatography with 2% methanol/98% methylene chloride gave 10.2 g (0.039 mole) of the Formula (K) compound, 6-acetamidophenyl-2,3-dihydro-imidazo[2,1-b]thiazole.

The amide described above (10.2 g, 0.039 mole) was refluxed in 200 ml of 6N hydrochloric acid for one hour, cooled, neutralized and extracted with methylene chloride. The organic layer was washed with brine, dried over sodium sulfate and evaporated to give 6.8 g of the Formula (III) compound 6-aminophenyl-2,3-dihydro-imidazo[2,1-b]thiazole.

To 6.8 g (0.034 mole) of the amine described above in 150 ml of dry dimethylformamide was added 8.4 g (0.039 mole) 1,4-dibromobutane and 15.5 g potassium carbonate (0.112 mole). The reaction was stirred at room temperature overnight, the dimethylformamide removed under high vacuum and the residue flash chromato-graphed on silica with 3% methanol/97% methylene chloride to give (after recrystallization from methanol) 0.88 g of the title Formula (K) compound, m.p. 218-220 °C (dec.).

**b) 5-(4-pyridyl)-6-(4-(pyrrolidin-1-yl)-phenyl)-2,3-dihydroimidazo[2,1-b]thiazole. Formula (IC) compound.**

The tertiary amine prepared in Example 8(a) above (4.0 g, 14.8 mmole) was suspended in methylene chloride (100 ml) and 3.58 ml pyridine was added. The mixture was cooled to 0 °C (ice bath). To this cooled solution was added ethyl chloroformate (4.73 g, 5.34 ml, 44 mmole; in 5 ml methylene chloride) dropwise over one hour. The solution was stirred at room temperature for one hour then heated to reflux for ten minutes. The solution was then cooled, 1.2 ml pyridine added, then dropwise ethyl chloroformate (1.6 g,

14.8 mmole) was added. The solution was heated to relux for 5 minutes. The solution was cooled and poured into ice water. This mixture was extracted with methylene chloride and the organic layer washed with water (3x50 ml). The methylene chloride was then stripped to afford the Formula (IV) compound. Decalin (20 ml) was added to the flask followed by sulfur (0.488 g, 14.8 mmole) and the mixture heated (with an oil bath) to 180 °C for 45 minutes. The reaction mixture was then diluted with methylene chloride (250 ml) and extracted with 12% hydrochloric acid (3x100 ml). The acidic layer was made basic with solid potassium carbonate until pH 9.0, then extracted with methylene chloride (5x200 ml). The organic layer was dried, then concentrated, and flash chromatographed on silica, twice eluted with methanol:methylene chloride (2:98) to give .24 g of the titled Formula (IC) compound.

## EXAMPLE 9

### 6-(4-fluorophenyl)-5-(4-pyridyl)-2,3-dihydroimidazo[2,1b]thiazole-1,1-dioxide. Formula (IA) compound.

A solution of 1.008 g (6.38 mmoles) of potassium permanganate dissolved in 100 ml of water was added over one hour to a solution of 3.0 g (9.6 mmole) of 6-(4-fluorophenyl)-5-(4-pyridyl)-2,3-dihydroimadazo-[2,1-b]thiazole-1-oxide in 200 ml of water containing 0.935 g of concentrated hydrochloric acid. Each drop of permanganate was decolorized almost instantaneously. After complete addition of the permanganate, the precipitated manganese dioxide was dissolved by passing sulfur dioxide thru the mixture. The resulting liquid was neutralized with 5% aqueous sodium hydroxide solution and extracted with methylene chloride. The extract was washed with water, brine, and evaporated giving 2.6 g of crude product. Flash chromatography on silica with 2-5% methanol/methylene chloride followed by recrystallization from methanol gave 1.9 g of 6-(4-fluorophenyl)-5-(4-pyridyl)-2,3-dihydroimidazo[2,1b]thiazole-1,1-dioxide. TLC $R_f = 61$, silica, 5%MeOH/95%$CH_2Cl_2$, mp 250 (dec).

## EXAMPLE 10

### 5,6-bis(4-cyanophenyl)-2,3-dihydroimidazo[2,1-b]thiazole. Formula (IC) compound

#### a) 2-Oxo-1,2-di(4-cyanophenyl)ethane. Formula (X) compound.

To a solution of 25 g of p-cyanobenzaldehyde in 50 ml of ethanol was added 5 g of potassium cyanide in 10 ml of water. The mixture was heated at reflux for 1 hr, cooled, and the crystalline product was filtered and washed with cold ethanol. Recrystallization from acetic acid afforded 4.7 g of the part (a) title compound, mp. 209-215 °C.

#### b) 1-Bromo-2-oxo-1,2-di-(4-cyanophenyl)ethane. Formula (XI) compound.

A mixture of 3.8 g of 2-oxo-1,2-di(4-cyanophenyl) ethane, prepared as described above in Example 10 (a), and 7.2 g of cuprous bromide in 500 ml of ethylacetate and 500 ml of chloroform was refluxed for 3 1/2 hours. The mixture was cooled, filtered through celite and the filtrate was evaporated to dryness. The residue was redissolved in ethyl acetate, washed with water, dried over magnesium sulfate and evaporated to dryness. The residue was triturated with ether to afford a crystalline product which was recrystallized from methanol-ether to afford the part (b) title compound, 3.9 g, mp. 167-170 °.

#### c) 5,6-bis(4-cyanophenyl)-2,3-dihydroimidazo[2,1-b) thiazole. Formula (IC) compound.

A mixture of 3.8 g of 1-bromo-2-oxo-1,2-di(4-cyanophenyl)ethane, prepared as described above in part (b) and 3.8 g of 2-aminothiazoline in 70 ml of dimethylformamide was stirred at room temperature for 18 hours. The reaction mixture was diluted with cold water, and the precipitated product was filtered and dried in vacuum. The material was suspended in 200 ml of toluene, 125 mg of p-toluenesulfonic acid was added, and the mixture was refluxed for 2 hours with removal of water. After cooling, the crude product was filtered and recrystallized twice from acetic acid to afford the part (c) title compound, 1.3 g, m.p. 255-260 °.

**EXAMPLE 11**

**6-(4-(1-Propylamino)phenyl)-5-(4-pyridyl)-2,3-dihydroimidazo[2,1-b]thiazole. Formula (IC) compound.**

**a) 6-(4-N-propylacetamido)phenyl-2,3-dihydroimidazo[2,1-b] thiazole. Formula (K) compound.**

6-acetamidophenyl-2,3-dihydroimidazo[2,1-b]-thiazole (1.0 g, 0.0038 mole), prepared as described in Example 8, was suspended in 25 ml dry dimethylformamide, and sodium hydride (50% in oil, 0.21 g, 0.0046 mole) was added. The suspension slowly dissolved and after stirring at room temperature for 30 min., n-propylbromide (.522 g, 0.0042 mole) was added to the reaction. The reaction was heated to 80 °C for one hour whereupon the dimethylformamide was removed under reduced pressure. The residue was then flash chromatographed (5% methanol/95% methylene chloride) to give 0.6 g of 6-(4-(N-propylacetamido)phenyl)-2,3-dihydroimidazo[2,1-b]thiazole (Formula (III) compound) (TLC: $R_f$ = 0.31, silica, 2.5% MeOH/97.5% methylene chloride).

**b) 6-(4-(1-propylamino)phenyl)-5-(4-pyridyl)-2,3-dihydroimidazo[2,1-b]thiazole. Formula (IC) compound.**

The N-propylacetamido Formula (K) compound (0.60 g, 0.002 mole), prepared in Example 11a above, was then suspended in methylene chloride (10 ml) and dry pyridine (0.47 ml, 0.006 mole) was added. The suspension was heated to aid in dissolving the solid, then cooled to ice-bath temperature. Ethyl chloroformate (0.72 ml, 0.648 g, 0.006 mole) in 2 ml methylene chloride was added dropwise to this mixture over a one hour period. The solution was stirred at room temperature for one hour and then heated at reflux for 10 min. The reaction was then cooled to 10 °C and an additional equivalent of pyridine (161 ul) and ethyl chloroformate (240 ul) was added. After heating at reflux for 10 min., the reaction was left to stir at room temperature overnight. The reaction was then washed with water (3 x 100 ml) and the organic layer stripped, then decalin (5 ml) and sulfur (0.063 g, 0.002 mole) were added and the mixture was heated to 170 °C. This temperature was maintained for one hour. The reaction was then diluted with methylene chloride and extracted with a 12% hydrochloric acid solution. The acidic layer was basified with solid potassium carbonate and then extracted with methylene chloride. After treating with brine and magnesium sulfate, the methylene chloride was removed, and the residue was flash chromatographed with methylene chloride containing 0 to 10% methanol as eluant to give 0.2 g of a (Formula (IC) compound), 6-(4-(N-propylacetamido)phenyl)-5-(4-pyridyl)-2,3-dihydroimidazo[2,1-b]thiazole (TLC: $R_f$ = 0.22, silica, 5% MeOH/95% methylene chloride).

The N-propylacetamido Formula (IB) compound (0.10 g, 0.26 mmole) was refluxed in 10 ml of 6N hydrochloric acid for one hour, cooled, neutralized and extracted with methylene chloride. The organic layer was washed with brine, dried over sodium sulfate and evaporated to give 0.082 g of (6-(4-(N-propylamino)-phenyl)-5-(4-pyridyl)2,3-dihydroimidazo[2,1-b]thiazole, (Formula (IC) compound), as a glassy material (TLC: $R_f$ = 0.35, 8% MeOH/92% methylene chloride, MS indicates presence of title compound, $(M+H)^+$ at 337).

**EXAMPLE 12**

**5,6-Bis(4-pyridyl)-2,3-dihydroimidazo(2,1-b)thiazole. Formula (IC) compound.**

10 g (0.10 mole) of 4-pyridine carboxaldehyde was slowly added to a solution of 7.6 g (0.1 mole) of thiourea and 2.0 g (0.03 mole) of potassium cyanide in 50 ml water at 0 °C. The solution was stirred at 0 °C for one hour, and then at ambient temperature overnight. A yellow precipitate of 4-pyridoin formed, which was filtered, dried and used without further purification.

A mixture of 1.1 g (5.4 mmoles) of the 4-pyridoin, prepared as described above, and 0.7 g (9.2 mmoles) of the thiourea was heated in 20 ml of dimethylacetamide at reflux for 6 hours. 4,5-Bis-(4-pyridyl)-2-mercaptoimidazole, a compound of Formula (IX), precipitated after dilution with water.

A solution of 2.90 g (11.5 mmoles) of the 2-mercaptoimidazole described above in 50 ml of dimethylformamide was treated with 1.64 g (11.5 mmoles) of 1-bromo-2-chloroethane, and stirred at 100 °C for 1 hour. After treatment with a second 1.64 g of the dihaloethane, the reaction mixture was heated at 120 °C for an additional hour and then cooled. 4.2 g (30.4 mmoles) of powdered potassium carbonate was added and the mixture heated to reflux for one hour. The reaction mixture was concentrated in vacuo to 40 ml, diluted with cold water, and extracted into methylene chloride. The organic phase was dried over

sodium carbonate and concentrated in vacuo. The residue was column chromatographed on alumina and eluted with chloroform. Evaporation of the solvent gave an oil which crystallized on trituration with ether to afford the title Formula (IC) compound, mp 219-222 °C.

**EXAMPLE 13**

**3-(4-Pyridyl)-2-(4-methoxyphenyl)-5,6,7,8-tetrahydroimidazo[1,2-a]pyridine. Formula (IC) compound)**

**a) 2-(4-methoxyphenyl)-5,6,7,8-tetrahydro-imidazo[1,2-a]pyridine, Formula (E) compound.**

A solution of 15.3 g (0.071 mole) of 25% (by weight) sodium methoxide in absolute methanol was added to a stirred solution of 10 g (0.074 mole) of 2-iminopiperidine hydrochloride in 40 ml of absolute methanol, chilled in an ice bath. The solvent was removed in vacuo, and the resin dissolved in 40 ml of chloroform. This solution was added dropwise under argon to a stirred solution of 17.4 g (0.074 mole) of 2-bromo-4'-methoxy-acetophenone in 150 ml of dry chloroform at 15 °C. After addition, the solution was stirred for 4 hours at ambient temperature and then concentrated in vacuo. The resin was dissolved in a minimal amount of methylene chloride and ether added to afford a heavy oily layer. The supernatant was decanted, and the oil layer dried of solvent in vacuo to afford a compound of Formula (H). This residue was dissolved in a minimal amount of hot water and the stirred solution heated on a steam bath under argon for 15 hours. On cooling, a precipitate formed which was filtered, made alkaline with aqueous sodium hydroxide and extracted into ethyl acetate. The organic layer was dried over potassium carbonate, filtered and concentrated in vacuo. The solid was triturated with hexane and air dried to give the titled product, mp 124-126 °C.

**b) 3-(N-ethyloxycarbonyl-1,4-dihydro-4-pyridyl)-2-(4-methoxyphenyl)-5,6,7,8-tetrahydroimidazo[1,2-a]pyridine, Formula (F) compound.**

A stirred solution of 2.7 g (11.8 mmoles) of 2-(4-methoxyphenyl)-5,6,7,8-tetrahydroimidazo[1,2-a] pyridine, prepared as described in Example 13(a) above, and 16.84 g (213 mmoles) of dry pyridine in 30 ml of dry methylene chloride over argon, was treated dropwise over two hours at ambient temperature in a water bath with 7.7 g (71 mmoles) of ethyl chloroformate. After 48 hours, another 3.84 g (35.4 mmoles) of ethyl chloroformate was added over 2 hours. The mixture was stirred overnight, poured into ice water, made alkaline and extracted into methylene chloride. The organic phase was suequentially washed with 0.2 N hydrochloric acid, water and aqueous potassium carbonate solution, dried over sodium sulfate and stripped in vacuo to afford the titled compound as a resin.

**c) 3-(4-pyridyl-2-(4-methoxyphenyl)-5,6,7,8-tetrahydroimidazo[1,2-a]pyridine, (Formula (IC) compound).**

2.7 g (7.1 mmoles) of the compound prepared as described in Example 13(b) above, was heated with stirring in 25 ml of decalin under argon. Upon reaching 100 °C, the solid dissolved, and 0.34 g (10.7 mmoles) of sulfur was added. The mixture was heated to 160 ° C for 30 minutes and another 0.34 g (10.7 mmoles) of sulfur added. After another 45 minutes, the reaction mixture was cooled, diluted with 25 ml of petroleum ether and extracted with acetonitrile. The acetonitrile phase was separated, and concentrated in vacuo to a resin. The resin, dissolved in methulene chloride, was extracted with 3 N hydrochloric acid and the aqueous acid layer treated with chloroform and 5% sodium carbonate solution. The chloroform layer was dried over anhydrous potassium carbonate, concentrated in vacuo, and chromatographed on silica, eluting with chloroform: ethyl acetate (1:2), containing 2% methanol. Evaporation of the solvent gave an oil which was crystallized from toluene-hexane to give the titled product, mp 136.5-138 °C.

## EXAMPLE 14

### 2-(4-Fluorophenyl)-6,7-dihydro-(5H)-pyrrolo[1,2-a]imidazole, Formula (E) Compound

### Method A.

#### (a) 2-Chloro-1-(fluorophenyl)-ethanone, Formula (D) compound

A 12 L round bottomed flask was equipped with a thermometer, mechanical stirrer, Claisen adapter, addition funnel, and reflux condenser surmounted with an hydrochloric acid scrubber. The flask was charged with 768.82 g (8.0 mol) of fluorobenzene and methylene chloride (3200 ml). The stirred solution was cooled to 5°C. Solid anhydrous, aluminum chloride (1166.0 g, 8.74 mol) was added to the flask. The addition funnel was charged with chloroacetyl chloride (640 ml, 8.0 mol). Chloroacetyl chloride was added to the reaction over 1 hour, keeping the temperature below 15°C by cooling in an ice bath. The reaction was exothermic, and hydrochloric acid gas was evolved. When addition was complete, the addition funnel was charged with a solution of 600 ml concentrated hydrochloric acid diluted with 2400 ml ice water. This was carefully added to the reaction vessel, so that the temperature did not exceed 30°C. The first 1000 ml was added over 1 hour; the rest over another hour. After all solids were dissolved, the two phase solution was transferred to a 12 L separtory funnel. The layers were separated. The aqueous layer was washed with 2x800 ml methylene chloride. The combined organic layers were washed with 1500 ml 5% aqueous sodium bicarbonate, and 1000 ml brine, dried over 50 g magnesium sulfate, and filtered.

A 12 L round bottomed flask was charged with the methylene chloride solution, along with 4000 ml of ethanol. The thermometer and distillation head were attached to the flask. The stirred solution was heated until a constant temperature of distillate (80°C) was reached and maintained. Approximately 6800 ml solvent was removed. The remaining solution was allowed to cool, subsequently forming crystals. The crude product in ethanol (approximately 2000 ml) is ready for use in the next step.

#### b) 2-(4-Fluorophenyl)-6,7-dihydro-(5H)-pyrrolo[1,2-a]imidazole, Formula (E) compound

A stirred solution of 15 g (87 mmoles) of 2-chloro-4-fluoroacetophenone in 75 ml of SD 30 alcohol was treated at 25° C with 10.65 g (104 mmoles) of 2-iminopyrrolidine, resulting in an exothermic temperature rise to 40°C. After stirring for one hour, approximately 75 ml of ethyl acetate was added, and the mixture was extracted with dilute hydrochloric acid to dissolve the precipitate. The aqueous acidic extract was separated from the organic phase, adjusted to a pH between 4 and 5, and heated on a steam bath for 24 hrs. The solution was adjusted to pH 2, extracted with ether, brought to pH 8, and extracted with methylene chloride. The basic organic phase was chromatographed on silica, eluting with 4% methanol in methylene chloride. The residue obtained on concentration of the pooled fractions was recrystallized from carbon tetrachloride, mp 137.5-139°C.

### Method B.

#### (a) 1-(4-Fluorophenyl)-2-(2-iminopyrrolidin-1-yl)-ethanone hydrocholoride, Formula (H) compound

A stirred solution of 37.3 g (216 mmoles) of 2-chloro-1-(4-fluorophenyl)ethanone of Example 14(a) Method A in 70 ml of chloroform chilled in a methanol-ice bath between 15-18°C, was treated with a solution of 20 g (238 mmoles) of 2-iminopyrrolidine in 50 ml of chloroform at such a rate as to maintain the temperature of the reaction mixture. After an additional 2 hours, the mixture was triturated with 300 ml diethyl ether, filtered, and the crystals were washed with ether and recrystallized from alcohol to give white needles of the named Formula (H) compound, mp 207-208°C.

#### (b) 2-(4-Fluorophenyl)-6,7-dihydro-(5H)-pyrrolo [1,2-a]imidazole, Formula (E) Compound

An aqueous solution of 31 g (0.12 mole) of the named Formula (H) compound of Method B, part a above, was heated in 300 ml of water on a steam bath for 8 hours. The solution was adjusted to pH 6.5, and the resulting precipitate was filtered, dried under vacuum and recrystallized from carbon tetrachloride to give the named Formula (E) compound, mp 137.5-139°C.

**EXAMPLE 15**

**2-(4-fluorophenyl)-3-(4-pyridyl)-6,7-dihydro-[5H]-pyrrolo [1,2-a]imidazole, Formula (IC) Compound**

A stirred solution of 13.1 g (0.065 mole) of 2-(4-fluorophenyl)-6,7-dihydro-[5H]-pyrrolo[1,2-a]imidazole, prepared as described in Example 14, and 51.4 g (0.65 mole) of dry pyridine in 17 ml of dry methylene chloride at 22°-25°C was treated over 1.5 hours with 35.3 g (0.325 mmole) of ethyl chloroformate. The solution was stirred at 25°C overnight, and the treatment with pyridine and ethyl chloroformate repeated as before, followed by a 24 hour period of stirring. After 3 more treatments as described above, the solvent was removed in vacuo. The residue was dissolved in 5% aqueous sodium bicarbonate and extracted into methylene chloride. The organic phase was washed with 5% aqueous sodium bicarbonate and dried over anhydrous potassium carbonate. The volatile solvents were removed in vacuo and the residue extracted into methylene chloride. The organic phase was extracted repeatedly with 0.2M hydrochloric acid until traces of starting material were removed, then washed with 5% sodium carbonate solution, dried over potassium carbonate (anhydrous), and stripped in vacuo. The residue was crystallized from toluene-hexane to give the compound of Formula (F) known as 3-(N-ethoxy-carbonyl-1,4-dihydro-4-pyridyl)-2-(4-fluorophenyl)-6,7-dihydro[5H]-pyrrolo(1,2-a]imidazole, mp 146-147°C.

Method A. 0.5 g (1.4 mmoles) of the Formula (F) product described above was heated with stirring in 5 ml of decalin under argon. Upon reaching a temperature of 80°C, 0.06 g (1.8 mmoles) of sulfur was added and the mixture heated to 165°C until starting material was consumed. The cooled mixture was filtered and the solid washed with petroleum ether and dissolved in chloroformethyl acetate (1:1). This solution was decolorized with Darco, and chromatographed on silica. Elution with 20% methanol in chloroform-ethyl acetate (1:1) afforded a fraction which was concentrated in vacuo, and recrystallized from carbon tetrachloride to give the desired Example 15 title product, mp 163-164.5°C.

Method B. 15.0 g (42.4 mmoles) of a Formula (F) compound, i.e., 3-(N-ethoxycarbonyl-1,4-dihydro-4-pyridyl)-2-(4-fluorophenyl)-6,7-dihydro[5H)-pyrrolo[1,2-a]imidazole, prepared as described above, was added to a stirred solution of 28.6 g (255 mmoles) of potassium tert.-butoxide dissolved in tert.-butanol (250 ml) into which oxygen was being bubbled. The solution was heated to reflux for 15 minutes, and the solvent then removed in vacuo. The solid product was extracted into methylene chloride, washed with water and then extracted into aqueous 3N hydrochloric acid. This aqueous acidic phase was made basic with cold 10% aqueous sodium hydroxide and extracted with methylene chloride. The resulting organic phase was dried over anhydrous potassium carbonate and the solvent was removed in vacuo. Two recrystallizations from toluene gave the Example 15 title product, mp 165-166°C.

**EXAMPLE 16**

**3-(N-ethyloxycarbonyl-1,4-dihydro-4-pyridyl)-2-(4-methoxyphenyl)-6,7-dihydro-[5H]-pyrrolo[1,2-a]-imidazole, Formula (F) Compound**

**a) 2-(4-Methoxyphenyl)-6,7-dihydro-[5H]-pyrrolo [1,2-a]imidazole, Formula (E) Compound.**

To a solution of 6.8 g (29.7 mmoles) of 2-bromo-4'-methoxyacetophenone in 50 ml of chloroform was added a solution of 5 g (59.4 mmoles) of 2-iminopyrrolidine in 30 ml of chloroform with chilling. After 4 hours of stirring at 25 °C, the solvent was removed in vacuo. The residue was dissolved in water, the pH adjusted to 2.5 and the solution heated on a steam bath under argon atmosphere for 8 hours. The cooled solution was adjusted to pH 6. The resulting precipitate, filtered, washed with water and dried in vacuo to afford the titled compound, mp 116-117.5 °C.

**b) 3-(N-Ethyloxycarbonyl-1,4-dihydro-4-pyridyl)-2-(4-methoxyphenyl)-6,7-dihydro-[5H]-pyrrolo[1,2-a]-imidazole,Formula (F) compound**

A stirred solution of 2.8 g (13.1 mmoles) of 2-(4-methoxyphenyl)-6,7-dihydro-[5H]-pyrrolo[1,2-a] imidazole, prepared as described above, and 6.2 g (78.4 mmoles) of dry pyridine in 30 ml of dry methylene chloride was treated dropwise over 1 hour at 5 °C under argon atmosphere with 4.25 g (39.2 mmoles) of ethyl chloroformate. After stirring for 1 hour an additional 3.1 g (39.2 mmoles) of pyridine was added, followed by 2.15 g (19.8 mmoles) of ethyl chloroformate added over 2 hours. The mixture was stirred overnight at 25 °C, then poured into ice water made alkaline with sodium carbonate and extracted with methylene chloride. The organic phase was sequentially washed with 0.2 N hydrochloric acid, water, and

aqueous potassium carbonate solution, dried over sodium sulfate and stripped in vacuo to afford the titled compound as an amber resin.

**EXAMPLE 17**

**2-(4-methoxyphenyl)-3-(4-pyridyl)-6,7-dihydro-[5H]-pyrrolo [1,2-a]imidazole, Formula (IC) Compound**

4.1 g (11.2 mmole) of the named compound described in Example 16, prepared as described in Example 16, was heated with stirring in 25 ml of decalin under argon. Upon reaching 85 °C, the solid was dissolved, and 0.468 g (14.6 mmoles) of sulfur was added. The mixture was heated to 165 °C and another 0.235 g (7.3 mmoles) of sulfur was added. After another 45 minutes, the starting material was consumed, and the cooled reaction mixture was diluted with 25 ml of petroleum ether and filtered. The filtered solid was washed with additional petroleum ether, dissolved in chloroformethyl acetate and chromatographed on silica. The material eluting with 8 to 25% methanol in chloroform-ethylacetate (1:1) was concentrated in vacuo and recrystallized from toluene-cyclohexane to give the desired Formula (IC) compound, mp 157.5-158.5 °C.

**EXAMPLE 18**

**3-Bromo-2-(4-fluorophenyl)-6,7-dihydro-[5H]-pyrrolo[1,2-a]imidazole, Formula (G) Compound**

A stirred solution of 100 mg (0.50 mmole) of 2-(4-fluorophenyl)-6,7-dihydro-[5H]-pyrrolo[1,2-a]imidazole, prepared as described in Example 14, was treated dropwise with a solution of 90 mg (0.56 mmole) of bromine in 0.5 ml of methylene chloride. After 45 minutes, the solution was made basic with 5% aqueous NaOH and dried organics over anhydrous potassium carbonate. The solvent was removed in vacuo, and the residue was recrystallized from carbon tetrachloridehexane to give the desired title product, mp 188-189 °C dec.

**EXAMPLE 19**

**2,3-Bis(4-fluorophenyl)-6,7-dihydro-5H-pyrrolo[1,2-a]imidazole, Formula (IC) compound.**

**a) 4,4'-di-fluorodesoxybenzoin, Formula (X) compound**

A 250 ml round bottomed flask equipped with a mechanical stirrer, condenser with drying tube and solid addition apparatus was charged with a solution of 17.2 g (0.1 mole) acid chloride 19.2 g (0.2 mole) fluorobenzene and 75 ml of methylene chloride. To this solution was added 16.0 g (0.12 moles) aluminum chloride. The reaction mixture was heated to reflux for 1 hour and then cooled to room temperature. The reaction mixture was then poured into a beaker containing 40 ml of concentrated hydrochloric acid and about 100 g of ice. The resulting mixture turned purple. The organic portion was separated and the aqueous phase extracted twice with methylene chloride. The organic layers were combined and washed three times with 2.5% aqueous sodium bicarbonate, three times with water, and dried over anhydrous magnesium sulfate. Solvent was removed under reduced pressure to afford a pink solid.

The resulting product was treated with hot diethyl ether and cooled in the freezer. The light pink extracts were collected via vacuum filtration and air dried to afford 10.65 g of the desired product, mp 96-97 °C.

**b) 2-Bromo-1,2-di-(4-fluorophenyl)ethanone, Formula (XI) compound**

Bromine (7.99 g, 0.05 mol) was added dropwise to a stirred solution of the desoxybenzoin of Example 19(a) (0.05 mol) in carbon tetrachloride or benzene (100 mL). A slight excess of bromine was added to obtain a persistent orange color. Irradiation with a 275-W sunlamp was employed to enhance bromination. After 1 hour at room temperature, the solvent was evaporated to afford the crude product.

**c) 2,3-Bis(4-fluorophenyl)-6,7-dihydro-5H-pyrrolo[1,2-a]imidazole, Formula (IC) compound.**

A mixture of 10.0 g (0.0322 mol) of 2-bromo-1,2-di(4-fluorophenyl)ethanone of Example 19(b) and 8.1 g (0.0964 mol) of 2-iminopyrrolidine in 100 ml of dry dimethylformamide was stirred for 5 days at room

temperature. The resulting solution was then added to water and extracted with methylene chloride. The organic phase was washed several times with water, dried over potassium carbonate and evaporated to yield a tan solid. The crude material was recrystallized from methanol with charcoal and water to give the Formula (IC) compound as a white solid; 4.05 g (42.4%), mp 155.5 - 157 °C.

## UTILITY EXAMPLES

In the following Examples where mice were used, they were male Balb/c mice (20-28 g), and where rats were used, they were male Lewis rats (180-210 g). All mice and rats were obtained from Charles River Breeding Laboratories, Kingston, N.Y. Within a single experiment, mice and rats were sex and age matched.

In the following examples, reagents used were employed as follows:

Auranofin, phenidone, indomethacin, naproxen, and ibuprofen were each used as the free base. The compounds of Formula (IA) were used either as the free base or in the appropriate salt form. Levamisole was used as the hydrochloride salt. The compounds were homogenized in 0.5% tragacanth. Compounds were administered by gavage at the indicated dose in a final volume of 10 ml/kg. Nordihydroguaiaretic acid (NDGA) was solubilized in dimethylacetamide and diluted with olive oil for subcutaneous administration.

For in vitro experiments, compounds were dissolved at appropriate concentrations in ethanol or DMSO (dimethylsulfoxide) (final concentration <1.0%) and then diluted to final concentrations using the buffers indicated in the text.

## I. METHODS

### Mouse Carrageenan Peritonitis

Mice were pretreated with either the test compound or vehicle one hour before the intraperitoneal injection of a 1.0% carrageenan suspension in saline (0.2 ml/ mouse). Mice were sacrificed by cervical dislocation two hours after injection, and 3.0 ml of phosphate buffered saline, without $Ca^{++}$ or $Mg^{++}$, was injected into the peritoneum. Following massage, a 2.0 ml aliquot of the lavage fluid was removed, and the total cell count determined on a Coulter counter and differential cell count determined by microscopic examination of Giesma-stained slides. Data is summarized in Tables 1-4.

### Arachidonic Acid-Induced Mouse Ear Inflammation

Arachidonic acid in acetone (2 mg/20 ul) was applied to the inner surface of the left ear. The thickness of both ears was then measured with a dial micrometer one hour after treatment, and the data were expressed as the change in thickness ($10^{-3}$ cm) between treated and untreated ears.

Test compounds were given orally in 0.5% tragacanth at the times indicated in the text prior to the topical application of arachidonic acid.

Parenteral administration of compound was accomplished by subcutaneous injection of solution as indicated.

Data is summarized in Tables 7 and 7A.

### Arachidonic Acid-Induced Rat Paw Swelling

After determining pretreatment paw volumes by plethysmography by the method of Webb and Griswold, J. Pharmacol. Methods, 12, 149-153 (1984), rats were given test compound or vehicle one hour prior to the subplantar injection of 0.1 ml of 1 mg/ml arachidonic acid. Paw volumes were then remeasured and compared to pretreatment values and the increase in paw volume was expressed as mean values + S.D. Data is summarized in Table 6.

### Carrageenan and Arachidonic Acid-Induced Air Pouch Inflammation

Rats were shaved on the dorsal flank, and then injected subcutaneously one day later with 20 ml of air to form a defined pouch, by the method of Sedgwick et al., J. Pathology, 141, 483-495 (1983). The air pouch was reinflated as necessary over the next 6 days. To assay anti-inflammatory activity, animals were treated orally with test compound or vehicle (10 ml/kg) one hour before injection of 2.0 ml of a 2.0% carrageenan suspension containing 200 units/ml penicillin and 100 $\mu$g/ml streptomycin into the air pouch. In other experiments, 5 ml of 0.1% arachidonic acid in 0.2 M bicarbonate buffer was injected two hours after

drug treatment. Animals were sacrificed using $CO_2$ three hours after instillation of irritant. The exudate was then aspirated from the pouch and neutrophil count and differential cell count were measured.
Data is summarized in Table 5.

**$PGE_2$ Production by Peritoneal Macrophages**

Mice were injected intraperitoneally with 350 $\mu$g Corynebacterium parvum and the peritoneal exudate cell population was harvested 12-14 days later by peritoneal lavage with cold phosphate-buffered saline, and resuspended in Eagles minimal essential medium supplemented with 5% fetal calf serum. The recovered cells, representing 95% macrophages as determined by morphology, phagocytic capacity and reactivity with a macrophage-specific monoclonal antibody [Koestler et al., Proc. Natl. Acad. Sci., USA, 81, 4504 (1984)], were placed in wells of 24-well microtiter plate ($10^6$ cells/900 ul) and allowed to adhere for 1 hour at 37°C. Test compounds were added (100 ul) to bring the final volume to 1.0 ml. Lipopolysaccharide (5 $\mu$g/ml) was then added to stimulate $PGE_2$ synthesis. After incubation at 37°C for 2 hours, cell-free supernatants were harvested, placed in polypropylene tubes and frozen at -20°C until assayed for their $PGE_2$ content using a commercial radioimmunoassay kit. Data is summarized in Table 10. $IC_{50}$ is the concentration that inhibits 50% of control activity.

**Assay of 5-Lipoxygenase and Cyclooxygenase Activities**

The activities of these enzymes in extracts of RBL-1 cells were assayed using the method of Jakschik and Lee, Nature, 287, 51-52 (1980). RBL-1 cells were obtained from the American Type Culture Collection (#CRL 1378) and were grown at 37°C (5% $CO_2$ in air) in spinner culture in MEM supplemented with 10% heat inactivated fetal calf serum. Harvested cells were washed with 50 mM sodium phosphate buffer, pH 7.0, containing 1 mM EDTA and 0.1% gelatin, resuspended in fresh buffer (5 x $10^7$ cells/ml) and disrupted by nitrogen cavitation using the Parr bomb at 750 psi for 10 min. The broken cell extract was then centrifuged at 10,000 x g for 20 minutes and the supernatant centrifuged at 100,000 x g for 60 minutes. Aliquots (0.25 mls) of the supernatant were preincubated with or without drugs for 10 min, after which 10 ul $CaCl_2$ (50 mM) was added and the reaction was initiated with 2.5 ul of 2.5 mM arachidonic acid-1-$^{14}C$ (final concentration was 25 mM; specific activity 20,000 dpm/nmole). After incubation for 3 min at 37°C, the reaction was terminated by addition of 2 volumes (0.5 ml) ice cold acetone and the sample was allowed to deproteinize on ice for 10 min prior to centrifugation at 1,000 x g for 10 min. The deproteinized supernatant was adjusted to pH 3.5 with 2N formic acid and extracted with 2 volumes of ice cold ethyl acetate. The extracted samples were dried under argon, redissolved in ethyl acetate and applied to Whatman LK5D thin layer chromatography (TLC) plates which were developed using the A-9 solvent system [organic phase of ethyl acetate: 2,2,5-trimethylpentane:acetic acid: water (110:50:20:10)] described by Hamberg and Samuelsson, J. Biol. Chem., 241, 257-263 (1966). Arachidonic acid, 5-HETE, di-HETE and $PGD_2$ were quantified with a Berthold LB 2832 autoscanner.

The 5-lipoxygenase and $LTA_4$ synthetase activities were further studied under the following conditions. An additional centrifugation of the RBL-1 supernatant was run at 100,000 x g for 60 min, to remove the particulate cyclooxygenase activity. Sample incubation was done under conditions similar to those described above, i.e., 2 mM $CaCl_2$ and 25 uM arachidonic acid-1-$^{14}C$, however, with an incubation time of 5 min at 5°C. Under these conditions, only the 5-lipoxygenase pathway metabolites were detectable. The 5-HETE and di-HETEs were formed at a linear rate, and substantial amounts of the arachidonic acid-1-$^{14}C$ substrate were utilized.

Drug-induced effects on enzyme activities are described as the concentration of drug causing a 50% inhibition of metabolite synthesis ($IC_{50}$). Data is summarized in Tables 8, 8A, 9, and 9A.

**Experimental Allergic Encephalomyelitis Induced in Female Lewis Rats**

The effect of Compound 1, 5-(4-pyridyl)-6-(4-fluorophenyl)-2,3-dihydroimidazo[2,1-b]thiazole, and indomethacin on hindleg paralysis in experimental allergic encephalomyelitis, hereinafter EAE, induced in female Lewis rats was evaluated according to the following protocol. EAE is induced in Female (Charles River) Lewis rats by a single intradermal injection of 0.1 ml of an encephalitogenic emulsion consisting of equial parts of a 50% w/v homogenate of guinea pig spinal cord and mid-brain in 0.5% aqueous phenol and Freund's complete adjuvant (4 mg/ml killed, dried M. butyricum) into a hindpaw (left) footpad. Within 9-11 days, the animals exhibit tail limpness, hindleg weakness, and decrease in body weight. By day 16, the animals develop complete hindleg paralysis and exhibit a further decrease in body weight. Any animal

which becomes paralyzed during the course of the experiment is considered to have developed EAE whether or not the paralysis is permanent. Test compounds are administered daily beginning on the day of injection, for 19 days, exclusive of days 5, 6 12 and 13. Drug activity is determined by comparing the indicidence of EAE (paralysis) of the treated group with a control (vehicle) group. The level of significant difference between the treated groups and control groups is determined by a Chi-square test using a 2x2 contingency table. Body weight changes from day 0 are also statistically compared to the control group using a Student "t" test. Data is summarized in Table 11.

**$LTC_4$ Production by Human Monocytes**

The compounds of Formula (IA) were evaluated for their ability to inhibit the production of $LTC_4$ by human monocytes according to the following assay. Human monocytes were prepared from whole blood supplied by the American Red Cross. The blood was fractionated by a two-step procedure employing sedimentation on Ficoll followed by sedimentation on Percoll. The mononuclear cell fraction recovered was composed of 80-90% monocytes with the remainder of the cells being predominantly lymphocytes. The monocytes were plated at $1x10^6$ cells per well in a Costar 24 well tissue culture plate and allowed to adhere for 1 hour at 37°C. Non-adherent cells were removed by washing. The cells were stimulated with 1 uM A23187 calcium ionophore for 3 hours at 37°C to induce $LTC_4$ production. When drugs were evaluated, they were added to the cells 30 minutes prior to the A23187. Supernatants were collected, clarified by centrifugation and stored frozen at -20°C until assay. The $LTC_4$ content was determined by using a New England Nuclear Leukotriene C-4 ($^3H$) RIA Kit as per instructions.
Drug induced effects are described as the concentration of drug causing a 50% inhibition of metabolite synthesis ($IC_{50}$). Data is summarized on Table 12.

## II. RESULTS

### The Effects of Compound 1 on Leukocyte Infiltration into Inflammatory Lesions

The effect of Compound 1, 5-(4-pyridyl)-6-(4-fluorophenyl)-2,3-dihydroimidazole[2,1-b]thiazole, on the infiltration of cells into inflammatory lesions was examined in several assay systems. As shown in Table 1, the infiltration of polymorphonuclear leukocytes (PMN) induced by intraperitoneal injection of carrageenan in mice is reduced dramatically by oral administration of Compound 1. The inhibition is dose-related, with an $ED_{50}$ of 43.9 mg/kg, p.o. Also, the leukotriene synthesis inhibitors phenidone (Table 1), NDGA and dexamethasone (Table 2) were effective in inhibiting PMN infiltration in this peritonitis model. In contrast, the cyclooxygenase inhibitors indomethacin (10 mg/kg, p.o.) and naproxen (100 mg/kg, p.o.), the organo-gold compound auranofin (2 mg Au/kg, p.o.) and levamisole (100 mg/kg, p.o.) did not impair inflammatory cell infiltration in this assay system despite the use of near maximally tolerated doses (Table 3). Compound 1 also produced significant inhibition of polymorphonuclear leukocyte infiltration into rat air pouch inflammatory lesions induced by carrageenan (Table 4). The reduction in PMN infiltration was accompanied by a relative increase in mononuclear cell counts. A reduction in the PMN count and the PMN:mononuclear cell ratio was also produced by phenidone (100 mg/kg, p.o.) and a high dose of indomethacin (5 mg/kg, p.o.). Unlike the mouse carrageenan model, this assay system was sensitive to the anti-inflammatory activity of indomethacin.

### The Effect of Compound 1 on Arachidonic Acid-induced Inflammation

In order to help define the anti-inflammatory properties of Compound 1, 5-(4-pyridyl)-6-(4-fluorophenyl)-2,3-dihydroimidazo[2,1-b]thiazole, inflammatory lesions were induced using arachidonic acid. As shown in Table 5, Compound 1 and phenidone significantly reduced PMN and mononuclear cell infiltration into arachidonic acid-induced inflammation in the rat air pouch but indomethacin had no significant effect on cell infiltration. Further elucidation of the anti-inflammatory activity of Compound 1 was achieved in models of arachidonic acid-induced edema in mice and rats. The mouse ear edematous response to arachidonic acid has been shown to be sensitive to agents that inhibit both lipoxygenase and cyclooxygenase-generated mediators or that selectively inhibit lipoxygenase, but not cyclooxygenase, enzyme activity. The inflammatory response induced in rat paws by arachidonic acid injection was inhibited significantly by both Compound 1 and phenidone, but not by indomethacin (Table 6). Similarly, Compound 1 produced marked inhibition of the edematous response normally seen 1 hour after the application of 2 mg of arachidonic acid to the ear ($ED_{50}$ of 19.5 mg/kg, p.o.). The anti-inflammatory activity of Compound 1 in this assay is greater

than for phenidone ($ED_{50}$ = 44.0 mg/kg, p.o.) and for dexamethasone which was only moderately effective. The cyclooxygenase inhibitors, indomethacin (10 mg/kg, p.o.), ibuprofen (250 mg/kg, p.o.) and naproxen (100 mg/kg, p.o.) did not exhibit detectable anti-inflammatory activity in this assay, despite use at near maximally tolerated doses (Table 7). Table 7A represents the results of testing other compounds of Formula (IA) in the arachidonic acid-induced ear swelling assay. Such results indicate that the compounds of Formula (IA) exhibit antiinflammatory activity in an assay in which selective cyclooxygenase inhibitors do not exhibit such activity.

Collectively, these findings indicate that Compound 1 is a potent inhibitor of both the cellular and edematous responses of inflammation in rats and mice. These inflammatory responses were also inhibited by agents that inhibit lipoxygenase activity but not by selective cyclooxygenase inhibitors.

**The Effect of Compound 1 on Arachidonic Acid Metabolism**

The generation of the 5-lipoxygenase product, 5-HETE, and the generation of the cyclooxygenase product, $PGD_2$, by RBL-1 cell extracts can be distinguished as shown by the inhibition of $PGD_2$ production by indomethacin ($IC_{50}$ = 2.5 uM) and the inhibition of 5-HETE synthesis by phenidone ($IC_{50}$ = 10 uM) (Table 8). The generation of both enzyme products was inhibited, however, by Compound 1, with $IC_{50}$ values of 75 uM and 100 uM for 5-HETE and $PGD_2$, respectively. The effect of other compounds of Formulae (IA) on the inhibition of 5-HETE is presented in Table 8A which indicates that compounds of Formula (IA) are inhibitors of the 5-lipoxygenase pathway as evidenced by their ability to inhibit 5-HETE a 5-lipoxygenase pathway product.

Additional experiments using a soluble extract preparation of RBL-1 cells containing only lipoxygenase activity (di-HETE production by RB1-cells) confirmed the inhibitory effects of Compound 1 on eicosanoid formation ($IC_{50}$ = 7.5 uM) (Table 9). Indomethacin at concentrations up to $10^{-4}$ M was inactive.

Table 9A represents the testing of compounds of Formula (IA) for their ability to inhibit 5-lipoxygenase activity. The results presented in Table 9A indicate that the compounds of Formula (IA) possess 5-lipoxygenase pathway inhibiting activity as evidenced by their ability to inhibit di-HETE generation, a 5-lipoxygenase pathway product.

The production of $PGE_2$ by inflammatory macrophages is inhibited by Compound 1, 5-(4-pyridyl)-6-(4-fluorophenyl)-2,3-dihydroimidazo[2,1-b]thiazole, (Table 10). The $IC_{50}$ value of 0.7 uM in this assay is comparable to those exhibited by the non-steroidal anti-inflammatory agents, ibuprofen and naproxen ($IC_{50}$ = 0.5 uM and 1.8 uM, respectively) and higher than that of indomethacin ($IC_{50}$ = 0.04 uM). Phenidone proved much less active in inhibiting $PGE_2$ production by inflammatory macrophages ($IC_{50}$ = 28 uM).

**The Effect of Compound 1 on Hindleg Paralysis in Experimental Allergic Encephalomyelitis Induced in Female Lewis Rats**

As shown in Table 11, Compound 1, but not indomethacin, was efficacious in inhibiting experimental allergic encephalomyelitis (EAE) in rats.

**$LTC_4$ Inhibition Assay**

As shown in Table 12, compounds of Formula (IA) were efficacious in inhibiting $LTC_4$ production by human monocytes. These data confirm the ability of compounds of Formula (IA) to inhibit the 5-lipoxygenase pathway, as evidenced by their ability to inhibit $LTC_4$, a 5-lipoxygenase pathway product.

As seen in Table 7A not all compounds of Formula (IA) significantly inhibited arachidonic acid-induced ear swelling, but such non-significant inhibitors of ear swelling did significantly inhibit the production of 5-HETE by RBL-1 high speed supernatant (Table 8A), the production of di-HETE by RBL-1 high speed supernatant (Table 9A) and/or the production of $LTC_4$ by human monocytes (Table 12) indicating that such compounds are inhibitors of the 5-lipoxygenase pathway.

TABLE 1

| Infiltration of Polymorphonuclear Leukocytes into Sites of Carrageenan-Induced Inflammation | | |
|---|---|---|
| Treatment[a] | PMN x $10^{-5}$/ml (mean ± S.D.) | % Change |
| Vehicle | 10.90 ± 0.89 | -- |
| Compound 1* | | |
| 100 mg/kg, p.o.<br>50 mg/kg, p.o.<br>25 mg/kg, p.o. | 2.48 ± 0.98<br>4.84 ± 3.01<br>7.92 ± 4.15 | -77[b]<br>-56[c]<br>-27[e] |
| Phenidone | | |
| 200 mg/kg, p.o.<br>100 mg/kg, p.o.<br>50 mg/kg, p.o. | 0.66 ± 0.53<br>6.97 ± 3.81<br>5.30 ± 2.25 | -94[b]<br>-36[d]<br>-51[c] |

[a] Mice were pretreated with the compounds indicated one hour prior to the i.p. injection of carrageenan, and cellular infiltration was measured two hours later as described in the Methods. The data represent mean values (± S.D.) derived from measurements on five animals in each treatment group.
[b] Statistically significant: $P<0.001$.
[c] Statistically significant: $P<0.01$.
[d] Statistically significant: $P<0.05$.
[e] Not Significant.
* 5-(4-pyridyl)-6-(4-fluorophenyl)-2,3-dihydroimidazo[2,1-b]thiazole

TABLE 2

| Infiltration of Polymorphonuclear Leukocytes into Sites of Carrageenan-Induced Inflammation | | |
|---|---|---|
| Treatment[a] | PMN x $10^{-5}$/ml (mean ± S.D.) | % Change |
| Experiment 1 | | |
| Vehicle, p.o. | 19.8 ± 3.2 | -- |
| Dexamethasone | | |
| 50 mg/kg, p.o.<br>25 mg/kg, p.o.<br>12.5 mg/kg, p.o. | 6.1 ± 1.5<br>8.8 ± 2.6<br>9.1 ± 2.0 | -69[b]<br>-55[b]<br>-54[b] |
| Experiment 2 | | |
| Vehicle, s.c. | 9.7 ± 3.1 | -- |
| NDGA | | |
| 50 mg/kg, s.c. | 5.7 ± 2.1 | 41[c] |

[a] Mice were pretreated with the compounds indicated one hour prior to the i.p. injection of carrageenan, and cellular infiltration was measured two hours later as described in the Methods. The data represent mean values (± S.D.) derived from measurements on 5 animals in each treatment group.
[b] Statistically significant at a $P<0.001$.
[c] Statistically significant at a $P<0.01$.

TABLE 3

| Infiltration of Polymorphonuclear Leukocytes into Sites of Carrageenan- Induced Inflammation | | | |
|---|---|---|---|
| Treatment[a] | Dosage mg/kg, p.o. | PMN x $10^{-5}$/ml (Mean ± S.D.) | % Change |
| Indomethacin | 10 | 8.20 ± 2.65 | -12[b] |
| Naproxen | 100 | 10.28 ± 2.49 | -11[b] |
| Levamisole | 100 | 5.88 ± 2.70 | -37[b] |
| Auranofin | 2 (mg Au/kg) | 7.50 ± 1.57 | - 6[b] |

[a] Experiments were conducted using the protocol described in Table 1. The results represent mean values (± S.D.) derived from measurements on 5 animals/group. Control values for these experiments ranged from 7.8 ± 2.59 to 11.49 ± 3.56 PMN x $10^{-5}$/ml.

[b] Not statistically significant.

TABLE 4

| Carrageenan-Induced Cellular Infiltration into the Rat "Air Pouch."[a] | | | | |
|---|---|---|---|---|
| Treatment | Exudate Volume (ml) | Cellular Infiltrate (Total x $10^{-6}$) | | |
| | | PMN[b] | MN[c] | PMN/MN Ratio |
| Control | 1.8 ± 0.8 | 6.3 ± 4.0 | 2.8 ± 0.7 | 2.21 ± 1.09 |
| Phenidone (100 mg/kg) | 1.5 ± 0.8 | 1.7 ± 0.6[d] | 3.5 ± 1.4 | 0.52 ± 0.13[d] |
| Compound 1* (100 mg/kg) | 1.0 ± 0.9 | 2.6 ± 1.3[d] | 5.5 ± 3.8[e] | 0.50 ± 0.14[d] |
| Indomethacin (5 mg/kg) | 2.0 ± 0.5 | 2.8 ± 0.7[d] | 4.0 ± 0.8 | 0.69 ± 0.19[d] |

[a] Cell infiltration was measured 3 hours after injection of carrageenan into a preformed air pouch as described in the Methods. The results represent mean values (± S.D.) derived from measurements on 8 animals.

[b] Polymorphonuclear leukocytes.

[c] Mononuclear leukocytes.

[d] Statistically significant at a $P<0.01$.

[e] Statistically significant at a $P<0.05$.

* 5(4-pyridyl)-6-(4-fluorophenyl)-2,3-dihydroimidazo[2,1-b]thiazole

TABLE 5

| Arachidonic Acid-Induced Cellular Infiltration into the Rat "Air Pouch."[a] | | | | |
|---|---|---|---|---|
| Treatment | Exudate Volume (ml) | Cellular Infiltrate (Total x $10^{-6}$) | | |
| | | PMN[b] | MN[c] | PMN/MN Ratio |
| Control | 2.8 ± 0.7 | 4.4 ± 3.7 | 9.8 ± 4.7 | 0.65 ± 0.62 |
| Compound 1* (100 mg/kg) | 2.6 ± 0.8 | 1.2 ± 0.7[e] | 1.5 ± 1.1[d] | 1.20 ± 0.88 |
| Phenidone (100 mg/kg) | 2.8 ± 0.4 | 1.3 ± 0.8[e] | 2.2 ± 1.5[d] | 0.74 ± 0.39 |
| Indomethacin (5 mg/kg) | 2.5 ± 0.8 | 5.0 ± 3.8 | 7.0 ± 6.4 | 0.94 ± 0.83 |

[a] Cell Infiltration was measured 3 hours after injection of arachidonic acid into a preformed air pouch as described in the Methods. The results represent mean values (± S.D.) derived from measurement of 6 to 8 animals.
[b] Polymorphonuclear leukocytes.
[c] Mononuclear leukocytes.
[d] Statistically significant at a P<0.01.
[e] Statistically significant at a P<0.05.
* 5-(4-pyridyl)-6-(4-fluorophenyl)-2,3-dihydroimidazo[2,1b]thiazole

TABLE 6

| Arachidonic Acid-Induced Rat Paw Edema.[a] | |
|---|---|
| Treatment | Change in Paw Volume (ml) (% Inhibition) |
| Control | 0.27 ± 0.05 |
| Compound 1* (100 mg/kg) | 0.06 ± 0.05[b] (78) |
| Phenidone (100 mg/kg) | 0.13 ± 0.05[b] (52) |
| Indomethacin (5 mg/kg) | 0.29 ± 0.06 ( 0) |

[a] Animals were treated with the indicated compounds one hour before subplantar injection of arachidonic acid. The results represent mean values (± S.D.) derived from measurements on 8 animals read at 2 hours post arachidonic acid injection.
[b] Statistically significant at a P<0.01.
* 5-(4-pyridyl)-6-(4-fluorophenyl)-2,3-dihydroimidazo[2,1-b]thiazole

TABLE 7

| Arachidonic Acid-Induced Inflammation of the Mouse Ear.[a] | | | |
|---|---|---|---|
| Treatment | Dose (mg/kg, p.o.) | Increase in Ear Thickness at 1 Hour (x $10^{-3}$ cm) | % Change |
| Compound 1* | 50 | 10.0 ± 1.5 | -67[b] |
| Phenidone | 50 | 12.2 ± 1.6 | -57[b] |
| Dexamethasone | 25 | 18.2 ± 4.1 | -35[c] |
| Indomethacin | 10 | 24.4 ± 0.8 | - 5 |
| Naproxen | 100 | 26.4 ± 2.6 | + 3 |
| Ibuprofen | 250 | 30.8 ± 2.0 | +20[b] |

[a] Compounds were administered 15 minutes (dexamethasone was pretreated at 2 hrs) before application of arachidonic acid to the ear as described in the Methods. The results represent mean values (± S.D.) derived from measurements on 5 animals. Control values for these experiments ranged from 28.1 ± 0.8 to 30.0 ± 1.3.

[b] Statistically significant at a $P<0.001$.

[c] Statistically significant at a $P<0.05$

* 5-(4-pyridyl)-6-(4-fluorophenyl)-2,3-dihydroimidazo[2,1-b]thiazole

TABLE 7A. Arachidonic Acid-Induced Ear Swelling

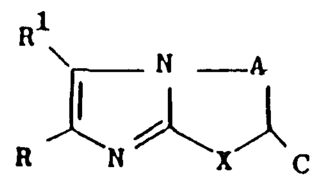

Formula (IA) (C)

| Compound Number | R' | R | X | A | n | % Inhibition(A,B) of Ear Swelling |
|---|---|---|---|---|---|---|
| 2 | 4-pyridyl | 4-(1-propylamino)phenyl | S(O)n | $CH_2$ | 0 | 23* |
| 3 | 4-(pyrrolidin-1-yl)phenyl | 4-(pyrrolidin-1-yl)phenyl | S(O)n | $CH_2$ | 0 | 44*** |
| 4 | 4-(piperidin-1-yl)phenyl | 4-(piperidin-1-yl)phenyl | S(O)n | $CH_2$ | 0 | 22* |
| 5 | 4-pyridyl | 3,4-(methylenedioxy)phenyl | S(O)n | $CH_2$ | 0 | NS |
| 6 | 4-fluorophenyl | 4-dimethylaminophenyl | S(O)n | $CH_2$ | 0 | 48*** |
| 7 | 3,4-(methylenedioxy)phenyl | 4-pyridyl | S(O)n | $CH_2$ | 0 | 62*** |
| 8 | 4-fluorophenyl | 4-fluorophenyl | $CH_2$ | $CH_2$ | - | 40***(p.o.) |
| 9 | 4-diethylaminophenyl | 4-diethylaminophenyl | S(O)n | $CH_2$ | 0 | 17* (p.o.) |
| 10 | 4-dimethylaminophenyl | 4-fluorophenyl | S(O)n | $CH_2$ | 0 | 17* |
| 11 | 4-pyridyl | 4-pyridyl | S(O)n | $CH_2$ | 0 | 29*** |
| 12 | 4-fluorophenyl | 4-pyridyl | S(O)n | $CH_2$ | 0 | 58*** |
| 13 | 4-cyanophenyl | 4-cyanophenyl | S(O)n | $CH_2$ | 0 | 50*** |
| 14 | 4-fluorophenyl | 4-methoxyphenyl | S(O)n | $CH_2$ | 0 | 64*** |
| 15 | 4-trifluoromethylphenyl | 4-trifluoromethylphenyl | S(O)n | $CH_2$ | 0 | NS (p.o.) |
| 16 | 3,4-methylenedioxyphenyl | 3,4-methylenedioxyphenyl | S(O)n | $CH_2$ | 0 | 32***(p.o.) |
| 17 | 4-ethylaminophenyl | 4-ethylaminophenyl | S(O)n | $CH_2$ | 0 | 38*** |
| 18 | 4-pyridyl | 4-methoxyphenyl | S(O)n | $CH_2$ | 0 | 40***(p.o.) |
| 19 | 4-fluorophenyl | 4-fluorophenyl | S(O)n | $CH_2$ | 0 | 34***(p.o.) |
| 20 | 4-methoxyphenyl | 4-methoxyphenyl | S(O)n | $CH_2CH_2$ | 0 | 47*** |
| 21 | 4-methoxyphenyl | 4-methoxyphenyl | S(O)n | $CH_2$ | 0 | NS (p.o.) |
| 22 | 4-methoxyphenyl | 4-methoxyphenyl | $CH_2$ | $CH_2$ | - | 15* |
| 23 | 2-pyridyl | 4-fluorophenyl | S(O)n | $CH_2$ | 0 | 65*** |
| 24 | 4-fluorophenyl | 2-pyridyl | S(O)n | $CH_2$ | 0 | 69*** |
| 25 | 4-pyridyl | 4-(pyrrolidin-1-yl)phenyl | S(O)n | $CH_2$ | 0 | 17* (p.o.) |

TABLE 7A. (con't)

| Compound Number | $R^1$ | R | X | A | n | % Inhibition(A,B) of Ear Swelling |
|---|---|---|---|---|---|---|
| 26 | 4-fluorophenyl | 4-fluorophenyl(hydrate) | S(O)n | $CH_2$ | 0 | NT |
| 27 | 4-fluoromethylphenyl | 4-trifluoromethylphenyl (hydrate) | S(O)n | $CH_2$ | 0 | NT |
| 28 | 4-pyridyl | 4-fluorophenyl | S(O)n | $CH_2$ | 1 | 69*** |
| 29 | 4-pyridyl | 4-fluorophenyl | S(O)n | $CH_2$ | 2 | 67*** (p.o.) |
| 30 | 4-fluorophenyl | 4-fluorophenyl | S(O)n | $CH_2$ | 1 | NT |
| 31 | 4-fluorophenyl | 4-fluorophenyl | S(O)n | $CH_2$ | 2 | NT |
| 32 | 4-methylthiophenyl | 4-methylthiophenyl | S(O)n | $CH_2$ | 0 | NS (p.o.) |
| 33 | phenyl | phenyl | S(O)n | $CH_2$ | 0 | NT |
| 34 | 4-methylphenyl | 4-methylphenyl | S(O)n | $CH_2$ | 0 | NT |
| 35 | 4-(prop-2-ene-1-oxy)phenyl | 4-(prop-2-ene-1-oxy)phenyl | S(O)n | $CH_2$ | 0 | NT |
| 36 | 4-(2,2,2-trifluoroethoxy) phenyl | 4-(2,2,2-trifluoroethyoxy) phenyl | S(O)n | $CH_2$ | 0 | NT |
| 37 | 3,4,5-trimethoxyphenyl | 3,4,5-trimethoxyphenyl | S(O)n | $CH_2$ | 0 | NT |
| 38 | 4-pyridyl | 4-acetamidophenyl | S(O)n | $CH_2$ | 0 | NT |
| 39 | 4-pyridyl | 4-fluorophenyl | $CH_2$ | $CH_2$ | 0 | 93 |
| 40 | 4-pyridyl | 4-methoxyphenyl | $CH_2$ | $CH_2$ | 0 | 83 (p.o.) |
| 41 | 4-pyridyl | 4-methoxyphenyl | S(O)n | $CH_2$ | 1 | 45*** |
| 42 | 4-fluorophenyl | 4-pyridyl | S(O)n | $CH_2$ | 1 | 50*** |
| 43 | 4-methoxyphenyl | 4-methoxyphenyl | S(O)n | $CH_2$ | 2 | NS |
| 44 | 4-hydroxyphenyl | 4-hydroxyphenyl | $CH_2$ | $CH_2$ | - | NS |
| 45 | 4-aminophenyl | 4-aminophenyl | S(O)n | $CH_2$ | 0 | NS |
| 46 | 4-methylthiophenyl | 4-fluorophenyl | S(O)n | CH | 0 | NS |
| 47 | 4-fluorophenyl | 4-methylthiophenyl | S(O)n | $CH_2$ | 0 | 22*** |
| 48 | N-ethoxycarbonyl-1,4-dihydro-4-pyridyl | 4-fluorophenyl | S(O)n | $CH_2$ | 0 | 25*** |
| 49 | N-phenylcarbonyl-1,4-dihydro-4-pyridyl | 4-fluorophenyl | S(O)n | $CH_2$ | 0 | 15* |

A) Screened at 50 mg/kg s.c. or i.p. unless indicated as oral dosing (p.o.).
B)* = p<.05, ** = p<.01, ***p<.001, NS = not significant, NT = not tested
C) B and C are H for all compounds

TABLE 8

| Cyclooxygenase and 5-Lipoxygenase Activities in RBL-1 Cells.[a] | | |
|---|---|---|
| Treatment | $IC_{50}$ ($\mu$M) | |
| | Cyclooxygenase | 5-Lipoxygenase |
| Compound 1* | 100 | 75 |
| Phenidone | Inactive @ 100 | 10 |
| Indomethacin | 2.5 | Inactive @ 30 |

[a] Enzyme activities were measured by the amount of $PGD_2$ and 5-HETE produced from $^{14}C$-labeled arachidonic acid incubated with the 10,000 x g supernatant of RBL-1 cells as described in the Methods.

* 5-(4-pyridyl)-6-(4-fluorophenyl)-2,3-dihydroimidazo[2,1-b]thiazole

TABLE 8A. The Effect of Compounds of Formula (IA) on 5-HETE Production

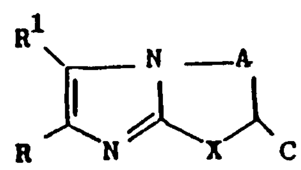

Formula (IA) (E)

| Compound Number | $R^1$ | R | X | A | n | 5-HETE[(A)] $IC_{50}$ (μM) |
|---|---|---|---|---|---|---|
| 2 | 4-pyridyl | 4-(1-propylamino)phenyl | S(O)n | $CH_2$ | 0 | 12 |
| 3 | 4-(pyrrolidin-1-yl)phenyl | 4-(pyrrolidin-1-yl)phenyl | S(O)n | $CH_2$ | 0 | .23 |
| 4 | 4-(piperidin-1-yl)phenyl | 4-(piperidin-1-yl)phenyl | S(O)n | $CH_2$ | 0 | 1.2 |
| 5 | 4-pyridyl | 3,4-(methylenedioxy) phenyl | S(O)n | $CH_2$ | 0 | 10 |
| 6 | 4-fluorophenyl | 4-dimethylaminophenyl | S(O)n | $CH_2$ | 0 | .80 |
| 7 | 3,4-(methylenedioxy)phenyl | 4-pyridyl | S(O)n | $CH_2$ | 0 | 10 |
| 8 | 4-fluorophenyl | 4-fluorophenyl | $CH_2$ | $CH_2$ | - | 12 |
| 9 | 4-diethylaminophenyl | 4-diethylaminophenyl | S(O)n | $CH_2$ | 0 | .35 |
| 10 | 4-dimethylaminophenyl | 4-fluorophenyl | S(O)n | $CH_2$ | 0 | 5 |
| 11 | 4-pyridyl | 4-pyridyl | S(O)n | $CH_2$ | 0 | 110 |
| 12 | 4-fluorophenyl | 4-pyridyl | S(O)n | $CH_2$ | 0 | 16 |
| 13 | 4-cyanophenyl | 4-cyanophenyl | S(O)n | $CH_2$ | 0 | 16 |
| 14 | 4-fluorophenyl | 4-methoxyphenyl | S(O)n | $CH_2$ | 0 | 2 |
| 15 | 4-trifluoromethylphenyl | 4-trifluoromethylphenyl | S(O)n | $CH_2$ | 0 | 10 |
| 16 | 3,4-methylenedioxyphenyl | 3,4-methylenedioxyphenyl | S(O)n | $CH_2$ | 0 | 3.8 |
| 17 | 4-ethylaminophenyl | 4-ethylaminophenyl | S(O)n | $CH_2$ | 0 | .10 |
| 18 | 4-pyridyl | 4-methoxyphenyl | S(O)n | $CH_2$ | 0 | 5.4 |
| 19 | 4-fluorophenyl | 4-fluorophenyl | S(O)n | $CH_2$ | 0 | 8.6 |
| 20 | 4-methoxyphenyl | 4-methoxyphenyl | S(O)n | $CH_2CH_2$ | 0 | 5.6 |
| 21 | 4-methoxyphenyl | 4-methoxyphenyl | S(O)n | $CH_2$ | 0 | 3.9 |
| 22 | 4-methoxyphenyl | 4-methoxyphenyl | $CH_2$ | $CH_2$ | 0 | 4.0 |
| 23 | 2-pyridyl | 4-fluorophenyl | S(O)n | $CH_2$ | 0 | 90 |
| 24 | 4-fluorophenyl | 2-pyridyl | S(O)n | $CH_2$ | 0 | 37 |

TABLE 8A. (cont'd)

| Compound Number | $R^1$ | R | X | A | n | 5-HETE [A] [B] $IC_{50}$ (μM) |
|---|---|---|---|---|---|---|
| 25 | 4-pyridyl | 4-(pyrrolidin-1-yl)phenyl | S(O)n | $CH_2$ | 0 | .5 |
| 26 | 4-fluorophenyl | 4-fluorophenyl(hydrate) | S(O)n | $CH_2$ | 0 | >10(C) |
| 27 | 4-trifluoromethylphenyl | 4-trifluoromethylphenyl (hydrate) | S(O)n | $CH_2$ | 0 | 28 |
| 28 | 4-pyridyl | 4-fluorophenyl | S(O)n | $CH_2$ | 1 | 1000 |
| 29 | 4-pyridyl | 4-fluorophenyl | S(O)n | $CH_2$ | 2 | 100 |
| 30 | 4-fluorophenyl | 4-fluorophenyl | S(O)n | $CH_2$ | 1 | 70 |
| 31 | 4-fluorophenyl | 4-fluorophenyl | S(O)n | $CH_2$ | 2 | 33 |
| 32 | 4-methylthiophenyl | 4-methylthiophenyl | S(O)n | $CH_2$ | 0 | >10(D) |
| 33 | phenyl | phenyl | S(O)n | $CH_2$ | 0 | NT |
| 34 | 4-methylphenyl | 4-methylphenyl | S(O)n | $CH_2$ | 0 | NT |
| 35 | 4-(prop-2-ene-1-oxy)phenyl | 4-(prop-2-ene-1-oxy)phenyl | S(O)n | $CH_2$ | 0 | NT |
| 36 | 4-(2,2,2-trifluoroethoxy)phenyl | 4-(2,2,2-trifluoroethoxy) phenyl | S(O)n | $CH_2$ | 0 | NT |
| 37 | 3,4,5-trimethoxyphenyl | 3,4,5-trimethoxyphenyl | S(O)n | $CH_2$ | 0 | NT |
| 38 | 4-pyridyl | 4-acetamidophenyl | S(O)n | $CH_2$ | 0 | 69 |
| 39 | 4-pyridyl | 4-fluorophenyl | $CH_2$ | $CH_2$ | 0 | 67 |
| 40 | 4-pyridyl | 4-methoxyphenyl | $CH_2$ | $CH_2$ | 0 | 16 |
| 41 | 4-pyridyl | 4-methoxyphenyl | S(O)n | $CH_2$ | 1 | NT |
| 42 | 4-fluorophenyl | 4-pyridyl | S(O)n | $CH_2$ | 1 | 101 |
| 43 | 4-methoxyphenyl | 4-methoxyphenyl | S(O)n | $CH_2$ | 2 | 10 |
| 44 | 4-hydroxyphenyl | 4-hydroxyphenyl | $CH_2$ | $CH_2$ | - | 17 |
| 45 | 4-aminophenyl | 4-aminophenyl | S(O)n | $CH_2$ | 0 | 8 |
| 46 | 4-methylthiophenyl | 4-fluorophenyl | S(O)n | CH | 0 | 4 |
| 47 | 4-fluorophenyl | 4-methylthiophenyl | S(O)n | $CH_2$ | 0 | NT |
| 48 | N-ethoxycarbonyl-1,4-dihydro-4-pyridyl | 4-fluorophenyl | S(O)n | $CH_2$ | 0 | 9 |
| 49 | N-phenylcarbonyl-1,4-dihydro-4-pyridyl | 4-fluorophenyl | S(O)n | $CH_2$ | 0 | 73 |

[A] $IC_{50}$ determined on 5-HETE production RBL-1 high speed supernatant

[B] NT = not tested

[C] 46% inhibition at 10 μM

[D] 35-40% inhibition at 10 μM

[E] B and C are H for all compounds

TABLE 9

| Production of Di-HETE and 5-HETE by the High Speed Supernatant of RBL-1 Cells.[a] | | | | |
|---|---|---|---|---|
| Treatment | Di-HETE (nM/ml) | % Control | 5-HETE (nM/ml) | % Control |
| Control | 1.94 ± 0.11 | -- | 10.15 ± 0.65 | -- |
| Compound 1* | | | | |
| 1 uM | 1.56 ± 0.06 | 19.7[b] | 8.86 ± 0.31 | 12.7[b] |
| 3.3 uM | 1.34 ± 0.12 | 30.9[b] | 7.63 ± 0.44 | 24.9[b] |
| 10 uM | 0.88 ± 0.21 | 54.8[b] | 5.57 ± 0.35 | 45.1[b] |
| 33 uM | 0.40 ± 0.08 | 79.6[b] | 3.36 ± 0.07 | 67.9[b] |
| 100 uM | 0.03 ± 0.04 | 98.3[b] | 1.08 ± 0.13 | 89.4[b] |

[a] Enzyme activities were measured by the production of 5-HETE and Di-HETE from $^{14}C$-labeled arachidonic acid incubated with a high speed supernatant of RBL-1 cells (see Methods for details). The results represent mean values (± S.D.) derived from measurements on 4 replicate analyses.

[b] Statistically significant from Control at a P<0.01 or better.

* 5-(4-pyridyl)-6-(4-fluorophenyl)-2,3-dihydroimidazo[2,1-b]thiazole

TABLE 9A. The Effect of Compounds of Formula (IA) on Lipoxygenase Activity (di-HETE Production)

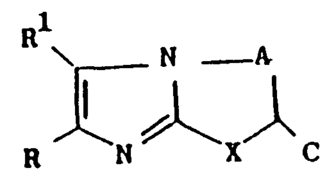

Formula (IA)[(C)]

| Compound Number | $R^1$ | R | A | X | n | 5-LO[(A)] $IC_{50}$(μM) |
|---|---|---|---|---|---|---|
| 2 | 4-pyridyl | 4-(1-propyl)aminophenyl | $CH_2$ | S(O)n | 0 | 7 |
| 3 | 4-(pyrrolidin-1-yl)phenyl | 4-(pyrrolidin-1-yl)phenyl | $CH_2$ | S(O)n | 0 | 0.1 |
| 4 | 4-(piperidin-1-yl)phenyl | 4-(piperidin-1-yl)phenyl | $CH_2$ | S(O)n | 0 | 0.6 |
| 5 | 4-pyridyl | 3,4-(methylenedioxy)phenyl | $CH_2$ | S(O)n | 0 | 10.0 |
| 6 | 4-fluorophenyl | 4-dimethylaminophenyl | $CH_2$ | S(O)n | 0 | 0.6 |
| 7 | 3,4-(methylenedioxy)phenyl | 4-pyridyl | $CH_2$ | S(O)n | 0 | 10.0 |
| 8 | 4-fluorophenyl | 4-fluorophenyl | $CH_2$ | $CH_2$ | | 8.0 |
| 9 | 4-diethylaminophenyl | 4-diethylaminophenyl | $CH_2$ | S(O)n | 0 | 0.16 |
| 10 | 4-dimethylaminophenyl . | 4-fluorophenyl | $CH_2$ | S(O)n | 0 | 1.5 |
| 11 | 4-pyridyl | 4-pyridyl | $CH_2$ | S(O)n | 0 | 33.0 |
| 12 | 4-fluorophenyl | 4-pyridyl | $CH_2$ | S(O)n | 0 | 17.0 |
| 13 | 4-cyanophenyl | 4-cyanophenyl | $CH_2$ | S(O)n | 0 | 15.0 |
| 14 | 4-fluorophenyl | 4-methoxyphenyl | $CH_2$ | S(O)n | 0 | 0.6 |
| 15 | 4-trifluoromethylphenyl | 4-trifluoromethylphenyl | $CH_2$ | S(O)n | 0 | 10.0 |
| 16 | 3,4-methylenedioxyphenyl | 3,4-methylenedioxyphenyl | $CH_2$ | S(O)n | 0 | 1.5 |
| 17 | 4-ethylaminophenyl | 4-ethylaminophenyl | $CH_2$ | S(O)n | 0 | 0.1 |
| 18 | 4-pyridyl | 4-methoxyphenyl | $CH_2$ | S(O)n | 0 | 1.9 |
| 19 | 4-fluorophenyl | 4-fluorophenyl | $CH_2$ | S(O)n | 0 | 5.0 |
| 20 | 4-methoxyphenyl- | 4-methoxyphenyl | $CH_2CH_2$ | S(O)n | 0 | 5.6 |
| 21 | 4-methoxyphenyl | 4-methoxyphenyl | $CH_2$ | S(O)n | 0 | 2.8 |
| 22 | 4-methoxyphenyl | 4-methoxyphenyl | $CH_2$ | $CH_2$ | | NT |
| 23 | 2-pyridyl | 4-fluorophenyl | $CH_2$ | S(O)n | 0 | 42 |
| 24 | 4-fluorophenyl | 2-pyridyl | $CH_2$ | S(O)n | 0 | 20 |
| 25 | 4-pyridyl | 4-(pyrrolidin-1-yl)phenyl | $CH_2$ | S(O)n | 0 | 0.5 |
| 26 | 4-fluorophenyl | 4-fluorophenyl(hydrate) | $CH_2$ | S(O)n | 0 | >10 |

TABLE 9A. (cont'd)

| Compound Number | $R^1$ | R | A | X | n | 5-LO[A] [B] $IC_{50}$ (μM) |
|---|---|---|---|---|---|---|
| 27 | 4-trifluoromethylphenyl | 4-trifluoromethylphenyl (hydrate) | $CH_2$ | S(O)n | 0 | NT |
| 28 | 4-pyridyl | 4-fluorophenyl | $CH_2$ | S(O)n | 1 | 1000 |
| 29 | 4-pyridyl | 4-fluorophenyl | $CH_2$ | S(O)n | 2 | >100 |
| 30 | 4-fluorophenyl | 4-fluorophenyl | $CH_2$ | S(O)n | 1 | 60 |
| 31 | 4-fluorophenyl | 4-fluorophenyl | $CH_2$ | S(O)n | 2 | <33 |
| 32 | 4-methylthiophenyl | 4-methylthiophenyl | $CH_2$ | S(O)n | 0 | >100 |
| 33 | phenyl | phenyl | $CH_2$ | S(O)n | 0 | NT |
| 34 | 4-methylphenyl | 4-methylphenyl | $CH_2$ | S(O)n | 0 | NT |
| 35 | 4-(prop-2-ene-oxy)phenyl | 4-(prop-2-ene-oxy)phenyl | $CH_2$ | S(O)n | 0 | NT |
| 36 | 4-(2,2,2-trifluoroethoxy)phenyl | 4-(2,2,2-trifluoroethyoxy) phenyl | $CH_2$ | S(O)n | 0 | NT |
| 37 | 3,4,5-trimethoxyphenyl | 3,4,5-trimethoxyphenyl | $CH_2$ | S(O)n | 0 | NT |
| 38 | 4-pyridyl | 4-acetamidophenyl | $CH_2$ | S(O)n | 0 | 30 |
| 39 | 4-pyridyl | 4-fluorophenyl | $CH_2$ | $CH_2$ | 0 | 32 |
| 40 | 4-pyridyl | 4-methoxyphenyl | $CH_2$ | $CH_2$ | 0 | 10 |
| 41 | 4-pyridyl | 4-methoxyphenyl | $CH_2$ | S(O)n | 1 | NT |
| 42 | 4-fluorophenyl | 4-pyridyl | $CH_2$ | S(O)n | 1 | 60 |
| 43 | 4-methoxyphenyl | 4-methoxyphenyl | $CH_2$ | S(O)n | 2 | 10 |
| 44 | 4-hydroxyphenyl | 4-hydroxyphenyl | $CH_2CH_2$ | $CH_2$ | - | 11 |
| 45 | 4-aminophenyl | 4-aminophenyl | $CH_2$ | S(O)n | 0 | 6 |
| 46 | 4-methylthiophenyl | 4-fluorophenyl | CH | S(O)n | 0 | 3 |
| 47 | 4-fluorophenyl | 4-methylthiophenyl | $CH_2$ | S(O)n | 0 | NT |
| 48 | N-ethoxycarbonyl-1,4-dihydro-4-pyridyl | 4-fluorophenyl | $CH_2$ | S(O)n | 0 | 7 |
| 49 | N-phenylcarbonyl-1,4-dihydro-4-pyridyl | 4-fluorophenyl | $CH_2$ | S(O)n | 0 | 53 |

A) $IC_{50}$ determined on di-HETE production by $RBL^{-1}$ high speed supernatant.

B) Not tested = NT

C) B and C are H for each compound.

TABLE 10

| The Effect of Compound 1*, Indomethacin, Ibuprofen, Naproxen and Phenidone on the Production of $PGE_2$ by Mouse Peritoneal Exudate Cells | |
|---|---|
| Compound | $IC_{50}$ ($\mu$M) |
| Compound 1* | 0.7 |
| Indomethacin | 0.04 |
| Ibuprofen | 0.5 |
| Naproxen | 1.8 |
| Phenidone | 28.0 |

[a] Release of $PGE_2$ was measured by RIA of cell free supernatants from LPS-stimulated, C. parvum-elicited peritoneal exudate cells as described in the Methods.

* 5-(4-pyridyl)-6-(4-fluorophenyl)-2,3-dihydroimidazo 2,1b]thiazole

TABLE 11

| Hindleg Paralysis in Experimental Allergic Encephalomyelitis Induced in Lewis Female Rats | | |
|---|---|---|
| COMPOUND (DOSE)*** | HINDLEG PARALYSIS - CUMULATIVE INCIDENCE | |
| | DAY 9 TO 13 | DAY 14 TO 18 |
| Compound 1* (60) | 10/12 | 0/11** |
| INDOMETHACIN (2) | 6/11 | 10/11 |
| METHOTREXATE (0.3) | 0/11** | 0/11** |
| CONTROL | 12/16 | 14/16 |

* 5-(4-pyridyl)-6-(4-fluorophenyl)-2,3-dihydroimidazo[2,1-b]thiazole

** Significantly different from control (p<0.01).

*** Mg/kg/day, p.o. days 0-4, 7-11, 14-18.

TABLE 12. Effect of Compounds of Formula (IA) in Inhibiting $LTC_4$ Production

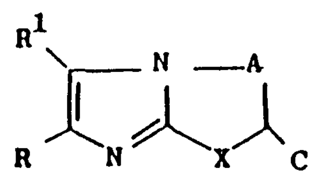

Formula (IA)[(D)]

| Compound Number | $R^1$ | R | A | X | n | $IC_{50}$ (A) (B) (µM) |
|---|---|---|---|---|---|---|
| 1 | 4-pyridyl | 4-fluorophenyl | $CH_2$ | S(O)n | 0 | 1.8(C) |
| 2 | 4-pyridyl | 4-(1-propyl)aminophenyl | $CH_2$ | S(O)n | 0 | NT |
| 3 | 4-(pyrrolidin-1-yl)phenyl | 4-(pyrrolidin-1-yl)phenyl | $CH_2$ | S(O)n | 0 | NT |
| 4 | 4-(piperidin-1-yl)phenyl | 4-(piperidin-1-yl)phenyl | $CH_2$ | S(O)n | 0 | NT |
| 5 | 4-pyridyl | 3,4-(methylenedioxy)phenyl | $CH_2$ | S(O)n | 0 | NT |
| 6 | 4-fluorophenyl | 4-dimethylaminophenyl | $CH_2$ | S(O)n | 0 | NT |
| 7 | 3,4-(methylenedioxy)phenyl | 4-pyridyl | $CH_2$ | S(O)n | 0 | NT |
| 8 | 4-fluorophenyl | 4-fluorophenyl | $CH_2$ | $CH_2$ | 0 | NT |
| 9 | 4-diethylaminophenyl | 4-diethylaminophenyl | $CH_2$ | S(O)n | 0 | NT |
| 10 | 4-dimethylaminophenyl | 4-fluorophenyl | $CH_2$ | S(O)n | 0 | NT |
| 11 | 4-pyridyl | 4-pyridyl | $CH_2$ | S(O)n | 0 | NT |
| 12 | 4-fluorophenyl | 4-pyridyl | $CH_2$ | S(O)n | 0 | NS |
| 13 | 4-cyanophenyl | 4-cyanophenyl | $CH_2$ | S(O)n | 0 | NT |
| 14 | 4-fluorophenyl | 4-methoxyphenyl | $CH_2$ | S(O)n | 0 | NT |
| 15 | 4-trifluoromethylphenyl | 4-trifluoromethylphenyl | $CH_2$ | S(O)n | 0 | NT |
| 16 | 3,4-methylenedioxyphenyl | 3,4-methylenedioxyphenyl | $CH_2$ | S(O)n | 0 | NT |
| 17 | 4-ethylaminophenyl | 4-ethylaminophenyl | $CH_2$ | S(O)n | 0 | 1.2 |
| 18 | 4-pyridyl | 4-methoxyphenyl | $CH_2$ | S(O)n | 0 | 9.9 |
| 19 | 4-fluorophenyl | 4-fluorophenyl | $CH_2$ | S(O)n | 0 | NT |
| 20 | 4-methoxyphenyl- | 4-methoxyphenyl | $CH_2CH_2$ | S(O)n | 0 | NT |
| 21 | 4-methoxyphenyl | 4-methoxyphenyl | $CH_2$ | S(O)n | 0 | NT |
| 22 | 4-methoxyphenyl | 4-methoxyphenyl | $CH_2$ | $CH_2$ | 0 | NT |
| 23 | 4-pyridyl | 4-(pyrrolidin-1-yl)phenyl | $CH_2$ | S(O)n | 0 | NT |
| 24 | 4-fluorophenyl | 4-fluorophenyl(hydrate) | $CH_2$ | S(O)n | 0 | NT |

TABLE 12. (cont'd)

| Compound Number | $R^1$ | R | A | X | n | $IC_{50}$ [(A)] [(B)] (μM) |
|---|---|---|---|---|---|---|
| 25 | 4-trifluoromethylphenyl | 4-trifluoromethylphenyl (hydrate) | $CH_2$ | S(O)n | 0 | NT |
| 26 | 2-pyridyl | 4-fluorophenyl | $CH_2$ | S(O)n | 0 | NT |
| 27 | 4-fluorophenyl | 2-pyridyl | $CH_2$ | S(O)n | 0 | NT |
| 28 | 4-pyridyl | 4-fluorophenyl | $CH_2$ | S(O)n | 1 | 0.5 |
| 29 | 4-pyridyl | 4-fluorophenyl | $CH_2$ | S(O)n | 2 | 0.5 |
| 30 | 4-fluorophenyl | 4-fluorophenyl | $CH_2$ | S(O)n | 1 | NT |
| 31 | 4-fluorophenyl | 4-fluorophenyl | $CH_2$ | S(O)n | 2 | NT |
| 32 | 4-methylthiophenyl | 4-methylthiophenyl | $CH_2$ | S(O)n | 0 | NT |
| 33 | phenyl | phenyl | $CH_2$ | S(O)n | 0 | NT |
| 34 | 4-methylphenyl | 4-methylphenyl | $CH_2$ | S(O)n | 0 | NT |
| 35 | 4-(prop-2-ene-oxy)phenyl | 4-(prop-2-ene-oxy)phenyl | $CH_2$ | S(O)n | 0 | NT |
| 36 | 4-(2,2,2-trifluoroethoxy)phenyl | 4-(2,2,2-trifluoroethyoxy) phenyl | $CH_2$ | S(O)n | 0 | NT |
| 37 | 3,4,5-trimethoxyphenyl | 3,4,5-trimethoxyphenyl | $CH_2$ | S(O)n | 0 | NT |
| 38 | 4-pyridyl | 4-acetamedophenyl | $CH_2$ | S(O)n | 0 | NT |
| 39 | 4-pyridyl | 4-fluorophenyl | $CH_2$ | $CH_2$ | 0 | 0.1-2.1 |
| 40 | 4-pyridyl | 4-methoxyphenyl | $CH_2$ | $CH_2$ | 0 | NT[(B)] |

A) NT = not tested; NS = not significant

B) $IC_{50}$ determined on $LTB_4$ production by human monocytes

C) average value vased on 4 separate tests

D) B and C are H for each compound.

## COMPOSITION EXAMPLES

### EXAMPLE A - CAPSULE COMPOSITION

A pharmaceutical composition of this invention in the form of a capsule is prepared by filling a standard two-piece hard gelatin capsule with 50 mg of a compound of Formula (IC), in powdered form, 110 mg of lactose, 32 mg of talc and 8 mg of magnesium stearate.

### EXAMPLE B - INJECTABLE PARENTERAL COMPOSITION

A pharmaceutical composition of this invention in a form suitable for administration by injection is prepared by stirring 1.5% by weight of a compound of Formula (IC) in 10% by volume propylene glycol and water. The solution is sterilized by filtration.

### EXAMPLE C - OINTMENT COMPOSITION

Compound of Formula (IC) 1.0 g
White soft paraffin to 100.0 g
The compound of Formula (IC) is dispersed in a small volume of the vehicle and gradually incorporated into the bulk of the vehicle to produce a smooth, homogeneous product. Collapsible metal tubes are then filled with the dispersion.

### EXAMPLE D - TOPICAL CREAM COMPOSITION

Compound of Formula (IC) 1.0 g
Polawax GP 200 20.0 g
Lanolin Anhydrous 2.0 g
White Beeswax 2.5 g
Methyl hydroxybenzoate 0.1 g
Distilled Water to 100.0 g

The polawax, beeswax and lanolin are heated together at 60°C. A solution of methyl hydroxybenzoate is added and homogenization is achieved using high speed stirring. The temperature is then allowed to fall to 50°C. The compound of Formula (IC) is then added and dispersed throughout, and the composition is allowed to cool with slow speed stirring.

### EXAMPLE E - TOPICAL LOTION COMPOSITION

Compound of Formula (IC) 1.0 g
Sorbitan Monolaurate 0.6 g
Polysorbate 20 0.6 g
Cetostearyl Alcohol 1.2 g
Glycerin 6.0 g
Methyl Hydroxybenzoate 0.2 g
Purified Water B.P. to 100.00 ml

The methyl hydroxybenzoate and glycerin are dissolved in 70 ml of the water at 75°. The sorbitan monolaurate, polysorbate 20 and cetostearyl alcohol are melted together at 75°C and added to the aqueous solution. The resulting emulsion is homogenized, allowed to cool with continuous stirring and the compound of Formula (IC) is added as a suspension in the remaining water. The whole suspension is stirred until homogenized.

### EXAMPLE F - EYE DROP COMPOSITION

Compound of Formula (IC) 0.5 g
Methyl Hydroxybenzoate 0.01 g
Propyl Hydroxybenzoate 0.04 g
Purified Water B.P. to 100.00 ml

The methyl and propyl hydroxybenzoates are dissolved in 70 ml purified water at 75°C and the resulting solution is allowed to cool. The compound of Formula (IC) is then added, and the solution is made

up to 100 ml with purified water. The solution is sterilized by filtration through a membrane filter (0.22 mu m pore size) and packed aseptically into suitable sterile containers.

**EXAMPLE G - COMPOSITION FOR ADMINISTRATION BY INHALATION**

For an aerosol container with a capacity of 15-20 ml: Mix 10 mg of a compound of Formula (IC) with 0.1-0.2% of a lubricating agent, such as Span 85 or oleic acid, and disperse such mixture in a propellant (c.a.), such as freon, preferably in a combination of freon 114 and freon 12, and put into an appropriate aerosol container adapted for either intranasal or oral inhalation administration.

**EXAMPLE H - COMPOSITION FOR ADMINISTRATION BY INHALATION**

For an aerosol container with a capacity of 15-20 ml: Dissolve 10 mg of a compound of Formula (IC) in ethanol (6-8 ml), add 0.1-0.2% of a lubricating agent, such as Span 85 or oleic acid; and disperse such in a propellant (c.a.), such as freon, preferably a combination of freon 114 and freon 12, and put into an appropriate aerosol container adapted for either intranasal or oral inhalation administration.

**Claims**

**Claims for the following Contracting States : BE, CH, DE, FR, GB, IT, LI, LU, NE, SE**

**1.** A compound of the Formula (IB) represented by the structure:

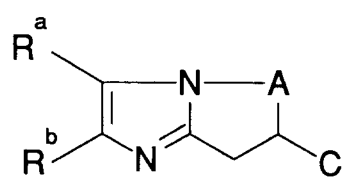

**Formula (IB)**

wherein:
A is methylene or 1,2-ethanediyl optionally substituted by methyl, ethyl or gem-dimethyl,
C is H, methyl, ethyl or gem-dimethyl; and

I. one of $R^a$ or $R^b$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, disubstituted phenyl wherein said substituents are independently selected from $C_{1-4}$alkyl or $C_{1-3}$alkoxy or the disubstituents together form a methylenedioxy group; and the other of $R^a$ or $R^b$ is selected from:

1) pyridyl;
2) phenyl;
3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);
4) disubstituted phenyl wherein said substituents are independently selected from $C_{1-4}$alkyl or $C_{1-3}$alkoxy or the disubstituents together form a methylenedioxy group; or
5) 3,4,5-trimethoxyphenyl;

provided that:

(a) when either $R^a$ or $R^b$ is 4-fluorophenyl, the other must be selected from pyridyl or phenyl substituted in the 4-position with 2,2,2-trihaloethoxy, or prop-2-en-1-oxy;
(b) $R^a$ and $R^b$ are not both phenyl substituted in the 2,3,5 or 6 position with $C_{1-3}$alkylamino; $C_{1-3}$dialkylamino; or 4-N-(azacyclo $C_{5-6}$alkyl);
(c) when either of $R^a$ or $R^b$ is disubstituted phenyl the other must be 4-pyridyl;
(d) when either of $R^a$ or $R^b$ is cyanophenyl the other must be cyanophenyl or 4-pyridyl;
(e) $R^a$ and $R^b$ are not both 3-pyridyl or 2-pyridyl, or

II. one of $R^a$ or $R^b$ is pyridyl and the other is selected from:

(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-($C_{1-3}$alkyl)-($C_{1-3}$alkanamido), N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or hydroxy, or the disubstituents together form a methylenedioxy group; or

(c) disubstituted phenyl wherein said substituents are not the same and are independently selected from halo, $C_{1-3}$alkylamino, nitro, N-($C_{1-3}$alkyl)-N-($C_{1-3}$alkanamido), $C_{1-3}$dialkylamino, amino or N-(azacyclo $C_{5-6}$alkyl); or

(d) disubstituted phenyl wherein one of said substituents must be $C_{1-3}$alkoxy, hydroxy, 2,2,2-trihaloethoxy or prop-2-en-1-oxy and the other substitutent is independently selected from halo, $C_{1-3}$alkylamino, N-($C_{1-3}$alkyl)-$C_{1-3}$alkanamido), $C_{1-3}$dialkylamino, amino, or N-(azacyclo $C_{5-6}$alkyl);

provided that:

(1) when $R^a$ is 2 or 3-pyridyl and $R^b$ is monosubstituted phenyl, the substituent is selected from other than bromo, iodo, N-($C_{1-3}$alkyl)-($C_{1-3}$alkanamido), $C_{1-3}$alkylthio, $C_{1-3}$alkylsulfinyl or $C_{1-3}$alkylsulfonyl;

(2) when $R^a$ is 2- or 3-pyridyl and $R^b$ is disubstituted phenyl, the disubstituents are both selected from other than bromo, iodo, amino, nitro, hydroxy, or ($C_{1-3}$alkyl)-($C_{1-3}$alkanamido);

(3) when $R^b$ is 2-, 3- or 4-pyridyl and $R^a$ is monosubstituted phenyl, the substituents are selected from other than bromo, iodo, N-($C_{1-3}$alkyl)-($C_{1-3}$alkanamido), $C_{1-3}$alkylthio, $C_{1-3}$alkylsulfinyl, or $C_{1-3}$alkylsulfonyl; and

(4) when $R^b$ is 2, 3 or 4-pyridyl and $R^a$ is disubstituted phenyl, the substituents are both selected from other than bromo, iodo, amino, nitro, hydroxy, N-($C_{1-3}$alkyl)-N-($C_{1-3}$alkanamido),

or a pharmaceutically acceptable salt thereof.

**2.** The compound of claim 1 wherein :

(a) $R^a$ is 4-pyridyl, $R^b$ is 4-fluorophenyl, C is H, and A is $CH_2$; or

(b) $R^b$ is 4-methoxyphenyl, $R^a$ is 4-pyridyl, C is H, and A is $CH_2$.

**3.** A compound according to Claim 1 of Formula (IC) represented by the structure:

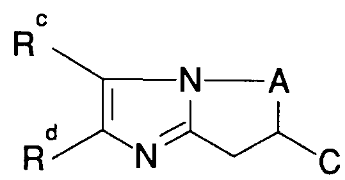

**Formula (IC)**

wherein

A is methylene or 1,2-ethanediyl optionally substituted by methyl, ethyl or gem-dimethyl, and C is H, methyl, ethyl or gem-dimethyl;

I. one of $R^c$ or $R^d$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, or disubstituted phenyl wherein said substituents are independently selected from $C_{1-3}$alkyl or $C_{1-3}$alkoxy or the disubstituents together form a methylendioxy group; and the other of $R^c$ or $R^d$ is selected from :

1) pyridyl;

2) phenyl;

3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);

4) disubstituted phenyl wherein said substituents are independently selected from $C_{1-4}$alkyl or $C_{1-3}$alkoxyor the disubstituents together form a methylenedioxy group; or

5) 3,4,5-trimethoxyphenyl;

provided that:

(a) when either $R^c$ or $R^d$ is 4-fluorophenyl, the other must be selected from pyridyl or phenyl substituted in the 4-position with 2,2,2-trihaloethoxy, or prop-2-en-1-oxy;

(b) $R^c$ and $R^d$ are both not phenyl substituted in the 2,3,5 or 6 position with $C_{1-3}$alkylamino; $C_{1-3}$dialkylamino; or 4-N-(azacyclo $C_{5-6}$alkyl);

(c) when either of $R^c$ or $R^d$ is disubstituted phenyl the other must be 4-pyridyl;

(d) when either of $R^c$ or $R^d$ is cyanophenyl the other must be cyanophenyl or 4-pyridyl;

(e) when $R^d$ is $C_{1-3}$alkanamido or N-($C_{1-3}$alkyl)-($C_{1-3}$alkanamido), $R^c$ must be 4-pyridyl;

(f) $R^c$ and $R^d$ are not both 3-pyridyl or 2-pyridyl;

or

II. when one of $R^c$ or $R^d$ is pyridyl, the other is selected from:

(a) monosubstituted phenyl wherein said substitutent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or

(c) disubstituted phenyl wherein said substituents are not the same and are independently selected from $C_{1-3}$alkylamino, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo$C_{5-6}$alkyl);

provided that:

(1) when $R^c$ is 2 or 3-pyridyl and $R^d$ is monosubstituted phenyl, the substituent is selected from other than bromo, iodo, $C_{1-3}$alkylthio, $C_{1-3}$alkylsulfinyl, or $C_{1-3}$alkylsulfonyl;

(2) when $R^c$ is 2- or 3-pyridyl and $R^d$ is disubstituted phenyl, the disubstituents are both selected from other than bromo or iodo;

(3) when $R^d$ is 2-, 3- or 4-pyridyl and $R^c$ is monosubstituted phenyl, the substituents are selected from other than bromo, iodo, $C_{1-3}$alkylthio, $C_{1-3}$alkylsulfinyl, or $C_{1-3}$alkylsulfonyl; and

(4) when $R^d$ is 2-, 3- or 4-pyridyl and $R^c$ is disubstituted phenyl, the substituents are both selected from other than bromo or iodo;

or a pharmaceutically acceptable salt thereof.

**4.** A pharmaceutical composition comprising a pharmaceutically acceptable carrier or diluent and an effective, non-toxic 5-lipoxygenase pathway inhibiting amount of a compound of the Formula (IC) as defined in Claim 3.

**5.** The composition of Claim 4 in a dosage unit form adapted for topical, parenteral, oral or inhalation administration.

**6.** A compound of the formula (IC) or a pharmaceutically acceptable salt thereof, as defined in Claim 3, for use in the treatment of rheumatoid arthritis in an animal.

**7.** The use of a compound in the preparation of a medicament for the treatment of a 5-lipoxygenase pathway mediated disease other than, or in addition to, rheumatoid arthritis, rheumatism, inflammation, or any other cyclooxygenase mediated disease state, said compound being of the formula (IA) :

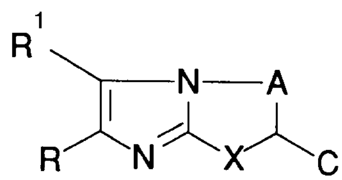

**Formula (IA)**

wherein :
A and C are as defined in Claim 1;
I. when X is $CH_2$ or $S(O)_n$ and n is 0, 1, or 2;
$R^1$ and R are independently selected from
(a) pyridyl, provided that when either or both of R and $R^1$ are pyridyl X is other then $CH_2$;
(b) phenyl;
(c) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);
(d) disubstituted phenyl wherein said substituents are independently selected from $C_{1-4}$alkyl or $C_{1-3}$alkoxyor the disubstituents together form a methylenedioxy group :
(e) 3,4,5-trimethoxyphenyl;
or a pharmaceutically acceptable salt thereof provided that :
(1) when X is $CH_2$, both of R and $R^1$ are other than phenyl substituted in the 2 or 6 position with $C_{1-3}$alkylamino; $C_{1-3}$dialkylamino or N-(azacyclo $C_{5-6}$alkyl;
(2) when either of R or $R^1$ is cyanophenyl the other must be cyanophenyl or 4-pyridyl;
(3) when X is $S(O)_n$ and R or $R^1$ is phenyl substituted in the 2,3,5 or 6 position with $C_{1-3}$alkylamino; $C_{1-3}$dialkylamino or N-(azacyclo $C_{5-6}$alkyl, the other must be 4-pyridyl; or
II. when X is $CH_2$ and one of R or $R^1$ is pyridyl, the other is selected from :
(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;
(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or
(c) disubstituted phenyl wherein said substituents are not the same and are independently selected from $C_{1-3}$alkylamino, $C_{1-3}$dialkylamino, or N-(azacyclo $C_{5-6}$alkyl), or
(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);
provided that :
(1) when $R^1$ is 2- or 3-pyridyl and R is monosubstituted phenyl, the substituent is selected from other than bromo, iodo, $C_{1-3}$alkylthio, $C_{1-3}$alkylsulfinyl, or $C_{1-3}$alkylsulfonyl;
(2) when $R^1$ is 2- or 3-pyridyl and R is disubstituted phenyl, the disubstituents are both selected from other than bromo or iodo;
(3) when R is 2-, 3-, or 4-pyridyl and $R^1$ is monosubstituted phenyl, the substituents are selected from other than bromo, iodo, $C_{1-3}$alkylthio, $C_{1-3}$alkylsulfinyl, or $C_{1-3}$alkylsulfonyl; and
(4) when R is 2-, 3- or 4-pyridyl and $R^1$ is disubstituted phenyl, the substituents are both selected from other than bromo or iodo;
or a hydrate thereof having a hydroxy group attached to the carbon atom to which R is attached wherein R is other than pyridyl, $R^1$ is 2- or 4-halo, $CF_3$ or cyano substituted phenyl and X is S;
or a pharmaceutically acceptable salt thereof.

**8.** A compound of the Formula (E) represented by the structure:

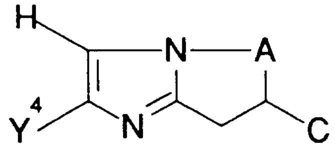

**Formula (E)**

wherein
A and C are as defined in Claim 1;
$Y^4$ is selected from:

(a) phenyl;

(b) monosubstituted phenyl, wherein said substituent is selected from halo, $C_{1-3}$alkoxy, $C_{1-3}$alkylthio, $C_{1-4}$alkyl, cyano, N-($C_{1-3}$alkyl)-($C_{1-3}$alkanamido), $C_{1-3}$alkanamido, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;

(c) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, hydroxy, or the disubstituents together form a methylenedioxy group;

(d) disubstituted phenyl wherein said substituents are not the same and are independently selected from halo, $C_{1-3}$alkylamino, $C_{1-3}$dialkylamino, amino, N-(azacyclo $C_{5-6}$alkyl), nitro, or N-($C_{1-3}$alkyl)-$C_{1-3}$alkanamido), or

(e) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that when A is $CH_2CH_2$, C is H, and $Y^4$ is other than 2,4-dimethoxyphenyl or 4-aminophenyl; or a pharmaceutically acceptable salt thereof.

**9.** A compound of the Formula (F) represented by the structure :

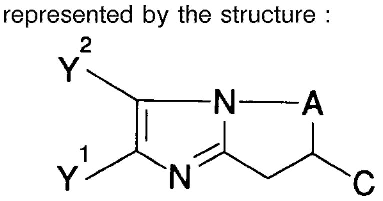

**Formula (F)**

wherein:

A and C are as defined in Claim 1; and

$Y^2$ is 4-(1,4-dihydro)pyridyl substituted with N-($C_{1-8}$ alkanoyl), N-($C_{1-8}$alkoxycarbonyl), N-(benzoyl), N-(phenoxycarbonyl), N-(phenylacetyl) or N-(benzyloxycarbonyl);

$Y^1$ is selected from

(a) phenyl or monosubstituted phenyl wherein said substituent is selected from halo, $C_{1-3}$alkoxy, $C_{1-3}$alkylthio, $C_{1-4}$alkyl, $CF_3$, N-($C_{1-3}$alkyl)-$C_{1-3}$alkanamido, $C_{1-3}$dialkylamino, N-(azacyclo $C_{5-6}$alkyl), prop-2-en-1-oxy, 2,2,2-trihaloethoxy, $C_{1-3}$alkanamido or $C_{1-3}$dialkylamino;

(b) disubstituted phenyl wherein said substituents are the same and are selected from halo, $C_{1-3}$alkoxy, $C_{1-3}$dialkylamino, N-(azacyclo $C_{5-6}$alkyl), $C_{1-3}$alkylamino, 2,2,2-trihaloethoxy, or prop-2-en-1-oxy, hydroxy or the disubstituents together form a methylenedioxy group;

(c) disubstituted phenyl wherein said substituents are not the same and are independently selected from halo, nitro, $C_{1-3}$dialkylamino or N-($C_{1-3}$alkyl)-($C_{1-3}$alkanamido), N-(azacyclo $C_{5-6}$alkyl); or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

or a pharmaceutically acceptable salt thereof.

**10.** A compound of the Formula (G) represented by the structure :

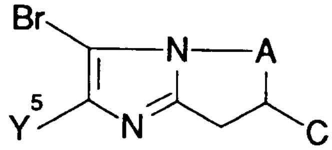

**Formula (G)**

wherein:
A and C are as defined in Claim 1;
$Y^5$ is selected from
(a) phenyl or monosubstituted phenyl wherein said substituent is selected from fluoro, chloro, $C_{1-3}$alkoxy, $C_{1-4}$alkyl, $C_{1-3}$dialkylamino, $CF_3$, $C_{1-3}$alkylamino, N-(azacyclo $C_{5-6}$alkyl), prop-2-en-1-oxy or 2,2,2-trihaloethoxy;
(b) disubstituted phenyl wherein said substituents are the same and are selected from fluoro, chloro, $C_{1-3}$alkoxy, $C_{1-3}$dialkylamino, N-(azacyclo $C_{5-6}$alkyl), 2,2,2-trihaloethoxy, or prop-2-en-1-oxy, or the disubstituents together form a methylenedioxy group; or
(c) disubstituted phenyl wherein said substituents are not the same and are independently selected from fluoro, chloro, $C_{1-3}$alkylamino, $C_{1-3}$dialkylamino, or N-(azacyclo $C_{5-6}$alkyl); or
(d) disubstituted phenyl wherein one of said substituents must be $C_{1-3}$alkoxy, 2,2,2-trihaloethoxy or prop-2-en-1-oxy and the other substituent is independently selected from fluoro, chloro, $C_{1-3}$alkylamino, $C_{1-3}$dialkylamino, or N-(azacyclo $C_{5-6}$alkyl);
or a pharmaceutically acceptable salt thereof.

**11.** A compound of the Formula (H) represented by the structure :

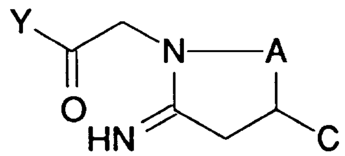

**Formula (H)**

wherein :
A and C are as defined in Claim 1;
Y is selected from :
(a) phenyl or monosubstituted phenyl, wherein said substituent is selected from halo, $C_{1-3}$alkoxy, $C_{1-3}$alkylthio, $C_{1-4}$alkyl, $C_{1-3}$alkanamido, $C_{1-3}$dialkylamino, $CF_3$, $C_{1-3}$alkylamino, N-(azacyclo $C_{5-6}$alkyl) or N-($C_{1-3}$alkyl)-($C_{1-3}$alkanamido);
(b) disubstituted phenyl wherein said substituents are the same and are selected from halo, $C_{1-3}$dialkylamino, N-(azacyclo $C_{5-6}$alkyl), or $C_{1-3}$alkoxy, or the disubstituents together form a methylenedioxy group;
(c) disubstituted phenyl wherein said substituents are not the same and are independently selected from halo, nitro, $C_{1-3}$alkanamido, $C_{1-3}$dialkylamino, or N-(azacyclo $C_{5-6}$alkyl), or
(d) disubstituted phenyl wherein one of said substituents must be $C_{1-3}$alkoxy, hydroxy, 2,2,2-trihaloethoxy or prop-2-en-1-oxy and the other substituent is independently selected from halo, N-($C_{1-3}$alkyl)-($C_{1-3}$alkanamido), $C_{1-3}$dialkylamino, or N-(azacyclo $C_{5-6}$alkyl);
provided that when A is $CH_2CH_2$ and C is H, Y is other than 2,4-dimethoxyphenyl or 4-aminophenyl;
or a pharmaceutically acceptable salt thereof.

**12.** A process for the preparation of a compound of Formula (IB) of Claim 1 which process comprises :
(i) reacting a compound of Formula (G) of Claim 11 with a $C_{1-5}$alkyllithium reagent to yield the corresponding lithium reagent by lithium-halogen exchange;
(ii) adding excess magnesium halide etherate to the lithium reagent to yield the corresponding Grignard reagent by transmetalation;
(iii) adding the Grignard reagent to an N-acylpyridium salt to yield the corresponding compound of Formula (F);
(iv) deacylating/oxidizing the compound of the Formula (F) to yield the corresponding compound of Formula (IB).

**13.** A compound according to Claim 1 selected from the group comprising :
the compound wherein $R^a$ is 4-pyridyl, $R^b$ is 4-fluorophenyl, A is $CH_2$, and C is H; and the compound wherein $R^a$ is 4-pyridyl, $R^b$ is 4-methoxyphenyl, A is $CH_2$, and C is H, and pharmaceutically acceptable salts thereof.

**14.** The use of a compound of the formula (1A) as defined in claim 7 in the preparation of a medicament for the treatment of asthma or inflammatory bowel disease.

**Claims for the following Contracting States : AT, GR, ES**

**1.** A process for the preparation of a compound of the Formula (IB) :

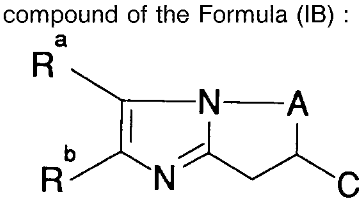

**Formula (IB)**

wherein:
A is methylene or 1,2-ethanediyl optionally substituted by methyl, ethyl or gem-dimethyl;
C is H, methyl, ethyl or gem-dimethyl; and

I. one of $R^a$ or $R^b$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, or disubstituted phenyl wherein said substituents are independently selected from $C_{1-4}$alkyl or $C_{1-3}$alkoxy or the disubstituents together form a methylenedioxy group; and the other of $R^a$ or $R^b$ is selected from:

1) pyridyl;
2) phenyl;
3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);
4) disubstituted phenyl wherein said substituents are independently selected from $C_{1-4}$alkyl or $C_{1-3}$alkoxyor the disubstituents together form a methylenedioxy group; or
5) 3,4,5-trimethoxyphenyl;

provided that:

(a) when either $R^a$ or $R^b$ is 4-fluorophenyl, the other must be selected from pyridyl or phenyl substituted in the 4-position with 2,2,2-trihaloethoxy, or prop-2-en-1-oxy;
(b) $R^a$ and $R^b$ are not both phenyl substituted in the 2,3,5 or 6 position with $C_{1-3}$alkylamino; $C_{1-3}$dialkylamino; or 4-N-(azacyclo $C_{5-6}$alkyl);
(c) when either of $R^a$ or $R^b$ is disubstituted phenyl the other must be 4-pyridyl;
(d) when either of $R^a$ or $R^b$ is cyanophenyl the other must be cyanophenyl or 4-pyridyl;
(e) $R^a$ and $R^b$ are not both 3-pyridyl or 2-pyridyl,

or

II. one of $R^a$ or $R^b$ is pyridyl and the other is selected from:

(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-($C_{1-3}$alkyl)-($C_{1-3}$alkanamido),N-(azacyclo $C_{5-6}$alkyl), prop-2-en-1-oxy or 2,2,2-trihaloethoxy;
(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or hydroxy, or the disubstituents together form a methylenedioxy group;
(c) disubstituted phenyl wherein said substituents are not the same and are independently selected from halo, $C_{1-3}$alkylamino, nitro, N-($C_{1-3}$alkyl)-($C_{1-3}$alkanamido), $C_{1-3}$dialkylamino, amino or N-(azacyclo $C_{5-6}$alkyl); or
(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that:

(1) when $R^a$ is 2- or 3-pyridyl and $R^b$ is monosubstituted phenyl, the substituent is selected from other than bromo, iodo, N-($C_{1-3}$alkyl)-($C_{1-3}$alkanamido), $C_{1-3}$alkylthio, $C_{1-3}$alkylsulfinyl or $C_{1-3}$alkylsulfonyl;

(2) when $R^a$ is 2- or 3-pyridyl and $R^b$ is disubstituted phenyl, the disubstituents are both selected from other than bromo, iodo, amino, nitro, hydroxy, or N-($C_{1-3}$alkyl)-($C_{1-3}$alkanamido);

(3) when $R^b$ is 2-, 3- or 4-pyridyl and $R^a$ is monosubstituted phenyl, the substituents are selected from other than bromo, iodo, N-($C_{1-3}$alkyl)-($C_{1-3}$alkanamido), $C_{1-3}$alkylthio, $C_{1-3}$alkylsulfinyl, or $C_{1-3}$alkylsulfonyl; and

(4) when $R^b$ is 2-, 3- or 4-pyridyl and $R^a$ is disubstituted phenyl, the substituents are both selected from other than bromo, iodo, amino, nitro, hydroxy, N-($C_{1-3}$alkyl)-($C_{1-3}$alkanamido),

or a pharmaceutically acceptable salt thereof,

A. when it is required to prepare a compound of Formula (IB) wherein $R^a$ is 4-pyridyl and $R^b$ is other than pyridyl, which process comprises reacting a compound of the Formula (E) represented by the structure:

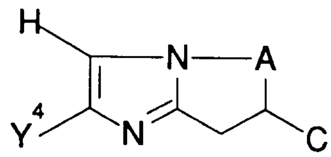

**Formula (E)**

wherein

A and C are as hereinbefore defined, and

$Y^4$ is selected from:

(a) phenyl;

(b) monosubstituted phenyl, wherein said substituent is selected from halo, $C_{1-3}$alkoxy, $C_{1-3}$alkylthio, $C_{1-4}$alkyl, cyano, N-($C_{1-3}$alkyl)-($C_{1-3}$alkanamido), $C_{1-3}$alkanamido, $C_{1-3}$alkylamino, $C_{1-3}$dialkylamino, $CF_3$, N-(azacyclo $C_{5-6}$alkyl), prop2-en-1-oxy or 2,2,2-trihaloethoxy;

(c) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, hydroxy, or the disubstituents together form a methylenedioxy group;

(d) disubstituted phenyl wherein said substituents are not the same and are independently selected from halo, $C_{1-3}$alkylamino, $C_{1-3}$dialkylamino, amino, N-(azacyclo $C_{5-6}$alkyl), nitro, or N-($C_{1-3}$alkyl)-($C_{1-3}$alkanamido), or

(e) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that when A is $CH_2CH_2$, and C is H, $Y^4$ is other than 2,4-dimethoxyphenyl or 4-aminophenyl;

or a pharmaceutically acceptable salt thereof,

with pyridine and an acyl halide or haloacyl ester to yield a compound of formula (F) represented by the structure :

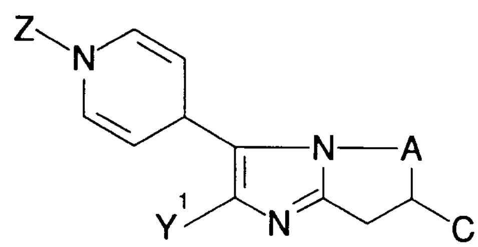

**Formula (F)**

wherein:
A and C are as hereinbefore defined; and
$Y^1$ is selected from

(a) phenyl or monosubstituted phenyl wherein said substituent is selected from halo, $C_{1-3}$alkoxy, $C_{1-3}$alkylthio, $C_{1-4}$alkyl, N-($C_{1-3}$alkyl)-$C_{1-3}$alkanamido, $C_{1-3}$dialkylamino, $CF_3$, N-(azacyclo $C_{5-6}$alkyl), $C_{1-3}$alkanamido, $C_{1-3}$alkylamino, prop-2-en-1-oxy or 2,2,2-trihaloethoxy;

(b) disubstituted phenyl wherein said substituents are the same and are selected from halo, $C_{1-3}$alkoxy, $C_{1-3}$dialkylamino,$C_{1-3}$alkylamino, hydroxy, N-(azacyclo $C_{5-6}$alkyl), 2,2,2-trihaloethoxy, or prop2-en-1-oxy, or the disubstituents together form a methylenedioxy group;

(c) disubstituted phenyl wherein said substituents are not the same and are independently selected from halo, nitro, $C_{1-3}$dialkylamino or N-($C_{1-3}$alkyl)-($C_{1-3}$alkanamido), N-(azacyclo $C_{5-6}$alkyl); or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

and Z is $C_{1-8}$alkanoyl, $C_{1-8}$alkoxycarbonyl, benzoyl, phenoxycarbonyl, phenylacetyl or benzyloxycarbonyl;
followed by deacylation/oxidation of a compound of Formula (F) to yield a compound of Formula (IB); and
B. when it is required to prepare a hydroxy compound of Formulae (IB), which process comprises demethylating a methoxyphenyl compound of Formula (I); Formula I being represented by the structure :

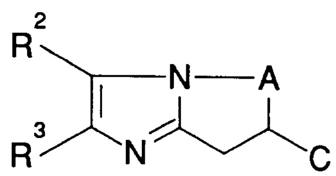

**Formula (I)**

wherein
A and C are as hereinbefore defined;
$R^2$ or $R^3$ are independently selected from:

a) pyridyl;

b) phenyl or monosubstituted phenyl wherein said substituent is selected from $C_{1-3}$dialkylamino, $C_{1-3}$alkylamino, N-(azacyclo $C_{5-6}$alkyl, cyano, 2,2,2-trihaloethoxy, prop-2-en-1-oxy, N-($C_{1-3}$alkyl)-($C_{1-3}$alkanamido), $C_{1-3}$alkanamido, amino, hydroxy, $C_{1-3}$alkylthio, $C_{1-4}$alkyl, halo, $CF_3$, $C_{1-3}$alkoxy, $C_{1-3}$alkylsulfinyl,or $C_{1-3}$alkylsulfonyl,

c) disubstituted phenyl wherein said substituents are independently selected from $C_{1-4}$alkyl $C_{1-3}$alkoxy, hydroxy, nitro, amino, halo,$C_{1-3}$alkylamino, $C_{1-3}$dialkylamino, N-(azacyclo $C_{5-6}$alkyl), 2,2,2-trihaloethoxy, prop-2-en-1-oxy, or N-($C_{1-3}$alkyl)-($C_{1-3}$alkanamido), or the disubstituents together form a methylenedioxy group; or

d) 3,4,5-trimethoxyphenyl;

provided that:

(1) when $R^3$ is cyanophenyl, $R^2$ must be either cyanophenyl or 4-pyridyl;

or a pharmaceutically acceptable salt thereof;
with HBr in acetic acid or $BBr_3$ in methylene chloride to give the corresponding hydroxy compound of Formulae (IB), and
C. which process comprises an 0-alkylation of the hydroxy compound of Formula (I), as defined above when A is $CH_2$ and at least one of $R^2$ and $R^3$ is a hydroxyphenyl group, or when A is $CH_2CH_2$ and only one of $R^2$ and $R^3$ is a hydroxyphenyl group to give the corresponding 2,2,2-trihaloethoxyphenyl or prop2-en-1-oxyphenyl substituted compound of Formula (IB); and
D. which process comprises the acylation of an aminophenyl or ($C_{1-3}$alkylamino)phenyl compound of Formulae (IB), or (I);

wherein Formula (IB) and Formula (I) are each as previously defined, and A is $CH_2CH_2$ and $R^2$ and $R^3$ of Formula (I) are not pyridyl, or when A is $CH_2$ and one of $R^2$ or $R^3$ are pyridyl, with the correspondingly substituted acyl halide or anhydride in pyridine to give the corresponding ($C_{1-3}$alkanamido)phenyl or the corresponding N-($C_{1-3}$alkyl)-($C_{1-3}$alkanamido)phenyl compound of Formulae (IB); and

E. which process comprises the alkylation of an ($C_{1-3}$alkanamido)phenyl compound of Formulae (I) as previously defined with an alkylating agent in the presence of a base to give the corresponding N-($C_{1-3}$alkyl)-($C_{1-3}$alkanamido)phenyl compound of Formulae (IB); and

F. which process comprises the hydrolysis of an ($C_{1-3}$alkanamido)phenyl or N-($C_{1-3}$alkyl)-($C_{1-3}$alkanamido)phenyl compound or Formulae (I), as previously defined to give the corresponding aminophenyl or ($C_{1-3}$alkylamino)phenyl compound of Formulae (IB); and

G. which process comprises the oxidation of a compound of Formula (IB) wherein one or both of $R^a$ and $R^b$ is $C_{1-3}$alkylthio, with metachloroperbenzoic acid to give the corresponding $C_{1-3}$alkylsulphinyl, $C_{1-3}$alkylsulphonyl compound of Formula (IB); and

H. which process comprises the reduction of a compound of Formula (I) as previously defined, wherein one or both of $R^2$ and $R^3$ of Formula (I), is nitrophenyl with hydrogen gas on a paladium catalyst to give the corresponding aminophenyl compound of Formula (IB); and

I. which process comprises the cycloalkylation of a compound of Formulae (I) as previously defined wherein one or both of $R^2$ and $R^3$ is aminophenyl with dihalobutane or dihalopentane as appropriate in the presence of a base to give the corresponding N-(azacyclo$C_{5-6}$alkyl)phenyl compound of Formula (IB); and

J. when it is required to prepare compounds of Formulae (IB) as previously defined, wherein $R^a$ and $R^b$ are each ($C_{1-3}$alkylamino)phenyl, which process comprises reducing the corresponding $C_{1-3}$alkanamido compounds with borane or borane dimethylsulfide complex in tetrahydrofuran to yield the desired compounds of Formula (IB); and

K. when it is required to prepare compounds of Formula (IB) as previously defined wherein $R^a$ and $R^b$ are ($C_{1-3}$dialkylamino)phenyl, which process comprises reducing the corresponding N-($C_{1-3}$alkyl)-($C_{1-3}$alkanamido) compounds with borane or borane dimethylsulfide complex in tetrahydrofuran to yield the desired compounds of Formula(IB); and

L. which process comprises :

(i) reacting a compound of Formula (G) :

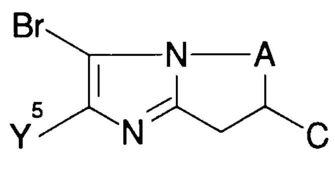

**Formula (G)**

wherein :

A and C are as hereinbefore defined;

$Y^5$ is selected from

(a) phenyl or monosubstituted phenyl wherein said substituent is selected from fluoro, chloro, $C_{1-3}$alkoxy, $C_{1-4}$alkyl, $C_{1-3}$dialkylamino, $CF_3$, $C_{1-3}$alkylamino, N-(azacyclo $C_{5-6}$alkyl), prop-2-en-1-oxy or 2,2,2-trihaloethoxy;

(b) disubstituted phenyl wherein said substituents are the same and are selected from fluoro, chloro, $C_{1-3}$alkoxy, $C_{1-3}$dialkylamino, N-(azacyclo $C_{5-6}$alkyl), 2,2,2-trihaloethoxy, or prop-2-en-1-oxy, or the disubstituents together form a methylenedioxy group;

(c) disubstituted phenyl wherein said substituents are not the same and are independently selected from fluoro, chloro, $C_{1-3}$alkylamino, $C_{1-3}$dialkylamino, or N-(azacyclo $C_{5-6}$alkyl); or

(d) disubstituted phenyl wherein one of said substituents must be $C_{1-3}$alkoxy, 2,2,2-trihaloethoxy or prop-2-en-1-oxy and the other substituent is independently selected from fluoro, chloro, $C_{1-3}$alkylamino, $C_{1-3}$dialkylamino, N-(azacyclo $C_{5-6}$alkyl); or a pharmaceutically acceptable salt thereof,

with a $C_{1-5}$alkyllithium reagent to yield the corresponding lithium reagent by lithium-halogen exchange;

(ii) adding excess magnesium halide etherate to the lithium reagent to yield the corresponding Grignard reagent by transmetalation;

(iii) adding the Grignard reagent to an N-acylpyridium salt to yield the corresponding compound of Formula (F);

(iv) deacylating/oxidizing the compound of Formula (F) to yield the corresponding compound of Formula (IB);

and thereafter optionally converting one compound of the Formula (IB) into another compound of the Formula (IB).

**2.** A process for the preparation of a compound of the Formula (E) as previously defined in Claim 1;

A. which process comprises refluxing in water or an aqueous solvent a compound of Formula (H) represented by the structure :

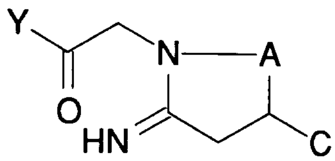

**Formula (H)**

wherein :

A and C are as defined in Claim 1;

Y is selected from :

(a) phenyl or monosubstituted phenyl, wherein said substituent is selected from halo, $C_{1-3}$alkoxy, $C_{1-3}$alkylthio, $C_{1-4}$alkyl, $C_{1-3}$alkylamino, $C_{1-3}$alkanamido, $C_{1-3}$dialkylamino, $CF_3$, N-(azacyclo $C_{5-6}$alkyl) or N-($C_{1-3}$alkyl)-($C_{1-3}$alkanamido);

(b) disubstituted phenyl wherein said substituents are the same and are selected from halo, $C_{1-3}$dialkylamino, N-(azacyclo $C_{5-6}$alkyl), or $C_{1-3}$alkoxy, or the disubstituents together form a methylenedioxy group;

(c) disubstituted phenyl wherein said substituents are not the same and are independently selected from halo, nitro, $C_{1-3}$alkanamido, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, N-($C_{1-3}$alkyl)-($C_{1-3}$alkanamido), $C_{1-3}$dialkylamino, or N-(azacyclo $C_{5-6}$alkyl);

provided that when A is $CH_2CH_2$ and C is H, Y is other than 2,4-dimethoxyphenyl or 4-aminophenyl; or a pharmaceutically acceptable salt thereof; and

B. which process comprises the alkylation of an ($C_{1-3}$alkanamido)phenyl compound of Formulae (E) as previously defined with an alkylating agent in the presence of a base to give the corresponding N-($C_{1-3}$alkyl)-($C_{1-3}$alkanamido)phenyl compound of Formulae (E); and

C. which process comprises the hydrolysis of an ($C_{1-3}$alkanamido)phenyl or N-($C_{1-3}$alkyl)-($C_{1-3}$alkanamido)phenyl compound or Formulae (E), as previously defined to give the corresponding aminophenyl or ($C_{1-3}$alkylamino)phenyl compound of Formulae (E), and

D. which process comprises the reduction of a compound of Formula (E) as previously defined, wherein $Y^4$ of Formula (E), is nitrophenyl with hydrogen gas on a palladium catalyst to give the corresponding aminophenyl compound of Formula (E); and

E. which process comprises the cycloalkylation of a compound of Formulae (E) as previously defined wherein $Y^4$ is aminophenyl with dihalobutane or dihalopentane as appropriate in the presence of a base to give the corresponding N-(azacyclo $C_{5-6}$alkyl)phenyl compound of Formula (E); and

F. which process comprises O-alkylation of the hydroxy compound of Formula (E) wherein A is $CH_2$ and $Y^4$ is a hydroxyphenyl group or A is $CH_2CH_2$ and $Y^4$ is a hydroxyphenyl group to give the corresponding 2,2,2-trihaloethoxyphenyl or prop-2-en-1-oxyphenyl substituted compound of Formula (E).

**3.** A process for the preparation of a compound of Formula (H) as defined in Claim 2 comprising reacting a compound of Formula (D)

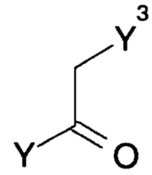

**Formula (D)**

wherein Y is as defined in Formula (H) and $Y^3$ is halo, with a compound of Formula (C)

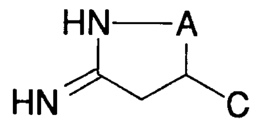

**Formula (C)**

wherein A and C are as defined in Formula (H).

**4.** A process for the preparation of a compound of Formula (G) as previously defined in Claim 1 :
A. which process comprises reacting a compound of Formula (E) as previously defined in Claim 1 with bromine; and
B. which process comprises the hydrolysis of an ($C_{1-3}$alkanamido)phenyl or N-($C_{1-3}$alkyl)-($C_{1-3}$alkanamido)phenyl compound of Formula (G) as previously defined to give the corresponding aminophenyl or ($C_{1-3}$alkylamino)phenyl compound of Formula (G).

**5.** A process for the preparation of a compound of Formula (F) as defined in Claim 1 which comprises reacting a compound of the formula (E) as defined in Claim 1 with pyridine and an acyl halide or haloacyl ester.

**6.** A process according to Claim 1 for preparing a compound of the Formula (IB) wherein :
(a) $R^a$ is 4-pyridyl, $R^b$ is 4-fluorophenyl, A is $CH_2$ and C is H; and
(b) $R^b$ is 4-methoxyphenyl, $R^a$ is 4-pyridyl, A is $CH_2$, and C is H.

**7.** A process according to Claim 1 for preparing a compound of the Formula (IC) :

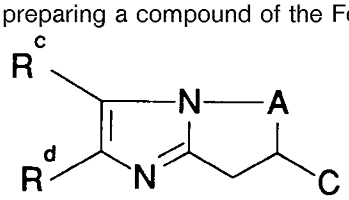

**Formula (IC)**

wherein
A and C are as defined in Claim 1;
one of $R^c$ or $R^d$ must be selected from 2-pyridyl, 3-pyridyl, 4-pyridyl, monosubstituted phenyl wherein said substituent is selected from $C_{1-3}$dialkylamino, $C_{1-3}$alkylamino, N-(azacyclo $C_{5-6}$alkyl), cyano, 2,2,2-trihaloethoxy, $C_{1-3}$alkanamido, N-($C_{1-3}$alkyl)-($C_{1-3}$alkanamido), or prop-2-en-1-oxy, or disubstituted phenyl wherein said substituents are independently selected from $C_{1-4}$alkyl or $C_{1-3}$alkoxy or the disubstituents together form a methylenedioxy group; and the other of $R^c$ or $R^d$ is selected from:

1) pyridyl;
2) phenyl;
3) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);
4) disubstituted phenyl wherein said substituents are independently selected from $C_{1-4}$alkyl or $C_{1-3}$alkoxyor the disubstituents together form a methylenedioxy group; or
5) 3,4,5-trimethoxyphenyl;

provided that:

(a) when either $R^c$ or $R^d$ is 4-fluorophenyl, the other must be selected from pyridyl or phenyl substituted in the 4-position with 2,2,2-trihaloethoxy, or prop-2-en-1-oxy;
(b) $R^c$ and $R^d$ are not both phenyl substituted in the 2,3,5 or 6 position with $C_{1-3}$alkylamino; $C_{1-3}$dialkylamino; or 4-N-(azacyclo $C_{5-6}$alkyl);
(c) when either of $R^c$ or $R^d$ is disubstituted phenyl the other must be 4-pyridyl;
(d) when either of $R^c$ or $R^d$ is cyanophenyl the other must be cyanophenyl or 4-pyridyl;
(e) $R^d$ is $C_{1-3}$alkanamido or N-($C_{1-3}$alkyl)-($C_{1-3}$alkanamido), $R^c$ must be 4-pyridyl;
(f) $R^c$ and $R^d$ are not both 3-pyridyl or 2-pyridyl; or

when one of $R^c$ or $R^d$ is pyridyl, the other is selected from:

(a) monosubstituted phenyl wherein said substituent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;
(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or
(c) disubstituted phenyl wherein said substituents are not the same and are independently selected from $C_{1-3}$alkylamino, $C_{1-3}$dialkylamino, N-(azacyclo $C_{5-6}$alkyl); or
(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-$C_{1-3}$alkanamido), $C_{1-3}$dialkylamino, amino, or N-(azacyclo $C_{5-6}$alkyl);

provided that:

(1) when $R^c$ is 2 or 3-pyridyl and $R^d$ is monosubstituted phenyl, the substituent is selected from other than bromo, iodo, $C_{1-3}$alkylthio, $C_{1-3}$alkylsulfinyl or $C_{1-3}$alkylsulfonyl;
(2) when $R^c$ is 2- or 3-pyridyl and $R^d$ is disubstituted phenyl, the disubstituents are both selected from other than bromo or iodo;
(3) when $R^d$ is 2-, 3- or 4-pyridyl and $R^c$ is monosubstituted phenyl, the substituents are selected from other than bromo, iodo, $C_{1-3}$alkylthio, $C_{1-3}$alkylsulfinyl, or $C_{1-3}$alkylsulfonyl; and
(4) when $R^d$ is 2-, 3- or 4-pyridyl and $R^c$ is disubstituted phenyl, the substituents are both selected from other than bromo or iodo.

**8.** A process according to Claim 1 for preparing a compound selected from the group comprising the compound wherein $R^a$ is 4-pyridyl, $R^b$ is 4-fluorophenyl, A is $CH_2$ and C is H; and the compound wherein $R^a$ is 4-pyridyl, $R^b$ is 4-methoxyphenyl, A is $CH_2$ and C is H; and pharmaceutically acceptable salts thereof.

**9.** A process for preparing a pharmaceutical composition containing a compound of the Formula (IC) as defined in Claim 7 which process comprises bringing the compound into association with a pharmaceutically acceptable carrier.

**10.** The use of a compound in the preparation of a medicament for the treatment of a 5-lipoxygenase pathway mediated disease other than, or in addition to, rheumatoid arthritis, rheumatism, inflammation, or any other cyclooxygenase mediated disease state, said compound being of the formula (IA) :

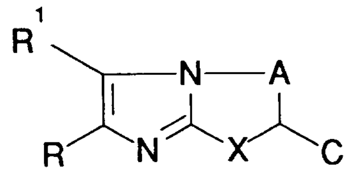

**Formula (IA)**

wherein :

A and C are as defined in Claim 1;

I. when X is $CH_2$ or $S(O)_n$ and n is 0, 1, or 2;

$R^1$ and R are independently selected from

(a) pyridyl, provided that when either or both of R and $R^1$ are pyridyl X is other then $CH_2$;

(b) phenyl;

(c) monosubstituted phenyl wherein said substituent is selected from $C_{1-3}$alkoxy, halo, $CF_3$, $C_{1-3}$alkylthio, $C_{1-4}$alkyl, 2,2,2-trihaloethoxy, prop-2-en-1-oxy, $C_{1-3}$alkylamino, $C_{1-3}$dialkylamino, cyano or N-(azacyclo $C_{5-6}$alkyl);

(d) disubstituted phenyl wherein said substituents are independently selected from $C_{1-4}$alkyl or $C_{1-3}$alkoxyor the disubstituents together form a methylenedioxy group :

(e) 3,4,5-trimethoxyphenyl;

or a pharmaceutically acceptable salt thereof provided that :

(1) when X is $CH_2$, both of R and $R^1$ are other than phenyl substituted in the 2 or 6 position with $C_{1-3}$alkylamino; $C_{1-3}$dialkylamino or N-(azacyclo $C_{5-6}$alkyl;

(2) when either of R or $R^1$ is cyanophenyl the other must be cyanophenyl or 4-pyridyl;

(3) when X is $S(O)_n$ and R or $R^1$ is phenyl substituted in the 2,3,5 or 6 position with $C_{1-3}$alkylamino; $C_{1-3}$dialkylamino or N-(azacyclo $C_{5-6}$alkyl, the other must be 4-pyridyl; or

II. when X is $CH_2$ and one of R or $R^1$ is pyridyl, the other is selected from :

(a) monosubstituted phenyl wherein said substitutent is selected from halo, $C_{1-3}$alkoxy, $C_{1-3}$alkylthio, $C_{1-4}$alkyl, $C_{1-3}$alkylsulfinyl, $C_{1-3}$alkylsulfonyl, $C_{1-3}$alkylamino, $C_{1-3}$dialkylamino, $CF_3$, N-(azacyclo $C_{5-6}$alkyl), prop-2-en-1-oxy or 2,2,2-trihaloethoxy;

(b) disubstituted phenyl wherein said substituents are the same and are selected from halo, $C_{1-3}$alkoxy, $C_{1-3}$alkylamino, $C_{1-3}$dialkylamino, N-(azacyclo $C_{5-6}$alkyl), 2,2,2-trihaloethoxy, prop-2-en-1-oxy, or the disubstituents together form a methylenedioxy group; or

(c) disubstituted phenyl wherein said substituents are not the same and are independently selected from $C_{1-3}$alkylamino, $C_{1-3}$dialkylamino, or N-(azacyclo $C_{5-6}$alkyl), or

(d) disubstituted phenyl wherein one of said substituents must be $C_{1-3}$alkoxy, hydroxy, 2,2,2-trihaloethoxy or prop-2-en-1-oxy and the other substituent is independently selected from halo, $C_{1-3}$alkylamino, N-($C_{1-3}$alkyl)-($C_{1-3}$alkanamido), $C_{1-3}$dialkylamino, amino, or N-(azacyclo $C_{5-6}$alkyl);

provided that :

(1) when $R^1$ is 2- or 3-pyridyl and R is monosubstituted phenyl, the substituent is selected from other than bromo, iodo, $C_{1-3}$alkylthio, $C_{1-3}$alkylsulfinyl, or $C_{1-3}$alkylsulfonyl;

(2) when $R^1$ is 2- or 3-pyridyl and R is disubstituted phenyl, the disubstituents are both selected from other than bromo or iodo;

(3) when R is 2-, 3-, or 4-pyridyl and $R^1$ is monosubstituted phenyl, the substituents are selected from other than bromo, iodo, $C_{1-3}$alkylthio, $C_{1-3}$alkylsulfinyl, or $C_{1-3}$alkylsulfonyl; and

(4) when R is 2-, 3- or 4-pyridyl and $R^1$ is disubstituted phenyl, the substituents are both selected from other than bromo or iodo;

or a hydrate thereof having a hydroxy group attached to the carbon atom to which R is attached wherein R is other than pyridyl, $R^1$ is 2- or 4-halo, $CF_3$ or cyano substituted phenyl and X is S;

or a pharmaceutically acceptable salt thereof.

**11.** The use of a compound of the formula (1A) as defined in claim 10 in the preparation of a medicament for the treatment of asthma or inflammatory bowel disease.

**Patentansprüche**
**Patentansprüche für folgende Vertragsstaaten : BE, CH, DE, FR, GB, IT, LI, LU, NE, SE**

1. Verbindung der Formel (IB), beschrieben durch die Struktur

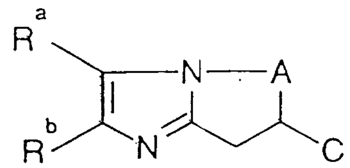

(IB)

wobei:
A eine Methylen- oder 1,2-Ethandiylgruppe, gegebenenfalls mit einer Methyl-, Ethyl- oder gem-Dimethylgruppe substituiert, darstellt;
C ein H-Atom, eine Methylgruppe, eine Ethylgruppe oder eine gem-Dimethylgruppe darstellt; und
I. einer der Reste $R^a$ oder $R^b$ gewählt werden muß aus: einer 2-Pyridyl-, 3-Pyridyl-, 4-Pyridylgruppe, einem monosubstituierten Phenylrest, wobei der Substituent aus einem $C_{1-3}$-Dialkylamino-, $C_{1-3}$-Alkylamino-, N-(Azacyclo-$C_{5-6}$-alkyl)-Rest, einer Cyanogruppe, einem 2,2,2-Trihalogenethoxy-, $C_{1-3}$-Alkanamido-, N-($C_{1-3}$-Alkyl)-($C_{1-3}$-alkanamido)-Rest oder einer Prop-2-en-1-oxy-Gruppe gewählt wird, einem disubstituierten Phenylrest, wobei die Substituenten unabhängig voneinander aus einem $C_{1-4}$-Alkyl- oder $C_{1-3}$-Alkoxyrest gewählt werden, oder die Disubstituenten zusammen eine Methylendioxygruppe bilden; und der andere der Reste $R^a$ oder $R^b$ gewählt wird aus:
1) einer Pyridylgruppe;
2) einer Phenylgruppe;
3) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;
4) einem disubstituierten Phenylrest, wobei die Substituenten unabhängig aus einem $C_{1-4}$-Alkyl- oder $C_{1-3}$-Alkoxyrest gewählt werden oder die Disubstituenten zusammen eine Methylendioxygruppe bilden; oder
5) einer 3,4,5-Trimethoxyphenylgruppe;
mit der Maßgabe, daß:
(a) wenn einer der Reste $R^a$ oder $R^b$ eine 4-Fluorphenylgruppe darstellt, der andere aus einer Pyridylgrup-pe oder einem Phenylrest, der in 4-Stellung mit einem 2,2,2-Trihalogenethoxyrest oder einer Prop-2-en-1-oxy-Gruppe substituiert ist, gewählt werden muß;
(b) $R^a$ und $R^b$ nicht beide einen Phenylrest, der in 2-, 3-, 5- oder 6-Stellung mit einem $C_{1-3}$-Alkylamino-, $C_{1-3}$-Dialkylamino- oder 4-N-(Azacyclo-$C_{5-6}$-alkyl)-Rest substituiert ist, darstellen;
(c) wenn einer der Reste $R^a$ oder $R^b$ einen disubstituierten Phenylrest darstellt, der andere eine 4-Pyridylgruppe sein muß;
(d) wenn einer der Reste $R^a$ oder $R^b$ eine Cyanophenylgruppe darstellt, der andere eine Cyanophenyl- oder eine 4-Pyridylgruppe sein muß;
(e) $R^a$ und $R^b$ nicht beide eine 3-Pyridyl- oder 2-Pyridylgruppe darstellen; oder
II. einer der Reste $R^a$ oder $R^b$ eine Pyridylgruppe darstellt und der andere gewählt wird aus:
(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-($C_{1-3}$-Alkyl)-($C_{1-3}$-alkanamido)-, einem N-(Azacyclo-$C_{5-6}$-alkyl)-Rest, einer Prop2-en-1-oxy-Gruppe oder einem 2,2,2-Trihalogenethoxyrest gewählt wird;
(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-,2,2,2-Trihalogenethoxyrest, einer Prop-2-en-1-oxy- oder Hydroxygruppe gewählt werden, oder die Disubstituenten zusammen eine Methylendioxygruppe bilden; oder
(c) einem disubstituierten Phenylrest, wobei die Substituenten nicht gleich sind und unabhängig aus einem Halogenatom, einem $C_{1-3}$-Alkylaminorest, einer Nitrogruppe, einem N-($C_{1-3}$-Alkyl)-N-($C_{1-3}$-alkanamido)-, $C_{1-3}$-Dialkylaminorest, einer Aminogruppe oder einem N-(Azacyclo-$C_{5-6}$-alkyl)-Rest gewählt werden; oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-N-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^a$ eine 2- oder 3-Pyridylgruppe und $R^b$ einen monosubstituierten Phenylrest darstellt, der Substituent nicht aus einem Bromatom, einem Iodatom, einem N-($C_{1-3}$-Alkyl)-($C_{1-3}$-alkanamido)-, $C_{1-3}$-Alkylthio-, $C_{1-3}$-Alkylsulfinyl- oder $C_{1-3}$-Alkylsulfonylrest gewählt wird;

(2) wenn $R^a$ eine 2- oder 3-Pyridylgruppe und $R^b$ einen disubstituierten Phenylrest darstellt, die Disubstituenten beide nicht aus einem Bromatom, einem Iodatom, einer Amino-, Nitro-, Hydroxygruppe oder einem ($C_{1-3}$-Alkyl)-($C_{1-3}$-alkanamido)-Rest gewählt werden;

(3) wenn $R^b$ eine 2-, 3- oder 4-Pyridylgruppe und $R^a$ einen monosubstituierten Phenylrest darstellt, die Substituenten nicht aus einem Bromatom, einem Iodatom, einem N-($C_{1-3}$-Alkyl)-($C_{1-3}$-alkanamido)-, $C_{1-3}$-Alkylthio-, $C_{1-3}$-Alkylsulfinyl- oder einem $C_{1-3}$-Alkylsulfonylrest gewählt werden; und

(4) wenn $R^b$ eine 2-, 3- oder 4-Pyridylgruppe und $R^a$ einen disubstituierten Phenylrest darstellt, die Substituenten beide nicht aus einem Bromatom, einem Iodatom, einer Amino-, Nitro-, Hydroxygruppe, einem N-($C_{1-3}$-Alkyl)-N-($C_{1-3}$-alkanamido)-Rest gewählt werden,

oder ein pharmazeutisch verträgliches Salz davon.

**2.** Verbindung nach Anspruch 1, wobei:

(a) $R^a$ eine 4-Pyridylgruppe, $R^b$ eine 4-Fluorphenylgruppe, C ein H-Atom und A eine $CH_2$-Gruppe darstellt; oder

(b) $R^b$ eine 4-Methoxyphenylgruppe, $R^a$ eine 4-Pyridylgruppe, C ein H-Atom und A eine $CH_2$-Gruppe darstellt.

**3.** Verbindung nach Anspruch 1 der Formel (IC) beschrieben durch die Struktur:

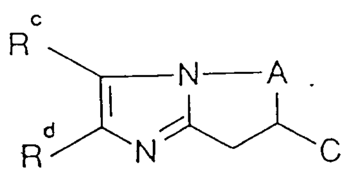

(IC)

wobei:

A eine Methylen- oder eine, gegebenenfalls mit einer Methyl-, Ethyl- oder gem-Dimethylgruppe substituierte 1,2-Ethandiylgruppe, darstellt und C ein H-Atom, eine Methyl-, Ethyl- oder gem-Dimethylgruppe darstellt;

I. einer der Reste $R^c$ oder $R^d$ gewählt werden muß aus: einer 2-Pyridyl-, 3-Pyridyl-, 4-Pyridylgruppe, einem monosubstituierten Phenylrest, wobei der Substituent aus einem $C_{1-3}$-Dialkylamino-, $C_{1-3}$-Alkylamino-, N-(Azacyclo-$C_{5-6}$-alkyl)-Rest, einer Cyanogruppe, einem 2,2,2-Trihalogenethoxy-, $C_{1-3}$-Alkanamido-, N-($C_{1-3}$-Alkyl)-($C_{1-3}$-alkanamido)-Rest oder einer Prop-2-en-1-oxy-Gruppe gewählt wird, oder einem disubstituierten Phenylrest, wobei die Substituenten unabhängig aus einem $C_{1-3}$-Alkyl- oder $C_{1-3}$-Alkoxyrest gewählt werden, oder die Disubstituenten zusammen eine Methylendioxygruppe bilden, und der andere der Reste $R^c$ oder $R^d$ gewählt wird aus:

1) einer Pyridylgruppe;

2) einer Phenylgruppe,

3) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;

4) einem disubstituierten Phenylrest, wobei die Substituenten unabhängig aus einem $C_{1-4}$-Alkyl- oder $C_{1-3}$-Alkoxyrest gewählt werden oder die Disubstituenten zusammen eine Methylendioxygruppe bilden; oder

5) einer 3,4,5-Trimethoxyphenylgruppe;

mit der Maßgabe, daß:

(a) wenn einer der Reste $R^c$ oder $R^d$ eine 4-Fluorphenylgruppe darstellt, der andere aus einer Pyridylgruppe oder einem Phenylrest, der in 4-Stellung mit einem 2,2,2-Trihalogenethoxyrest oder einer Prop-2-en-1-oxy-Gruppe substituiert ist, gewählt werden muß;

(b) $R^c$ und $R^d$ nicht beide einen Phenylrest, der in 2-, 3-, 5- oder 6-Stellung mit einem $C_{1-3}$-Alkylamino-, $C_{1-3}$-Dialkylamino- oder 4-N-(Azacyclo-$C_{5-6}$-alkyl)-Rest substituiert ist, darstellen;

(c) wenn einer der Reste $R^c$ oder $R^d$ einen disubstituierten Phenylrest darstellt, der andere eine 4-Pyridylgruppe sein muß;

(d) wenn einer der Reste $R^c$ oder $R^d$ eine Cyanophenylgruppe darstellt, der andere eine Cyanophenylgruppe oder eine 4-Pyridylgruppe sein muß;

(e) wenn $R^d$ einen $C_{1-3}$-Alkanamido- oder einen N-($C_{1-3}$-Alkyl)-($C_{1-3}$-alkanamido)-Rest darstellt, $R^c$ eine 4-Pyridylgruppe sein muß;

(f) $R^c$ und $R^d$ nicht beide eine 3-Pyridyl- oder 2-Pyridylgruppe darstellen; oder

II. wenn einer der Reste $R^c$ oder $R^d$ eine Pyridylgruppe darstellt, der andere gewählt wird aus:

(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-,2,2,2-Trihalogenethoxyrest, einer Prop-2-en-1-oxy-Gruppe gewählt werden oder die Disubstituenten zusammen eine Methylendioxygruppe bilden;

(c) einem disubstituierten Phenylrest, wobei die Substituenten nicht gleich sind und unabhängig aus einem $C_{1-3}$-Alkylamino-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^c$ eine 2- oder 3-Pyridylgruppe darstellt und $R^d$ einen monosubstituierten Phenylrest, der Substituent nicht aus einem Bromatom, einem Iodatom, einem $C_{1-3}$-Alkylthio-, $C_{1-3}$-Alkylsulfinyl- oder $C_{1-3}$-Alkylsulfonylrest gewählt wird;

(2) wenn $R^c$ eine 2- oder 3-Pyridylgruppe darstellt und $R^d$ einen disubstituierten Phenylrest, die Disubstituenten beide nicht aus einem Brom- oder Iodatom gewählt werden;

(3) wenn $R^d$ eine 2-, 3- oder 4-Pyridylgruppe darstellt und $R^c$ einen monosubstituierten Phenylrest, die Substituenten nicht aus einem Bromatom, einem Iodatom, einem $C_{1-3}$-Alkylthio-, $C_{1-3}$-Alkylsulfinyl- oder $C_{1-3}$-Alkylsulfonylrest gewählt werden; und

(4) wenn $R^d$ eine 2-, 3- oder 4-Pyridylgruppe darstellt und $R^c$ einen disubstituierten Phenylrest, die Substituenten beide nicht aus einem Brom- oder Iodatom gewählt werden;

oder ein pharmazeutisch verträgliches Salz davon.

**4.** Arzneimittel, das einen pharmazeutisch verträglichen Träger oder Verdünnungsmittel und eine wirksame, nichttoxische, den 5-Lipoxygenase-Weg inhibierende Menge einer Verbindung der Formel (IC), definiert gemäß Anspruch 3, umfaßt.

**5.** Mittel nach Anspruch 4, in einer Einheitsdosisform zur örtlichen, parenteralen, oralen oder Inhalierungsverabreichung.

**6.** Verbindung der Formel (IC) oder eines pharmazeutisch verträglichen Salzes davon, definiert gemäß Anspruch 3, zur Verwendung bei der Behandlung von rheumatoider Arthritis beim Tier.

**7.** Verwendung einer Verbindung bei der Herstellung eines Medikamentes zur Behandlung einer mit dem 5-Lipoxygenase-Weg zusammenhängenden Erkrankung anders als, oder zusätzlich zu, rheumatoider Arthritis, Rheumatismus, Entzündung oder jedes anderen von Cyclooxygenase hervorgerufenen Krankheitszustands, wobei die Verbindung die Formel (IA) aufweist:

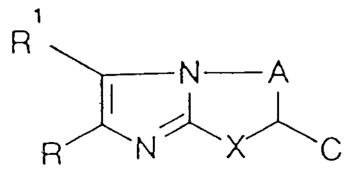

(IA)

wobei:

A und C gemäß Anspruch 1 definiert sind;

I. wenn X eine $CH_2$- oder $S(O)_n$-Gruppe darstellt und n den Wert 0, 1 oder 2 hat, die Reste $R^1$ und R unabhängig voneinander gewählt werden aus:

(a) einer Pyridylgruppe, mit der Maßgabe, daß, wenn einer oder beide der Reste R und $R^1$ eine Pyridylgruppe darstellen, X keine $CH_2$-Gruppe ist;

(b) einer Phenylgruppe;

(c) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;

(d) einem disubstituierten Phenylrest, wobei die Substituenten unabhängig voneinander aus einem $C_{1-4}$-Alkyl- oder $C_{1-3}$-Alkoxyrest gewählt werden, oder die Disubstituenten zusammen eine Methylendioxygruppe bilden;

(e) einer 3,4,5-Trimethoxyphenylgruppe;

oder einem pharmazeutisch verträglichen Salz davon, unter der Maßgabe, daß

(1) wenn X eine $CH_2$-Gruppe darstellt, beide Reste R und $R^1$ keinen Phenylrest darstellen, der in 2- oder 6-Stellung mit einem $C_{1-3}$-Alkylamino-, $C_{1-3}$-Dialkylamino- oder N-(Azacyclo-$C_{5-6}$-alkyl)-Rest substituiert ist;

(2) wenn einer der Reste R oder $R^1$ eine Cyanophenylgruppe darstellt, der andere eine Cyanophenylgruppe oder eine 4-Pyridylgruppe darstellen muß;

(3) wenn X eine $S(O)_n$-Gruppe darstellt und R oder $R^1$ einen Phenylrest, der in 2-, 3-, 5- oder 6-Stellung mit einem $C_{1-3}$-Alkylamino-, $C_{1-3}$-Dialkylamino- oder N-(Azacyclo-$C_{5-6}$-alkyl)-Rest substituiert ist, der andere eine 4-Pyridylgruppe darstellen muß; oder

II. wenn X eine $CH_2$-Gruppe darstellt und einer der Reste R oder $R^1$ eine Pyridylgruppe, wird der andere gewählt aus:

(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylamino-Rest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-,2,2,2-Trihalogenethoxyrest, einer Prop-2-en-1-oxy-Gruppe gewählt werden, oder die Disubstituenten zusammen eine Methylendioxygruppe bilden, oder

(c) einem disubstituierten Phenylrest, wobei die Substituenten nicht gleich sind und unabhängig aus einem $C_{1-3}$-Alkylamino-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^1$ eine 2- oder 3-Pyridylgruppe darstellt und R einen monosubstituierten Phenylrest, der Substituent nicht aus einem Bromatom, Iodatom, einem $C_{1-3}$-Alkylthio-, $C_{1-3}$-Alkylsulfinyl- oder $C_{1-3}$-Alkylsulfonylrest gewählt wird;

(2) wenn $R^1$ eine 2- oder 3-Pyridylgruppe darstellt und R einen disubstituierten Phenylrest, die Disubstituenten beide nicht aus einem Brom- oder einem Iodatom gewählt werden;

(3) wenn R eine 2-, 3- oder 4-Pyridylgruppe darstellt und $R^1$ einen monosubstituierten Phenylrest, die Substituenten nicht aus einem Bromatom, Iodatom, einem $C_{1-3}$-Alkylthio-, $C_{1-3}$-Alkylsulfinyl-

oder $C_{1-3}$-Alkylsulfonylrest gewählt werden; und

(4) wenn R eine 2-, 3- oder 4-Pyridylgruppe darstellt und $R^1$ einen disubstituierten Phenylrest, die Substituenten beide nicht aus einem Brom- oder Iodatom gewählt werden;

oder ein Hydrat davon, das eine Hydroxygruppe an dem Kohlenstoffatom aufweist, an dem der Rest R gebunden ist, wobei R keine Pyridylgruppe darstellt, $R^1$ einen Phenylrest darstellt, der in 2- oder 4-Stellung mit einem Halogenatom, einer $CF_3$-Gruppe oder einer Cyanogruppe substituiert ist, und X ein Schwefelatom darstellt;

oder ein pharmazeutisch verträgliches Salz davon.

**8.** Eine Verbindung der Formel (E), beschrieben durch die Struktur

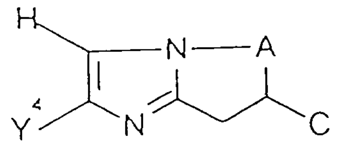

(E)

wobei:

A und C wie in Anspruch 1 definiert sind; und

$Y^4$ gewählt wird aus:

(a) einer Phenylgruppe;

(b) einem monosubstituierten Phenylrest, wobei der Substituent aus einem Halogenatom, einem $C_{1-3}$-Alkoxy-, $C_{1-3}$-Alkylthio-, $C_{1-4}$-Alkylrest, einer Cyanogruppe, einem N-($C_{1-3}$-Alkyl)-($C_{1-3}$-alkanamido)-, $C_{1-3}$-Alkanamido-, $C_{1-3}$-Alkylamino-, $C_{1-3}$-Dialkylaminorest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(c) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-, 2,2,2-Trihalogenethoxyrest, einer Prop-2-en-1-oxy-Gruppe, einer Hydroxygruppe gewählt werden, oder die Disubstituenten zusammen eine Methylendioxygruppe bilden;

(d) einem disubstituierten Phenylrest, wobei die Substituenten nicht gleich sind und unabhängig aus einem Halogenatom, einem $C_{1-3}$-Alkylamino-, $C_{1-3}$-Dialkylaminorest, einer Aminogruppe, einem N-(Azacyclo-$C_{5-6}$-alkyl)-Rest, einer Nitrogruppe oder einem N-($C_{1-3}$-Alkyl)-($C_{1-3}$-alkanamido)-Rest gewählt werden, oder

(e) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß, und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß, wenn A eine $CH_2CH_2$-Gruppe ist, C ein H-Atom darstellt und $Y^4$ keine 2,4-Dimethoxyphenyl- oder 4-Aminophenylgruppe ist;

oder ein pharmazeutisch verträgliches Salz davon.

**9.** Verbindung der Formel (F), beschrieben durch die Struktur:

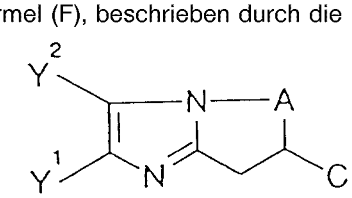

(F)

wobei:

A und C wie in Anspruch 1 definiert sind; und

$Y^2$ ein 4-(1,4-Dihydro)pyridylrest ist, der mit einem N-($C_{1-8}$-alkanoyl)-, N-($C_{1-8}$-Alkoxycarbonyl)rest, einer N-(Benzoyl)-, N-(Phenoxycarbonyl)-, N-(Phenylacetyl)- oder N-(Benzyloxycarbonyl)-Gruppe substituiert ist; $Y^1$ gewählt wird aus

(a) einer Phenylgruppe oder einem monosubstituierten Phenylrest, wobei der Substituent aus einem Halogenatom, einem $C_{1-3}$-Alkoxy-, $C_{1-3}$-Alkylthio-, $C_{1-4}$-Alkylrest, einer $CF_3$-Gruppe, einem N-

($C_{1-3}$-Alkyl)-($C_{1-3}$-alkanamido)-, $C_{1-3}$-Dialkylamino-, N-(Azacyclo-$C_{5-6}$-alkyl)-Rest, einer Prop-2-en-1-oxy-Gruppe, einem 2,2,2-Trihalogenethoxy-, $C_{1-3}$-Alkanamido- oder $C_{1-3}$-Dialkylaminorest gewählt wird;

(b) einem disubstituierten Phenylrest, wobei die Substituenten gleich sind und aus einem Halogenatom, einen $C_{1-3}$-Alkoxy-, $C_{1-3}$-Dialkylamino-, N-(Azacyclo-$C_{5-6}$-alkyl)-, $C_{1-3}$-Alkylamino-, 2,2,2-Trihalogenethoxyrest oder einer Prop-2-en-1-oxy-Gruppe, einer Hydroxygruppe gewählt werden oder die Disubstituenten zusammen eine Methylendioxygruppe bilden;

(c) einem disubstituierten Phenylrest, wobei die Substituenten nicht gleich sind und unabhängig aus einem Halogenatom, einer Nitrogruppe, einem $C_{1-3}$-Dialkylamino-, N-($C_{1-3}$-Alkyl)-($C_{1-3}$-alkanamido)-, oder N-(Azacyclo-$C_{5-6}$-alkyl)-Rest gewählt werden; oder

(d) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

oder ein pharmazeutisch verträgliches Salz davon.

**10.** Verbindung der Formel (G), beschrieben durch die Struktur:

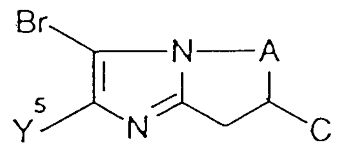

(G)

wobei:
A und C wie in Anspruch 1 definiert sind;
$Y^5$ gewählt wird aus:

(a) einer Phenylgruppe oder einem monosubstituierten Phenylrest, wobei der Substituent aus einem Fluoratom, einem Chloratom, einem $C_{1-3}$-Alkoxy-, $C_{1-4}$-Alkyl-,$C_{1-3}$-Dialkylaminorest, einer $CF_3$-Gruppe, einem $C_{1-3}$-Alkylamino-, N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Fluoratom, einem Chloratom, einem $C_{1-3}$-Alkoxy-, $C_{1-3}$-Dialkylamino-,N-(Azacyclo-$C_{5-6}$-alkyl)-, 2,2,2-Trihalogenethoxyrest oder einer Prop-2-en-1-oxy-Gruppe gewählt werden, oder die Disubstituenten zusammen eine Methylendioxygruppe bilden; oder

(c) einem disubstituiertem Phenylrest, wobei die Substituenten nicht gleich sind und unabhängig aus einem Fluoratom, einem Chloratom, einem $C_{1-3}$-Alkylamino-, $C_{1-3}$-Dialkylamino-, oder N-(Azacyclo-$C_{5-6}$-alkyl)-Rest gewählt werden; oder

(d) einem disubstituierten Phenylrest, wobei einer der Substituenten ein $C_{1-3}$-Alkoxy-, ein 2,2,2-Trihalogenethoxyrest oder eine Prop-2-en-1-oxy-Gruppe sein muß und der andere Substituent unabhängig davon aus einem Fluoratom, einem Chloratom $C_{1-3}$-Alkylamino-, $C_{1-3}$-Dialkylamino- oder N-(Azacyclo-$C_{5-6}$-alkyl)-Rest gewählt wird;

oder ein pharmazeutisch verträgliches Salz davon.

**11.** Verbindung der Formel (H), beschrieben durch die Struktur:

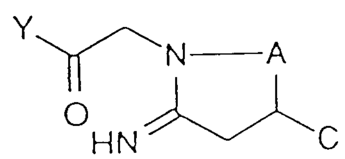

(H)

wobei:
A und C wie in Anspruch 1 definiert sind;
Y gewählt wird aus:

(a) einer Phenylgruppe oder einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkanamido-, $C_{1-3}$-Dialkylami-

norest, einer $CF_3$-Gruppe, einem $C_{1-3}$-Alkylamino-, N-(Azacyclo-$C_{5-6}$-alkyl)- oder N-($C_{1-3}$-Alkyl)-($C_{1-3}$-alkanamido)-Rest gewählt wird;

(b) einem disubstituierten Phenylrest, wobei die Substituenten gleich sind und aus einem Halogenatom, einem $C_{1-3}$-Alkylamino-, N-(Azacyclo-$C_{5-6}$-alkyl)- oder $C_{1-3}$-Alkoxyrest gewählt werden, oder die Disubstituenten zusammen eine Methylendioxygruppe bilden;

(c) einem disubstituierten Phenylrest, wobei die Substituenten nicht gleich sind und unabhängig aus einem Halogenatom, einer Nitrogruppe, einem $C_{1-3}$-Alkanamido-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß, und der andere Substituent unabhängig davon aus einem Halogenatom, einem N-($C_{1-3}$-Alkyl)-($C_{1-3}$-alkanamido)-, $C_{1-3}$-Dialkylamino- oder N-(Azacyclo-$C_{5-6}$-alkyl)-Rest gewählt wird;

mit der Maßgabe, daß, wenn A eine $CH_2CH_2$-Gruppe darstellt und C ein H-Atom, Y keine 2,4-Dimethoxyphenyl- oder 4-Aminophenylgruppe ist;

oder ein pharmazeutisch verträgliches Salz davon.

**12.** Verfahren zur Herstellung einer Verbindung der Formel (IB) von Anspruch 1, welches umfaßt:

(i) das Umsetzen einer Verbindung der Formel (G) von Anspruch 11 mit einem $C_{1-5}$-Alkyllithium-Reagens, um das entsprechende Lithiumreagens durch Lithium-Halogen-Austausch zu erhalten;

(ii) die Zugabe eines Überschusses an Magnesium-Halogenid-Etherat zum Lithium-Reagens, um das entsprechende Grignard-Reagens durch Transmetallierung zu erhalten;

(iii) die Zugabe des Grignard-Reagens zu einem N-Acylpyridium-Salz, um die entsprechende Verbindung der Formel (F) zu erhalten;

(iv) die Desacylierung/Oxidation der Verbindung der Formel (F), um die entsprechende Verbindung der Formel (IB) zu erhalten.

**13.** Verbindung nach Anspruch 1, gewählt aus der Gruppe umfassend:

eine Verbindung, in der $R^a$ eine 4-Pyridylgruppe, $R^b$ eine 4-Fluorphenylgruppe, A eine $CH_2$-Gruppe und C ein H-Atom darstellt; und eine Verbindung, in der $R^a$ eine 4-Pyridylgruppe, $R^b$ eine 4-Methoxyphenylgruppe, A eine $CH_2$-Gruppe und C ein H-Atom darstellt;

und pharmazeutisch verträgliche Salze davon.

**14.** Verwendung einer Verbindung der Formel (IA), gemäß Anspruch 7 definiert, bei der Herstellung eines Medikamentes zur Behandlung von Asthma oder Darmentzündung.

**Patentansprüche für folgende Vertragsstaaten : AT, GR, ES**

**1.** Verfahren zur Herstellung einer Verbindung der Formel (IB)

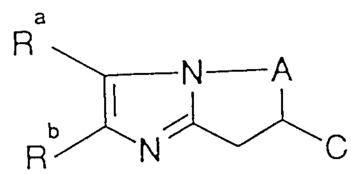

(IB)

wobei:

A eine Methylen- oder 1,2-Ethandiylgruppe, gegebenenfalls mit einer Methyl-, Ethyl- oder gem-Dimethylgruppe substituiert, darstellt;

C ein H-Atom, eine Methylgruppe, eine Ethylgruppe oder eine gem-Dimethylgruppe darstellt; und

I. einer der Reste $R^a$ oder $R^b$ gewählt werden muß aus: einer 2-Pyridyl-, 3-Pyridyl-, 4-Pyridylgruppe, einem monosubstituierten Phenylrest, wobei der Substituent aus einem $C_{1-3}$-Dialkylamino-, $C_{1-3}$-Alkylamino-, N-(Azacyclo-$C_{5-6}$-alkyl)-Rest, einer Cyanogruppe, einem 2,2,2-Trihalogenethoxy-, $C_{1-3}$-Alkanamido-, N-($C_{1-3}$-Alkyl)-($C_{1-3}$-alkanamido)-Rest oder einer Prop-2-en-1-oxy-Gruppe gewählt wird, oder einem disubstituierten Phenylrest, wobei die Substituenten unabhängig aus einem $C_{1-4}$-Alkyl- oder $C_{1-3}$-Alkoxyrest gewählt werden, oder die Disubstituenten zusammen eine Methylendioxygruppe bilden; und der andere der Reste $R^a$ oder $R^b$ gewählt wird aus:

1) einer Pyridylgruppe;

2) einer Phenylgruppe;

3) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;

4) einem disubstituierten Phenylrest, wobei die Substituenten unabhängig aus einem $C_{1-4}$-Alkyl- oder $C_{1-3}$-Alkoxyrest gewählt werden oder die Disubstituenten zusammen eine Methylendioxygruppe bilden; oder

5) einer 3,4,5-Trimethoxyphenylgruppe;

mit der Maßgabe, daß:

(a) wenn einer der Reste $R^a$ oder $R^b$ eine 4-Fluorphenylgruppe darstellt, der andere aus einer Pyridylgruppe oder einem Phenylrest, der in 4-Stellung mit einem 2,2,2-Trihalogenethoxyrest oder einer Prop-2-en-1-oxy-Gruppe substituiert ist, gewählt werden muß;

(b) $R^a$ und $R^b$ nicht beide einen Phenylrest, der in 2-, 3-, 5- oder 6-Stellung mit einem $C_{1-3}$-Alkylamino-, $C_{1-3}$-Dialkylamino- oder 4-N-(Azacyclo-$C_{5-6}$-alkyl)-Rest substituiert ist, darstellen;

(c) wenn einer der Reste $R^a$ oder $R^b$ einen disubstituierten Phenylrest darstellt, der andere eine 4-Pyridylgruppe sein muß;

(d) wenn einer der Reste $R^a$ oder $R^b$ eine Cyanophenylgruppe darstellt, der andere eine Cyanophenyl- oder eine 4-Pyridylgruppe sein muß;

(e) $R^a$ und $R^b$ nicht beide eine 3-Pyridyl- oder 2-Pyridylgruppe darstellen; oder

II. einer der Reste $R^a$ oder $R^b$ eine Pyridylgruppe darstellt und der andere gewählt wird aus:

(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-($C_{1-3}$-Alkyl)-($C_{1-3}$-alkanamido)-, einem N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-, 2,2,2-Trihalogenethoxyrest, einer Prop-2-en-1-oxy- oder Hydroxygruppe gewählt werden, oder die Disubstituenten zusammen eine Methylendioxygruppe bilden;

(c) einem disubstituierten Phenylrest, wobei die Substituenten nicht gleich sind und unabhängig aus einem Halogenatom, einem $C_{1-3}$-Alkylaminorest, einer Nitrogruppe, einem N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt werden; oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^a$ eine 2- oder 3-Pyridylgruppe und $R^b$ einen monosubstituierten Phenylrest darstellt, der Substituent nicht aus einem Bromatom, einem Iodatom, einem N-($C_{1-3}$-Alkyl)-($C_{1-3}$-alkanamido)-, $C_{1-3}$-Alkylthio-, $C_{1-3}$-Alkylsulfinyl- oder $C_{1-3}$-Alkylsulfonylrest gewählt wird;

(2) wenn $R^a$ eine 2- oder 3-Pyridylgruppe und $R^b$ einen disubstituierten Phenylrest darstellt, die Disubstituenten beide nicht aus einem Bromatom, einem Iodatom, einer Amino-, Nitro-, Hydroxygruppe oder einem N-($C_{1-3}$-Alkyl)-($C_{1-3}$-alkanamido)-Rest gewählt werden;

(3) wenn $R^b$ eine 2-, 3- oder 4-Pyridylgruppe und $R^a$ einen monosubstituierten Phenylrest darstellt, die Substituenten nicht aus einem Bromatom, einem Iodatom, einem N-($C_{1-3}$-Alkyl)-($C_{1-3}$-alkanamido)-, $C_{1-3}$-Alkylthio-, $C_{1-3}$-Alkylsulfinyl- oder $C_{1-3}$-Alkylsulfonylrest gewählt werden; und

(4) wenn $R^b$ eine 2-, 3- oder 4-Pyridylgruppe und $R^a$ einen disubstituierten Phenylrest darstellt, die Substituenten beide nicht aus einem Bromatom, einem Iodatom, einer Amino-, Nitro-, Hydroxygruppe, einem N-($C_{1-3}$-Alkyl)-($C_{1-3}$-alkanamido)-Rest gewählt werden,

oder eines pharmazeutisch verträglichen Salzes davon,

welches umfaßt:

A. wenn die Herstellung einer Verbindung der Formel (IB) gefordert ist, wobei $R^a$ eine 4-Pyridylgruppe darstellt und $R^b$ keine Pyridylgruppe ist, die Umsetzung einer Verbindung der Formel (E), beschrieben durch die Struktur:

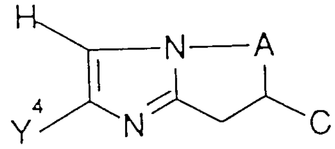

(E)

wobei

A und C wie vorstehend definiert sind; und

$Y^4$ gewählt wird aus

(a) einer Phenylgruppe;

(b) einem monosubstituierten Phenylrest, wobei der Substituent aus einem Halogenatom, einem $C_{1-3}$-Alkoxy-, $C_{1-3}$-Alkylthio-, $C_{1-4}$-Alkylrest, einer Cyanogruppe, einem N-($C_{1-3}$-Alkyl)-($C_{1-3}$-alkanamido)-, $C_{1-3}$-Alkanamido-, $C_{1-3}$-Alkylamino-, $C_{1-3}$-Dialkylaminorest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(c) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-, 2,2,2-Trihalogenethoxyrest, einer Prop-2-en-1-oxy-Gruppe, einer Hydroxygruppe gewählt werden, oder die Disubstituenten zusammen eine Methylendioxygruppe bilden;

(d) einem disubstituierten Phenylrest, wobei die Substituenten nicht gleich sind und unabhängig aus einem Halogenatom, einem $C_{1-3}$-Alkylamino-, $C_{1-3}$-Dialkylaminorest, einer Aminogruppe, einem N-(Azacyclo-$C_{5-6}$-alkyl)-Rest, einer Nitrogruppe oder einem N-($C_{1-3}$-Alkyl)-($C_{1-3}$-alkanamido)-Rest gewählt werden, oder

(e) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß, und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß, wenn A eine $CH_2CH_2$-Gruppe ist, und C ein H-Atom darstellt, $Y^4$ keine 2,4-Dimethoxyphenyl- oder 4-Aminophenylgruppe ist,

oder ein pharmazeutisch verträgliches Salz davon,

mit Pyridin und einem Acylhalogen oder einem Halogenacylester, um eine Verbindung der Formel (F), beschrieben durch die Struktur:

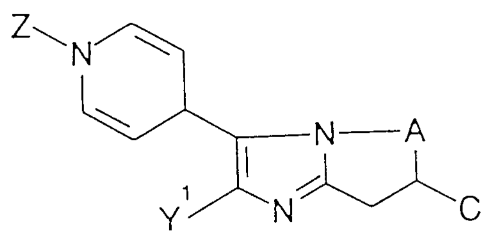

(F)

zu erhalten, wobei:

A und C wie vorstehend definiert sind; und

$Y^1$ gewählt wird aus

(a) einer Phenylgruppe oder einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, N-($C_{1-3}$-Alkyl)-($C_{1-3}$-alkanamido)-, $C_{1-3}$-Dialkylaminorest, einer $CF_3$-Gruppe, einem N-(Azacyclo-$C_{5-6}$-alkyl)-, $C_{1-3}$-Alkanamido- oder $C_{1-3}$-Alkylaminorest, einer Prop-2-en-1-oxy-Gruppe, oder einem 2,2,2-Trihalogenethoxyrest, gewählt wird;

(b) einem disubstituierten Phenylrest, wobei die Substituenten gleich sind und aus einem Halogenatom, einen $C_{1-3}$-Alkoxy-, $C_{1-3}$-Dialkylamino-, $C_{1-3}$-Alkylaminorest, einer Hydroxygruppe, einem N-(Azacyclo-$C_{5-6}$-alkyl)-, 2,2,2-Trihalogenethoxyrest oder einer Prop-2-en-1-oxy-Gruppe, gewählt werden oder die Disubstituenten zusammen eine Methylendioxygruppe bilden;

(c) einem disubstituierten Phenylrest, wobei die Substituenten nicht gleich sind und unabhängig aus einem Halogenatom, einer Nitrogruppe, einem $C_{1-3}$-Dialkylamino-, N-($C_{1-3}$-Alkyl)-($C_{1-3}$-

alkanamido)-, oder N-(Azacyclo-$C_{5-6}$-alkyl)-Restgewählt werden; oder

(d) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß und der andere Substituent unabhängig davon aus einem Halogenatom, einem $C_{1-3}$-Alkylamino-, N-($C_{1-3}$-Alkyl)-($C_{1-3}$-alkanamido), $C_{1-3}$-Dialkylaminorest, einer Aminogruppe oder einem N-(Azacyclo-$C_{5-6}$-alkyl)-Rest gewählt wird;

und Z einen $C_{1-8}$-Alkanoyl-, $C_{1-8}$-Alkoxycarbonylrest, eine Benzoyl-, Phenoxycarbonyl-, Phenylacetyl- oder Benzyloxycarbonylgruppe darstellt;

gefolgt von einer Deacylierung/Oxidation einer Verbindung der Formel (F), um eine Verbindung der Formel (IB) zu erhalten; und

B. wenn die Herstellung einer Hydroxyverbindung der Formel (IB) gefordert ist, die Demethylierung einer Methoxyphenylverbindung der Formel (I), wobei Formel I durch die Struktur

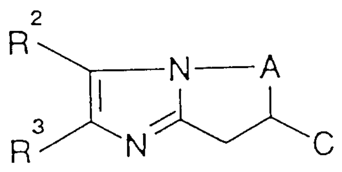

(I)

beschrieben wird und

A und C wie vorstehend definiert sind;

$R^2$ oder $R^3$ unabhängig gewählt werden aus:

a) einer Pyridylgruppe,

b) einer Phenylgruppe oder einem monosubstituierten Phenylrest, wobei der Substituent aus einem $C_{1-3}$-Dialkylamino-, $C_{1-3}$-Alkylamino-, N-(Azacyclo-$C_{5-6}$-alkyl)-Rest, einer Cyanogruppe, einem 2,2,2-Trihalogenethoxyrest, einer Prop-2-en-1-oxy-Gruppe, einem N-($C_{1-3}$-Alkyl)-($C_{1-3}$-alkanamido)-, $C_{1-3}$-Alkanamidorest, einer Amino-, Hydroxygruppe, einem $C_{1-3}$-Alkylthio-, $C_{1-4}$-Alkylrest, einem Halogenatom, einer $CF_3$-Gruppe, einem $C_{1-3}$-Alkoxy-, $C_{1-3}$-Alkylsulfinyl- oder $C_{1-3}$-Alkylsulfonylrest gewählt wird,

c) einem disubstituierten Phenylrest, wobei die Substituenten unabhängig aus einem $C_{1-4}$-Alkyl-, $C_{1-3}$-Alkoxyrest, einer Hydroxy-, Nitro-, Aminogruppe, einem Halogenatom, einem $C_{1-3}$-Alkylamino-, $C_{1-3}$-Dialkylamino-, N-(Azacyclo-$C_{5-6}$-alkyl), 2,2,2-Trihalogenethoxyrest, einer Prop-2-en-1-oxy-Gruppe oder einem N-($C_{1-3}$-Alkyl)-($C_{1-3}$-alkanamido)-Rest gewählt werden oder die Disubstituenten zusammen eine Methylendioxygruppe bilden oder

d) einer 3,4,5-Trimethoxyphenylgruppe;

mit der Maßgabe, daß:

(1) wenn $R^3$ eine Cyanophenylgruppe darstellt, $R^2$ entweder eine Cyanophenyl- oder eine 4-Pyridylgruppe sein muß,

oder eines pharmazeutisch verträglichen Salzes davon,

mit HBr in Essigsäure oder $BBr_3$ in Methylenchlorid, um die entsprechende Hydroxyverbindung der Formel (IB) zu erhalten, und

C. eine O-Alkylierung der Hydroxyverbindung der Formel (I), wie vorstehend definiert, wenn A eine $CH_2$-Gruppe darstellt, und mindestens einer der Reste $R^2$ und $R^3$ eine Hydroxyphenylgruppe darstellt, oder wenn A eine $CH_2CH_2$-Gruppe darstellt und nur einer der Reste $R^2$ und $R^3$ eine Hydroxyphenylgruppe darstellt, um die entsprechende 2,2,2-Trihalogenethoxyphenyl- oder Prop-2-en-1-oxyphenyl-substituierte Verbindung der Formel (IB) zu erhalten; und

D. die Acylierung einer Aminophenyl- oder ($C_{1-3}$-Alkylamino)phenyl-Verbindung der Formel (IB) oder (I),

wobei Formel (IB) und Formel (I) jeweils wie vorstehend definiert sind, und A eine $CH_2CH_2$-Gruppe darstellt und $R^2$ und $R^3$ der Formel (I) keine Pyridylgruppe darstellen, oder, wenn A eine $CH_2$-Gruppe darstellt und einer der Reste $R^2$ oder $R^3$ eine Pyridylgruppe darstellt, mit dem entsprechend substituierten Acylhalogenid oder Anhydrid in Pyridin, um die entsprechende ($C_{1-3}$-Alkanamido)phenyl- oder die entsprechende N-($C_{1-3}$-Alkyl)-($C_{1-3}$-alkanamido)phenyl-Verbindung der Formel (IB) zu erhalten; und

E. die Alkylierung einer ($C_{1-3}$-Alkanamido)phenyl-Verbindung der Formel (I), wie vorstehend definiert, mit einem Alkylierungsmittel in Gegenwart einer Base, um die entsprechende N-($C_{1-3}$-Alkyl)-($C_{1-3}$-alkanamido)phenyl-Verbindung der Formel (IB) zu erhalten; und

F. die Hydrolyse einer ($C_{1-3}$-Alkanamido)phenyl- oder N-($C_{1-3}$-alkyl)-($C_{1-3}$-alkanamido)phenyl-Verbindung der Formel (I), wie vorstehend definiert, um die entsprechende Aminophenyl- oder ($C_{1-3}$-Alkylamino)phenyl-Verbindung der Formel (IB) zu erhalten; und

G. die Oxidation einer Verbindung der Formel (IB), wobei einer der oder beide Reste $R^a$ und $R^b$ einen $C_{1-3}$-Alkylthiorest darstellen, mit Metachlorperbenzoesäure, um die entsprechende $C_{1-3}$-Alkylsulfinyl-, $C_{1-3}$-Alkylsulfonylverbindung der Formel (IB) zu erhalten; und

H. die Reduktion einer Verbindung der Formel (I), wie vorstehend definiert, wobei einer der oder beide Reste $R^2$ und $R^3$ der Formel (I) eine Nitrophenylgruppe darstellen, mit Wasserstoffgas über einen Palladiumkatalysator, um die entsprechende Aminophenylverbindung der Formel (IB) zu erhalten; und

I. die Cycloalkylierung einer Verbindung der Formel (I), wie vorstehend definiert, wobei einer der oder beide Reste $R^2$ und $R^3$ eine Aminophenylgruppe darstellen, mit Dihalogenbutan oder Dihalogenpentan, je nach Eignung, in Gegenwart einer Base, um die entsprechende N-(Azacyclo-$C_{5-6}$-alkyl)phenyl-Verbindung der Formel (IB) zu erhalten; und

J. wenn die Herstellung von Verbindungen der Formel (IB), wie vorstehend definiert, wobei $R^a$ und $R^b$ jeweils ($C_{1-3}$-Alkylamino)phenylreste darstellen, gefordert wird, die Reduktion der entsprechenden $C_{1-3}$-Alkanamidoverbindungen mit Boran oder einem Borandimethylsulfidkomplex in Tetrahydrofuran, um die gewünschten Verbindungen der Formel (IB) zu erhalten; und

K. wenn die Herstellung von Verbindungen der Formel (IB), wie vorstehend definiert, wobei $R^a$ und $R^b$ ($C_{1-3}$-Dialkylamino)phenylreste darstellen, gefordert wird, die Reduktion der entsprechenden N-($C_{1-3}$-Alkyl)-($C_{1-3}$-alkanamido)-Verbindungenmit Boran oder einem Borandimethylsulfidkomplex in Tetrahydrofuran, um die gewünschten Verbindungen der Formel (IB) zu erhalten; und

L.

(i) Umsetzen einer Verbindung der Formel (G):

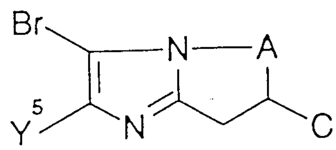

(G)

wobei:

A und C wie vorstehend definiert sind,

$Y^5$ gewählt wird aus:

(a) einer Phenylgruppe oder einem monosubstituierten Phenylrest, wobei der Substituent aus einem Fluoratom, einem Chloratom, einem $C_{1-3}$-Alkoxy-, $C_{1-4}$-Alkyl-, $C_{1-3}$-Dialkylaminorest, einer $CF_3$-Gruppe, einem $C_{1-3}$-Alkylamino-, N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Fluoratom, einem Chloratom, einem $C_{1-3}$-Alkoxy-, $C_{1-3}$-Dialkylamino-, N-(Azacyclo-$C_{5-6}$-alkyl)-, 2,2,2-Trihalogenethoxyrest oder einer Prop-2-en-1-oxy-Gruppe gewählt werden, oder die Disubstituenten zusammen eine Methylendioxygruppe bilden;

(c) einem disubstituiertem Phenylrest, wobei die Substituenten nicht gleich sind und unabhängig aus einem Fluoratom, einem Chloratom, einem $C_{1-3}$-Alkylamino-, $C_{1-3}$-Dialkylamino-, oder N-(Azacyclo-$C_{5-6}$-alkyl)-Rest gewählt werden; oder

(d) einem disubstituierten Phenylrest, wobei einer der Substituenten ein $C_{1-3}$-Alkoxy-, ein 2,2,2-Trihalogenethoxyrest oder eine Prop-2-en-1-oxy-Gruppe sein muß und der andere Substituent unabhängig davon aus einem Fluoratom, einem Chloratom, $C_{1-3}$-Alkylamino-, $C_{1-3}$-Dialkylamino- oder N-(Azacyclo-$C_{5-6}$-alkyl)-Rest gewählt wird;

oder eines pharmazeutisch verträglichen Salzes davon,

mit einem $C_{1-5}$-Alkyllithium-Reagens, um das entsprechende Lithiumreagens durch Lithium-Halogen-Austausch zu erhalten,

(ii) die Zugabe eines Überschusses an Magnesium-Halogenid-Etherat zum Lithium-Reagens, um das entsprechende Grignard-Reagens durch Transmetallierung zu erhalten;

(iii) die Zugabe des Grignard-Reagens zu einem N-Acylpyridium-Salz, um die entsprechende Verbindung der Formel (F) zu erhalten;

(iv) die Desacylierung/Oxidation der Verbindung mit der Formel (F), um die entsprechende Verbindung der Formel (IB) zu erhalten,

und danach gegebenenfalls die Überführung einer Verbindung der Formel (IB) in eine andere Verbindung der Formel (IB).

**2.** Verfahren zur Herstellung einer Verbindung der Formel (E), wie vorstehend in Anspruch 1 definiert; welches umfaßt:

A. Erhitzen unter Rückfluß in Wasser oder einem wäßrigen Lösungsmittel von der Verbindung der Formel (H), beschrieben durch die Struktur

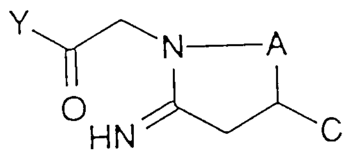

(H)

wobei:

A und C wie in Anspruch 1 definiert sind;

Y gewählt wird aus:

(a) einer Phenylgruppe oder einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylamino-, $C_{1-3}$-Alkanamido-, $C_{1-3}$-Dialkylaminorest, einer $CF_3$-Gruppe, einem N-(Azacyclo-$C_{5-6}$-alkyl)- oder N-($C_{1-3}$-Alkyl)-($C_{1-3}$-alkanamido)-Rest gewählt wird;

(b) einem disubstituierten Phenylrest, wobei die Substituenten gleich sind und aus einem Halogenatom, einem $C_{1-3}$-Dialkylamino-, N-(Azacyclo-$C_{5-6}$-alkyl)- oder $C_{1-3}$-Alkoxyrest gewählt werden, oder die Disubstituenten zusammen eine Methylendioxygruppe bilden;

(c) einem disubstituierten Phenylrest, wobei die Substituenten nicht gleich sind und unabhängig aus einem Halogenatom, einer Nitrogruppe, einem $C_{1-3}$-Alkanamido-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten ein $C_{1-3}$-Alkoxyrest, eine Hydroxygruppe, ein 2,2,2-Trihalogenethoxyrest oder eine Prop-2-en-1-oxy-Gruppe sein muß, und der andere Substituent unabhängig davon aus einem Halogenatom, einem N-($C_{1-3}$-Alkyl)-($C_{1-3}$-alkanamido)-, $C_{1-3}$-Dialkylamino- oder einem N-(Azacyclo-$C_{5-6}$-alkyl)-Rest gewählt wird;

mit der Maßgabe, daß, wenn A eine $CH_2CH_2$-Gruppe darstellt und C ein H-Atom, Y keine 2,4-Dimethoxyphenyl- oder 4-Aminophenylgruppe ist;

oder eines pharmazeutisch verträglichen Salzes davon und

B. die Alkylierung einer ($C_{1-3}$-Alkanamido)phenyl-Verbindung der Formel (E), wie vorstehend definiert, mit einem Alkylierungsmittel in Gegenwart einer Base, um die entsprechende N-($C_{1-3}$-Alkyl)-($C_{1-3}$-Alkanamido)phenyl-Verbindung der Formel (E) zu erhalten; und

C. die Hydrolyse einer ($C_{1-3}$-Alkanamido)phenyl- oder N-($C_{1-3}$-Alkyl)-($C_{1-3}$-alkanamido)phenyl-Verbindung der Formel (E), wie vorstehend definiert, um die entsprechende Aminophenyl- oder ($C_{1-3}$-Alkylamino)phenyl-Verbindung der Formel (E) zu erhalten; und

D. die Reduktion einer Verbindung der Formel (E), wie vorstehend definiert, wobei $Y^4$ der Formel (E) eine Nitrophenylgruppe darstellt, mit Wasserstoffgas über einen Palladiumkatalysator, um die entsprechende Aminophenylverbindung der Formel (E) zu erhalten; und

E. die Cycloalkylierung einer Verbindung der Formel (E), wie vorstehend definiert, wobei $Y^4$ eine Aminophenylgruppe darstellt, mit Dihalogenbutan oder Dihalogenpentan, je nach Eignung, in Gegenwart einer Base, um die entsprechende N-(Azacyclo-$C_{5-6}$-alkyl)phenyl-Verbindung der Formel (E) zu erhalten; und

F. eine O-Alkylierung der Hydroxyverbindung der Formel (E), wobei A eine $CH_2$-Gruppe darstellt und $Y^4$ eine Hydroxyphenylgruppe, oder A eine $CH_2CH_2$-Gruppe darstellt und $Y^4$ eine Hydroxyphenylgruppe, um die entsprechende 2,2,2-Trihalogenethoxyphenyl- oder Prop-2-en-1-oxyphenyl-substituierte Verbindung der Formel (E) zu erhalten.

**3.** Verfahren zur Herstellung einer Verbindung der Formel (H) wie in Anspruch 2 definiert, welches die Umsetzung einer Verbindung der Formel (D)

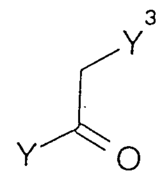

(D)

wobei Y wie in Formel (H) definiert ist und $Y^3$ ein Halogenatom darstellt, mit einer Verbindung der Formel (C)

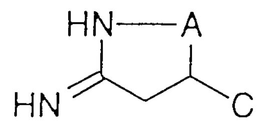

(C)

wobei A und C wie in Formel (H) definiert sind, umfaßt.

**4.** Verfahren zur Herstellung einer Verbindung der Formel (G), wie vorstehend in Anspruch 1 definiert, welches umfaßt:

A. die Umsetzung einer Verbindung der Formel (E), wie vorstehend in Anspruch 1 definiert, mit Brom; und

B. die Hydrolyse einer ($C_{1-3}$-Alkanamido)phenyl- oder N-($C_{1-3}$-Alkyl)-($C_{1-3}$-alkanamido)phenyl-Verbindung der Formel (G), wie vorstehend definiert, um die entsprechende Aminophenyl- oder ($C_{1-3}$-Alkylamino)phenyl-Verbindung der Formel (G) zu erhalten.

**5.** Verfahren zur Herstellung einer Verbindung der Formel (F), definiert wie in Anspruch 1, welches die Umsetzung einer Verbindung der Formel (E), definiert gemäß Anspruch 1, mit Pyridin und einem Acylhalogenid oder Halogenacylester umfaßt.

**6.** Verfahren nach Anspruch 1 zur Herstellung einer Verbindung der Formel (IB), wobei:

(a) $R^a$ eine 4-Pyridylgruppe, $R^b$ eine 4-Fluorphenylgruppe, A eine $CH_2$-Gruppe und C ein H-Atom darstellt; und

(b) $R^b$ eine 4-Methoxyphenylgruppe, $R^a$ eine 4-Pyridylgruppe, A eine $CH_2$-Gruppe und C ein H-Atom darstellt.

**7.** Verfahren nach Anspruch 1 zur Herstellung einer Verbindung der Formel (IC):

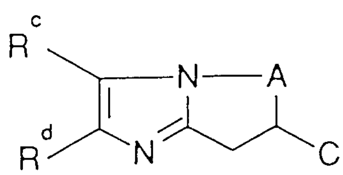

(IC)

wobei:

A und C wie in Anspruch 1 definiert sind,

einer der Reste $R^c$ oder $R^d$ gewählt werden muß aus: einer 2-Pyridyl-, 3-Pyridyl-, 4-Pyridylgruppe, einem monosubstituierten Phenylrest, wobei der Substituent aus einem $C_{1-3}$-Dialkylamino-, $C_{1-3}$-Alkylamino-, N-(Azacyclo-$C_{5-6}$-alkyl)-Rest, einer Cyanogruppe, einem 2,2,2-Trihalogenethoxy-, $C_{1-3}$-Alkanamido-, N-($C_{1-3}$-Alkyl)-($C_{1-3}$-alkanamido)-Rest oder einer Prop-2-en-1-oxy-Gruppe gewählt wird, oder einem disubstituierten Phenylrest, wobei die Substituenten unabhängig aus einem $C_{1-4}$-Alkyl- oder $C_{1-3}$-Alkoxyrest gewählt werden, oder die Disubstituenten zusammen eine Methylendioxygruppe bilden, und der andere der Reste $R^c$ oder $R^d$ gewählt wird aus:

1) einer Pyridylgruppe;

2) einer Phenylgruppe,

3) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;

4) einem disubstituierten Phenylrest, wobei die Substituenten unabhängig aus einem $C_{1-4}$-Alkyl- oder $C_{1-3}$-Alkoxyrest gewählt werden oder die Disubstituenten zusammen eine Methylendioxygruppe bilden; oder

5) einer 3,4,5-Trimethoxyphenylgruppe;

mit der Maßgabe, daß:

(a) wenn einer der Reste $R^c$ oder $R^d$ eine 4-Fluorphenylgruppe darstellt, der andere aus einer Pyridylgruppe oder einem Phenylrest, der in 4-Stellung mit einem 2,2,2-Trihalogenethoxyrest oder einer Prop-2-en-1-oxy-Gruppe substituiert ist, gewählt werden muß;

(b) $R^c$ und $R^d$ nicht beide einen Phenylrest, der in 2-, 3-, 5- oder 6-Stellung mit einem $C_{1-3}$-Alkylamino-, $C_{1-3}$-Dialkylamino- oder 4-N-(Azacyclo-$C_{5-6}$-alkyl)-Rest substituiert ist, darstellen;

(c) wenn einer der Reste $R^c$ oder $R^d$ einen disubstituierten Phenylrest darstellt, der andere eine 4-Pyridylgruppe sein muß;

(d) wenn einer der Reste $R^c$ oder $R^d$ eine Cyanophenylgruppe darstellt, der andere eine Cyanophenylgruppe oder eine 4-Pyridylgruppe sein muß;

(e) wenn $R^d$ einen $C_{1-3}$-Alkanamido- oder einen N-($C_{1-3}$-Alkyl)-($C_{1-3}$-alkanamido)-Rest darstellt, $R^c$ eine 4-Pyridylgruppe sein muß;

(f) $R^c$ und $R^d$ nicht beide eine 3-Pyridyl- oder 2-Pyridylgruppe darstellen; oder

wenn einer der Reste $R^c$ oder $R^d$ eine Pyridylgruppe darstellt, der andere gewählt wird aus:

(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-, 2,2,2-Trihalogenethoxyrest, einer Prop-2-en-1-oxy-Gruppe gewählt werden oder die Disubstituenten zusammen eine Methylendioxygruppe bilden; oder

(c) einem disubstituierten Phenylrest, wobei die Substituenten nicht gleich sind und unabhängig aus einem $C_{1-3}$-Alkylamino-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^c$ eine 2- oder 3-Pyridylgruppe darstellt und $R^d$ einen monosubstituierten Phenylrest, der Substituent nicht aus einem Bromatom, einem Iodatom, einem $C_{1-3}$-Alkylthio-, $C_{1-3}$-Alkylsulfinyl- oder $C_{1-3}$-Alkylsulfonylrest gewählt wird;

(2) wenn $R^c$ eine 2- oder 3-Pyridylgruppe darstellt und $R^d$ einen disubstituierten Phenylrest, die Disubstituenten beide nicht aus einem Brom- oder Iodatom gewählt werden;

(3) wenn $R^d$ eine 2-, 3- oder 4-Pyridylgruppe darstellt und $R^c$ einen monosubstituierten Phenylrest, die Substituenten nicht aus einem Bromatom, einem Iodatom, einem $C_{1-3}$-Alkylthio-, $C_{1-3}$-Alkylsulfinyl- oder $C_{1-3}$-Alkylsulfonylrest gewählt werden; und

(4) wenn $R^d$ eine 2-, 3- oder 4-Pyridylgruppe darstellt und $R^c$ einen disubstituierten Phenylrest, die Substituenten beide nicht aus einem Brom- oder Iodatom gewählt werden.

**8.** Verfahren nach Anspruch 1 zur Herstellung einer Verbindung, die gewählt wird aus: der Verbindung, bei der $R^a$ eine 4-Pyridylgruppe darstellt, $R^b$ eine 4-Fluorphenylgruppe, A eine $CH_2$-Grupope und C ein H-Atom; und der Verbindung, bei der $R^a$ eine 4-Pyridylgruppe darstellt, $R^b$ eine 4-Methoxyphenylgruppe, A eine $CH_2$-Gruppe und C ein H-Atom; und pharmazeutisch verträglichen Salzen davon.

**9.** Verfahren zur Herstellung eines Arzneimittels, das eine Verbindung der Formel (IC), wie in Anspruch 7 definiert, enthält, welches das Zusammenbringen der Verbindung mit einem pharmazeutisch verträglichen Träger umfaßt.

**10.** Verwendung einer Verbindung bei der Herstellung eines Medikamentes zur Behandlung einer mit dem 5-Lipoxygenase-Weg zusammenhängenden Erkrankung anders als, oder zusätzlich zu, rheumatoider Arthritis, Rheumatismus, Entzündung oder jedes anderen von Cyclooxygenase hervorgerufenen Krankheitszustands, wobei die Verbindung die Formel (IA) aufweist:

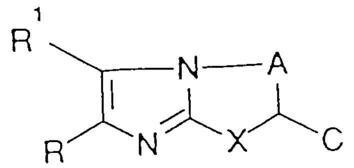

(IA)

wobei:

A und C gemäß Anspruch 1 definiert sind;

I. wenn X eine $CH_2$- oder $S(O)_n$-Gruppe darstellt und n den Wert 0, 1 oder 2 hat, die Reste $R^1$ und R unabhängig gewählt werden aus:

(a) einer Pyridylgruppe, mit der Maßgabe, daß, wenn einer oder beide der Reste R und $R^1$ eine Pyridylgruppe darstellen, X keine $CH_2$-Gruppe ist;

(b) einer Phenylgruppe;

(c) einem monosubstituierten Phenylrest, wobei der Substituent aus einem $C_{1-3}$-Alkoxyrest, einem Halogenatom, einer $CF_3$-Gruppe, einem $C_{1-3}$-Alkylthio-, $C_{1-4}$-Alkyl-, 2,2,2-Trihalogenethoxyrest, einer Prop-2-en-1-oxy-Gruppe, einem $C_{1-3}$-Alkylamino-, $C_{1-3}$-Dialkylaminorest, einer Cyanogruppe oder einem N-(Azacyclo-$C_{5-6}$-alkyl)-Rest gewählt wird;

(d) einem disubstituierten Phenylrest, wobei die Substituenten unabhängig aus einem $C_{1-4}$-Alkyl- oder $C_{1-3}$-Alkoxyrest gewählt werden, oder die Disubstituenten zusammen eine Methylendioxygruppe bilden;

(e) einer 3,4,5-Trimethoxyphenylgruppe;

oder einem pharmazeutisch verträglichen Salz davon, mit der Maßgabe, daß

(1) wenn X eine $CH_2$-Gruppe darstellt, beide Reste R und $R^1$ keinen Phenylrest darstellen, der in 2- oder 6-Stellung mit einem $C_{1-3}$-Alkylamino-, $C_{1-3}$-Dialkylamino- oder N-(Azacyclo-$C_{5-6}$-alkyl)-Rest substituiert ist;

(2) wenn einer der Reste R oder $R^1$ eine Cyanophenylgruppe darstellt, der andere eine Cyanophenylgruppe oder eine 4-Pyridylgruppe darstellen muß;

(3) wenn X eine $S(O)_n$-Gruppe darstellt und R oder $R^1$ einen Phenylrest, der in 2-, 3-, 5- oder 6-Stellung mit einem $C_{1-3}$-Alkylamino-,$C_{1-3}$-Dialkylamino- oder N-(Azacyclo-$C_{5-6}$-alkyl)-Rest substituiert ist, der andere eine 4-Pyridylgruppe darstellen muß; oder

II. wenn X eine $CH_2$-Gruppe darstellt und einer der Reste R oder $R^1$ eine Pyridylgruppe, der andere gewählt wird aus:

(a) einem monosubstituierten Phenylrest, wobei der Substituent aus einem Halogenatom, einem $C_{1-3}$-Alkoxy-, $C_{1-3}$-Alkylthio-, $C_{1-4}$-Alkyl-, $C_{1-3}$-Alkylsulfinyl-, $C_{1-3}$-Alkylsulfonyl-, $C_{1-3}$-Alkylamino-, $C_{1-3}$-Dialkylaminorest, einer $CF_3$-Gruppe, einem N-(Azacyclo-$C_{5-6}$-alkyl)-Rest, einer Prop-2-en-1-oxy-Gruppe oder einem 2,2,2-Trihalogenethoxyrest gewählt wird;

(b) einem disubstituierten Phenylrest, wobei die Substituenten gleich sind und aus einem Halogenatom, einem $C_{1-3}$-Alkoxy-, $C_{1-3}$-Alkylamino-, $C_{1-3}$-Dialkylamino-, N-(Azacyclo-$C_{5-6}$-alkyl)-, 2,2,2-Trihalogenethoxyrest, einer Prop-2-en-1-oxy-Gruppe gewählt werden, oder die Disubstituenten zusammen eine Methylendioxygruppe bilden, oder

(c) einem disubstituierten Phenylrest, wobei die Substituenten nicht gleich sind und unabhängig aus einem $C_{1-3}$-Alkylamino-, $C_{1-3}$-Dialkylamino- oder N-(Azacyclo-$C_{5-6}$-alkyl)-Rest gewählt werden, oder

(d) einem disubstituierten Phenylrest, wobei einer der Substituenten einen $C_{1-3}$-Alkoxyrest, eine Hydroxygruppe, einen 2,2,2-Trihalogenethoxyrest oder eine Prop-2-en-1-oxy-Gruppe darstellen muß und der andere Substituent unabhängig davon aus einem Halogenatom, einem $C_{1-3}$-Alkylamino-, N-($C_{1-3}$-Alkyl)-($C_{1-3}$-alkanamido)-, $C_{1-3}$-Dialkylaminorest, einer Aminogruppe oder einem N-(Azacyclo-$C_{5-6}$-alkyl)-Rest gewählt wird;

mit der Maßgabe, daß:

(1) wenn $R^1$ eine 2- oder 3-Pyridylgruppe darstellt und R einen monosubstituierten Phenylrest, der Substituent nicht aus einem Bromatom, Iodatom, $C_{1-3}$-Alkylthio-, $C_{1-3}$-Alkylsulfinyl- oder $C_{1-3}$-Alkylsulfonylrest gewählt wird;

(2) wenn $R^1$ eine 2- oder 3-Pyridylgruppe darstellt und R einen disubstituierten Phenylrest, die Disubstituenten beide nicht aus einem Brom- oder einem Iodatom gewählt werden;
(3) wenn R eine 2-, 3- oder 4-Pyridylgruppe darstellt und $R^1$ einen monosubstituierten Phenylrest, die Substituenten nicht aus einem Bromatom, einem Iodatom, einem $C_{1-3}$-Alkylthio-, $C_{1-3}$-Alkylsulfinyl- oder $C_{1-3}$-Alkylsulfonylrest gewählt werden; und
(4) wenn R eine 2-, 3- oder 4-Pyridylgruppe darstellt und $R^1$ einen disubstituierten Phenylrest, die Substituenten beide nicht aus einem Brom- oder Iodatom gewählt werden;

oder ein Hydrat davon, das eine Hydroxygruppe an dem Kohlenstoffatom aufweist, an dem der Rest R gebunden ist, wobei R keine Pyridylgruppe darstellt, $R^1$ einen Phenylrest darstellt, der in 2- oder 4-Stellung mit einem Halogenatom, einer $CF_3$-Gruppe oder einer Cyanogruppe substituiert ist, und X ein Schwefelatom darstellt;

oder ein pharmazeutisch verträgliches Salz davon.

**11.** Verwendung einer Verbindung der Formel (IA), gemäß Anspruch 10 definiert, bei der Herstellung eines Medikamentes zur Behandlung von Asthma oder Darmentzündung.

**Revendications**
**Revendications pour les Etats contractants suivants : BE, CH, DE, FR, GB, IT, LI, LU, NE, SE**

**1.** Composé de formule (IB) représentée par la structure:

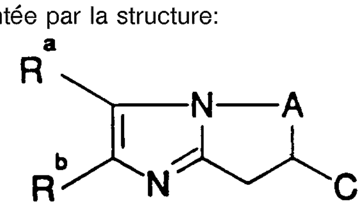

Formule (IB)

dans laquelle:
A est un groupe méthylène ou 1,2-éthanediyle, éventuellement substitué, par un groupe méthyle, éthyle, ou gem-diméthyle,
C est un atome d'hydrogène, un groupe méthyle, éthyle, ou gem-diméthyle; et

I.- un des groupes $R^a$ ou $R^b$ doit être choisi parmi les groupes 2-pyridyle, 3-pyridyle, 4-pyridyle, phényle monosubstitué dans lequel ledit substituant est choisi parmi les groupes dialkyl $C_{1-3}$ amino, alkyl $C_{1-3}$ amino, N-(azacycloalkyle $C_{5-6}$), cyano, 2,2,2-trihaloéthoxy, alcan $C_{1-3}$ amido, N-(alkyle $C_{1-3}$)-(alcan $C_{1-3}$ amido), ou prop-2-èn-1-oxy, phényle disubstitué dans lesquels lesdits substituants sont choisis indépendamment parmi les groupes alkyle $C_{1-4}$ ou alcoxy $C_{1-3}$, ou les disubstituants forment ensemble, un groupe méthylènedioxy; et l'autre groupe $R^a$ ou $R^b$, est choisi parmi les groupes:

1)- pyridyle;
2)- phényle;
3)- phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);
4)- phényle disubstitué dans lequel lesdits substituants sont choisis indépendamment parmi les groupes alkyle $C_{1-4}$ ou alcoxy $C_{1-3}$, ou les disubstituants, ensemble, forment un groupe méthylènedioxy, ou
5)- 3,4,5-triméthoxyphényle;

à condition que:

(a) lorsque l'un ou l'autre des groupes $R^a$ ou $R^b$ est un 4-fluorophényle, l'autre soit obligatoirement choisi parmi les groupes pyridyle, ou phényle substitué en position 4 par un groupe 2,2,2-trihaloéthoxy, ou prop-2-èn-1-oxy;
(b) $R^a$ et $R^b$ ne soient pas tous les deux un groupe phényle substitué en position 2, 3, 5, ou 6 par un groupe alkyl $C_{1-3}$ amino, dialkyl $C_{1-3}$ amino, cyano, ou N-(azacycloalkyle $C_{5-6}$);
(c) lorsque l'un des groupes $R^a$ ou $R^b$ est un groupe phényle disubstitué, l'autre soit obligatoirement un groupe 4-pyridyle;

(d) lorsque l'un des groupes $R^a$ ou $R^b$ est un groupe cyanophényle, l'autre soit obligatoirement un groupe cyanophényle ou 4-pyridyle;

(e) que $R^a$ et $R^b$ ne soient pas tous deux des groupes 3-pyridyle ou 2-pyridyle, ou

II.- un des groupes $R^a$ ou $R^b$ est un groupe pyridyle et l'autre est choisi parmi les groupes:

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(alkyle $C_{1-3}$)-(alcan $C_{1-3}$ amido), N-(azacycloalkyle $C_{5-6}$), prop2-èn-1-oxy, ou 2,2,2-trihaloéthoxy;

(b) phényle disubstitué dans lequel lesdits substituants sont identiques, et choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou hydroxy, ou les disubstituants ensemble, forment un groupe méthylènedioxy; ou

(c) phényle disubstitué dans lequel lesdits substituants ne sont pas identiques et sont choisis indépendamment parmi les groupes halo, alkyl $C_{1-3}$ amino, nitro, N-(alkyle $C_{1-3}$)-(alcan $C_{1-3}$ amido), dialkyl $C_{1-3}$ amino, ou N-(azacycloalkyle $C_{5-6}$); ou

(d) phényle disubstitué dans lequel un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que

- (1) lorsque $R^a$ est un groupe 2-, ou 3-pyridyle et que $R^b$ est un groupe phényle monosubstitué, le substituant soit choisi parmi des groupes autres que bromo, iodo, N-(alkyle $C_{1-3}$)-(alcan $C_{1-3}$ amido), alkyl $C_{1-3}$ thio, alkyl $C_{1-3}$ sulfinyle, ou alkyl $C_{1-3}$ sulfonyle;
- (2) lorsque $R^a$ est un groupe 2-, ou 3-pyridyle et que $R^b$ est un groupe phényle disubstitué, les disubstituants soient tous deux choisis parmi des groupes autres que bromo, iodo, amino, nitro, hydroxy, ou N-(alkyle $C_{1-3}$)-(alcan $C_{1-3}$ amido);
- (3) lorsque $R^b$ est un groupe 2-, 3-, ou 4-pyridyle, et que $R^a$ est un groupe phényle monosubstitué, les substituants soient choisis parmi des groupes autres que bromo, iodo, N-(alkyle $C_{1-3}$)-(alcan $C_{1-3}$ amido), alkyl $C_{1-3}$ thio, alkyl $C_{1-3}$ sulfinyle, ou alkyl $C_{1-3}$ sulfonyle; et
- (4) lorsque $R^b$ est un groupe 2-, 3-, ou 4-pyridyle, et que $R^a$ est un groupe phényle disubstitué, les substituants soient choisis tous deux parmi des groupes autres que bromo, iodo, amino, nitro, hydroxy, N-(alkyle $C_{1-3}$)-N-(alcan $C_{1-3}$ amido),

ou un sel de celui-ci, acceptable du point de vue pharmaceutique.

**2.** Composé suivant la revendication 1, dans lequel,

(a) $R^a$ est un groupe 4-pyridyle, $R^b$ est un groupe 4-fluorophényle, C est un atome d'hydrogène, et A est $CH_2$; ou

(b) $R^b$ est un groupe 4-méthoxyphényle, $R^a$ est un groupe 4-pyridyle, C est un atome d'hydrogène, et A est $CH_2$.

**3.** Composé suivant la revendication 1, de formule (IC) représentée par la structure:

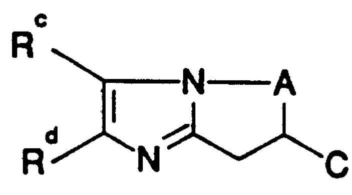

Formule (IC)

dans laquelle

A est un groupe méthylène ou 1,2-éthanediyle, éventuellement substitué, par un groupe méthyle, éthyle, ou gem-diméthyle,

C est un atome d'hydrogène, un groupe méthyle, éthyle, ou gem-diméthyle; et

I.- un des groupes $R^c$ ou $R^d$ doit être choisi parmi les groupes 2-pyridyle, 3-pyridyle, 4-pyridyle, phényle monosubstitué dans lequel ledit substituant est choisi parmi les groupes dialkyl $C_{1-3}$ amino,

alkyl $C_{1-3}$ amino, N-(azacycloalkyle $C_{5-6}$), cyano, 2,2,2-trihaloéthoxy, alcan $C_{1-3}$ amido, N-(alkyle $C_{1-3}$)-(alcan $C_{1-3}$ amido), ou prop-2-èn-1-oxy, phényle disubstitué dans lesquels lesdits substituants sont choisis indépendamment parmi les groupes alkyle $C_{1-3}$ ou alcoxy $C_{1-3}$, ou les disubstituants forment ensemble, un groupe méthylènedioxy; et l'autre groupe $R^c$ ou $R^d$, est choisi parmi les groupes:

1)- pyridyle;

2)- phényle,

3)- phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);

4)- phényle disubstitué dans lequel lesdits substituants sont choisis indépendamment parmi les groupes alkyle $C_{1-4}$ ou alcoxy $C_{1-3}$, ou les disubstituants forment ensemble, un groupe méthylènedioxy; ou

5)- 3,4,5-triméthoxyphényle;

à condition que:

(a) lorsque l'un ou l'autre des groupes $R^c$ ou $R^d$ est un groupe 4-fluorophényle, l'autre soit obligatoirement choisi parmi les groupes pyridyle, ou phényle substitué en position 4 par le groupe 2,2,2-trihaloéthoxy ou prop-2-èn-1-oxy:

(b) $R^c$ et $R^d$ ne soient pas tous deux phényle substitués en position 2, 3, 5, ou 6 par un groupe alkyl $C_{1-3}$ amino, dialkyl $C_{1-3}$ amino, ou N-(azacycloalkyle $C_{5-6}$);

(c) lorsque l'un ou l'autre des groupes $R^c$ ou $R^d$ est un groupe phényle disubstitué, l'autre soit obligatoirement un groupe 4-pyridyle;

(d) lorsque l'un ou des groupes $R^c$ ou $R^d$ est un groupe cyanophényle, l'autre soit obligatoirement un groupe cyanophényle ou 4-pyridyle;

(e) lorsque $R^d$ est un groupe alcan $C_{1-3}$ amido ou N-(alkyle $C_{1-3}$)-(alcan $C_{1-3}$ amido), $R^c$ soit obligatoirement un groupe 4-pyridyle;

(f) $R^c$ et $R^d$ ne soient pas tous deux des groupes 3-pyridyle ou 2-pyridyle; ou

II.- lorsque l'un des groupes $R^c$ ou $R^d$ est un groupe pyridyle, l'autre est choisi parmi les groupes:

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(azacycloalkyle $C_{5-6}$), prop-2-èn-1-oxy, ou 2,2,2-trihaloéthoxy;

(b) phényle disubstitué dans lequel lesdits substituants sont identiques, et sont choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou les disubstituants forment ensemble un groupe méthylènedioxy; ou

(c) phényle disubstitué dans lequel lesdits substituants ne sont pas identiques et sont choisis indépendamment parmi les groupes alkyl $C_{1-3}$ amino, dialkyl $C_{1-3}$ amino, ou N-(azacycloalkyle $C_{5-6}$), ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que:

- (1) lorsque $R^c$ est un groupe 2-, ou 3-pyridyle, et que $R^d$ est un groupe phényle monosubstitué, le substituant soit choisi parmi des groupes autres que bromo, iodo, alkyl $C_{1-3}$ thio, alkyl $C_{1-3}$ sulfinyle, ou alkyl $C_{1-3}$ sulfonyle;
- (2) lorsque $R^c$ est un groupe 2-, ou 3-pyridyle, et que $R^d$ est un groupe phényle disubstitué, les substituants soient choisis tous deux parmi des groupes autres que bromo ou iodo;
- (3) lorsque $R^d$ est un groupe 2-, 3-, ou 4-pyridyle, et que $R^c$ est un groupe phényle monosubstitué, les substituants soient choisis parmi des groupes autres que bromo, iodo, alkyl $C_{1-3}$ thio, alkyl $C_{1-3}$ sulfinyle, ou alkyl $C_{1-3}$ sulfonyle; et
- (4) lorsque $R^d$ est un groupe 2-, 3-, ou 4-pyridyle, et que $R^c$ est un groupe phényle disubstitué, les substituants soient tous deux, choisis parmi des groupes autres que bromo, ou iodo;

ou un sel de celui-ci, acceptable du point de vue pharmaceutique.

**4.** Composition pharmaceutique comprenant un véhicule ou un diluant, acceptable du point de vue pharmaceutique, et une quantité efficace d'un composé de formule (IC), non toxique, inhibiteur du mode d'action de la 5-lipoxygénase, suivant la revendication 3.

**5.** Composition suivant la revendication 4, sous une forme de dose unitaire adaptée à l'administration topique, parentérale, orale, ou par inhalation.

**6.** Composé de formule (IC) ou un sel de celui-ci, acceptable du point de vue pharmaceutique, suivant la revendication 3, pour l'utilisation dans le traitement de l'arthrite rhumatoïde chez l'animal.

**7.** Utilisation d'un composé dans la préparation d'un médicament pour le traitement d'une maladie concernée par le mode d'action de la 5-lipoxygénase, autre que, (ou s'ajoutant à), l'arthrite rhumatoïde, le rhumatisme, l'inflammation, ou tout autre état morbide impliquant la cyclooxygénase, ledit composé étant de formule (IA):

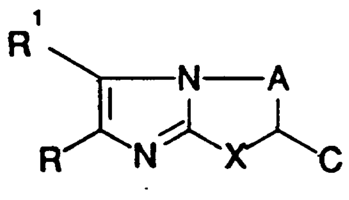

**Formule (IA)**

dans laquelle:
A et C sont tels que définis dans la revendication 1;

I.- lorsque X est un groupe $CH_2$ ou $S(O)_n$, et n est égal à 0, 1, ou 2: $R^1$ et R sont choisis indépendamment parmi les groupes

(a) pyridyle, à condition que, lorsque l'un ou l'autre des groupes R et $R^1$, ou les deux, sont des groupes pyridyle, X soit un autre groupe que $CH_2$:
(b) phényle
(c) phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);
(d) phényle disubstitué dans lequel lesdits substituants sont choisis indépendamment parmi les groupes alkyle $C_{1-4}$ ou alcoxy $C_{1-3}$, ou les disubstituants forment ensemble un groupe méthylènedioxy; ou
(e) 3,4,5-triméthoxyphényle;

ou un sel de celui-ci, acceptable du point de vue pharmaceutique, à condition que:

- (1) lorsque X est un groupe $CH_2$, R et $R^1$ soient tous deux, autres qu'un groupe phényle substitué en position 2 ou 6 par un groupe alkyl $C_{1-3}$ amino; dialkyl $C_{1-3}$ amino, ou N-(azacycloalkyle $C_{5-6}$);
- (2) lorsque l'un des groupes R ou $R^1$ est un groupe cyanophényle, l'autre soit obligatoirement un groupe cyanophényle ou 4-pyridyle;
- (3) lorsque X est un groupe $S(O)_n$, et que l'un des groupes R ou $R^1$ est un groupe phényle substitué en position 2, 3, 5, ou 6 par un groupe alkyl $C_{1-3}$ amino, dialkyl $C_{1-3}$ amino, ou N-(azacycloalkyle $C_{5-6}$), l'autre soit obligatoirement un groupe 4-pyridyle; ou

II.- lorsque X est un groupe $CH_2$ et que l'un des groupes R ou $R^1$ est un groupe pyridyle, l'autre est choisi parmi les groupes

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(azacycloalkyle $C_{5-6}$), prop-2-èn-1-oxy, ou 2,2,2-trihaloéthoxy;
(b) phényle disubstitué dans lequel lesdits substituants sont identiques et sont choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou les disubstituants forment ensemble un groupe méthylènedioxy; ou
(c) phényle disubstitué dans lequel lesdits substituants ne sont pas identiques et sont choisis indépendamment parmi les groupes alkyl $C_{1-3}$ amino, dialkyl $C_{1-3}$ amino, ou N-(azacycloalkyle $C_{5-6}$), ou
(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$

amino, amino, ou N-(azacycloalkyle $C_{5-6}$);

à condition que:

- (1) lorsque $R^1$ est un groupe 2-, ou 3-pyridyle, et que R est un groupe phényle monosubstitué, le substituant soit choisi parmi des groupes autres que bromo, iodo, alkyl $C_{1-3}$ thio, alkyl $C_{1-3}$ sulfinyle, ou alkyl $C_{1-3}$ sulfonyle;
- (2) lorsque $R^1$ est un groupe 2-, ou 3-pyridyle et que R est un groupe phényle disubstitué, les disubstituants soient tous deux choisis parmi des groupes autres que bromo ou iodo;
- (3) lorsque R est un groupe 2-, 3-, ou 4-pyridyle et $R^1$ est un groupe phényle monosubstitué, les substituants soient choisis parmi des groupes autres que bromo, iodo, alkyl $C_{1-3}$ thio, alkyl $C_{1-3}$ sulfinyle, ou alkyl $C_{1-3}$ sulfonyle; et
- (4) lorsque R est un groupe 2-, 3-, ou 4-pyridyle et $R^1$ est un groupe phényle disubstitué, les substituants soient tous deux choisis parmi des groupes autre que bromo ou iodo;

ou un hydrate de celui-ci ayant un groupe hydroxy attaché à l'atome de carbone auquel R est attaché, dans lequel R est un groupe autre que pyridyle, $R^1$ est un groupe phényle substitué en position 2 ou 4, par un groupe halo, $CF_3$ ou cyano, et X est un atome de soufre;

ou un sel de celui-ci, acceptable du point de vue pharmaceutique.

**8.** Composé de formule (E) représentée par la structure:

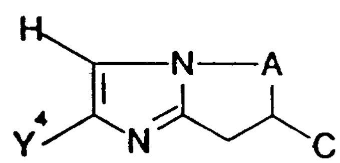

**Formule (E)**

dans laquelle

A et C sont tels que définis dans la revendication 1;

$Y^4$ est choisi parmi les groupes:

(a) phényle

(b) phényle monosubstitué dans lequel ledit substituant est choisi parmi les groupes halo, alcoxy $C_{1-3}$, alkyl $C_{1-3}$ thio, alkyle $C_{1-4}$, cyano, N-(alkyle $C_{1-3}$)-(alcan $C_{1-3}$ amido), alcan $C_{1-3}$ amido, alkyl $C_{1-3}$ amino, dialkyl $C_{1-3}$ amino, $CF_3$, N-(azacycloalkyle $C_{5-6}$), prop-2-èn-1-oxy, ou 2,2,2-trihaloéthoxy;

(c) phényle disubstitué dans lequel lesdits substituants sont identiques et sont choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, hydroxy, ou les disubstituants forment ensemble un groupe méthylènedioxy;

(d) phényle disubstitué dans lequel lesdits substituants ne sont pas identiques et sont choisis indépendamment parmi les groupes halo, alkyl $C_{1-3}$ amino, dialkyl $C_{1-3}$ amino, amino, N-(azacycloalkyle $C_{5-6}$), nitro, ou N-(alkyle $C_{1-3}$)-(alcan $C_{1-3}$ amido), ou

(e) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que, lorsque A est un groupe $CH_2CH_2$, C soit un atome d'hydrogène, et que $Y^4$ soit autre qu'un groupe 2,4-diméthoxyphényle ou 4-aminophényle;

ou un sel de celui-ci, acceptable du point de vue pharmaceutique.

**9.** Composé de formule (F) représentée par la structure:

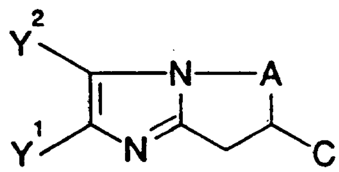

Formule (F)

dans laquelle:

A et C sont tels que définis dans la revendication 1; et

$Y^2$ est un groupe 4-(1,4-dihydro)pyridyle substitué par un groupe N-(alcanoyle $C_{1-8}$), N-(alcoxy $C_{1-8}$ carbonyle), N-(benzoyle), N-(phénoxycarbonyle), N-(phénylacétyle) ou N-(benzyloxycarbonyle);

$Y^1$ est choisi parmi les groupes:

(a) phényle ou phényle monosubstitué dans lequel ledit substituant est choisi parmi les groupes halo, alcoxy $C_{1-3}$, alkyl $C_{1-3}$ thio, alkyle $C_{1-4}$, $CF_3$, N-(alkyle $C_{1-3}$)-(alcan $C_{1-3}$ amido), dialkyl $C_{1-3}$ amino, N-(azacycloalkyle $C_{5-6}$), prop-2-èn-1-oxy, ou 2,2,2-trihaloéthoxy;

(b) phényle disubstitué dans lequel lesdits substituants sont identiques et sont choisis parmi les groupes halo, alcoxy $C_{1-3}$, dialkyl $C_{1-3}$ amino, N-(azacycloalkyle $C_{5-6}$), alkyl $C_{1-3}$ amino, 2,2,2-trihaloéthoxy, ou prop-2-èn-1-oxy, hydroxy, ou les disubstituants forment ensemble un groupe méthylènedioxy;

(c) phényle disubstitué dans lequel lesdits substituants ne sont pas identiques et sont choisis indépendamment parmi les groupes halo, nitro, dialkyl $C_{1-3}$ amino, ou N-(alkyle $C_{1-3}$)-(alcan $C_{1-3}$ amido), N-(azacycloalkyle $C_{5-6}$); ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

ou un sel de celui-ci, acceptable du point de vue pharmaceutique.

**10.** Composé de formule (G) représentée par la structure:

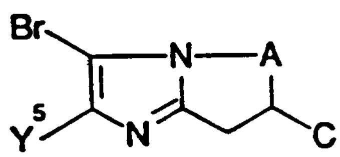

Formule (G)

dans laquelle:

A et C sont tels que définis dans la revendication 1;

$Y^5$ est choisi parmi les groupes:

(a) phényle ou phényle monosubstitué dans lequel ledit substituant est choisi parmi les groupes fluoro, chloro, alcoxy $C_{1-3}$, alkyle $C_{1-4}$, dialkyl $C_{1-3}$ amino, $CF_3$, alkyl $C_{1-3}$ amino, N-(azacycloalkyle $C_{5-6}$), prop-2-èn-1-oxy, ou 2,2,2-trihaloéthoxy;

(b) phényle disubstitué dans lequel lesdits substituants sont identiques et sont choisis parmi les groupes fluoro, chloro, alcoxy $C_{1-3}$, dialkyl $C_{1-3}$ amino, N-(azacycloalkyle $C_{5-6}$), 2,2,2-trihaloéthoxy, ou prop-2-èn-1-oxy, ou les disubstituants forment ensemble un groupe méthylènedioxy; ou

(c) phényle disubstitué dans lequel lesdits substituants ne sont pas identiques et sont choisis indépendamment parmi les groupes fluoro, chloro, alkyl $C_{1-3}$ amino, dialkyl $C_{1-3}$ amino, ou N-(azacycloalkyle $C_{5-6}$); ou

(d) phényle disubstitué dans lequel l'un desdits substituants doit être un groupe alcoxy $C_{1-3}$, 2,2,2-trihaloéthoxy, ou prop-2-èn-1-oxy, et l'autre substituant est choisi indépendamment parmi les groupes fluoro, chloro, alkyl $C_{1-3}$ amino, dialkyl $C_{1-3}$ amino, ou N-(azacycloalkyle $C_{5-6}$);

ou un sel de celui-ci, acceptable du point de vue pharmaceutique.

**11.** Composé de formule (H) représentée par la structure:

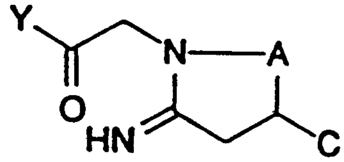

Formule (H)

dans laquelle:
A et C sont tels que définis dans la revendication 1;
Y est choisi parmi les groupes:

(a) phényle ou phénye monosubstitué, dans lequel ledit substituant est choisi parmi les groupes halo, alcoxy $C_{1-3}$, alkyl $C_{1-3}$ thio, alkyle $C_{1-4}$, alcan $C_{1-3}$ amido, dialkyl $C_{1-3}$ amino, $CF_3$, alkyl $C_{1-3}$ amino, N-(azacycloalkyle $C_{5-6}$) ou N-(alkyle $C_{1-3}$)-(alcan $C_{1-3}$ amido);
(b) phényle disubstitué dans lequel lesdits substituants sont identiques et sont choisis parmi les groupes halo, dialkyl $C_{1-3}$ amino, N-(azacycloalkyle $C_{5-6}$), ou alcoxy $C_{1-3}$, ou les disubstituants forment ensemble un groupe méthylènedioxy;
(c) phényle disubstitué dans lequel lesdits substituants ne sont pas identiques et sont choisis indépendamment parmi les groupes halo, nitro, alcan $C_{1-3}$ amido, dialkyl $C_{1-3}$ amino, ou N-(azacycloalkyle $C_{5-6}$); ou
(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, N-(alkyle $C_{1-3}$)-(alcan $C_{1-3}$ amido), dialkyl $C_{1-3}$ amino, ou N-(azacycloalkyle $C_{5-6}$);

à condition que, lorsque A est un groupe $CH_2CH_2$ et C est un atome d'hydrogène, Y soit un groupe autre que 2,4-diméthoxyphényle ou 4-aminophényle;
ou un sel de celui-ci, acceptable du point de vue pharmaceutique.

**12.** Procédé de préparation d'un composé de formule (IB) suivant la revendication 1, lequel procédé comprend:

(i) la réaction d'un composé de formule (G) suivant la revendication 11, avec un réactif alkyl $C_{1-5}$ lithium peur donner, par échange lithium-halogène, le réactif lithien correspondant;
(ii) l'addition au réactif lithien, d'éthèrate d'halogénure magnésium en excès, pour donner, par transmétallation, le réactif de Grignard correspondant;
(iii) l'addition du réactif de Grignard à un sel de N-acylpyridium pour donner le composé de formule (F) correspondant;
(iv) la désacylation/oxydation du composé de formule (F) pour donner le composé de formule (IB) correspondant.

**13.** Composé suivant la revendication 1, choisi parmi le groupe comprenant:

- le composé dans lequel $R^a$ est un groupe 4-pyridyle, $R^b$ est un groupe 4-fluorophényle, A est un groupe $CH_2$, et C est un atome d'hydrogène;
- et le composé dans lequel $R^a$ est un groupe 4-pyridyle, $R^b$ est un groupe 4-méthoxyphényle, A est un groupe $CH_2$, et C est un atome d'hydrogène,
- ou les sels de ceux-ci, acceptables du point de vue pharmaceutique.

**14.** Utilisation d'un composé de formule (IA) tel que défini dans la revendication 7, dans la préparation d'un médicament destiné au traitement de l'asthme ou de la maladie inflammatoire de l'intestin.

**Revendications pour les Etats contractants suivants : AT, GR, ES**

**1.** Procédé de préparation d'un composé de formule (IB):

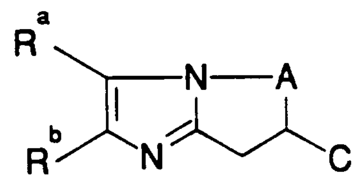

Formule (IB)

dans laquelle:

A est un groupe méthylène ou 1,2-éthanediyle, éventuellement substitué, par un groupe méthyle, éthyle, ou gem-diméthyle,

C est un atome d'hydrogène, un groupe méthyle éthyle, ou gem-diméthyle; et

I.- un des groupes $R^a$ ou $R^b$ doit être choisi parmi les groupes 2-pyridyle, 3-pyridyle, 4-pyridyle, phényle monosubstitué dans lequel ledit substituant est choisi parmi les groupes dialkyl $C_{1-3}$ amino, alkyl $C_{1-3}$ amino, N-(azacycloalkyle $C_{5-6}$), cyano, 2,2,2-trihaloéthoxy, alcan $C_{1-3}$ amido, N-(alkyle $C_{1-3}$)-(alcan $C_{1-3}$ amido), ou prop-2-èn-1-oxy, ou phényle disubstitué dans lesquels lesdits substituants sont choisis indépendamment parmi les groupes alkyle $C_{1-4}$ ou alcoxy $C_{1-3}$, ou les disubstituants forment ensemble un groupe méthylènedioxy; et l'autre groupe $R^a$ ou $R^b$, est choisi parmi les groupes:

1)- pyridyle;

2)- phényle;

3)- phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);

4)- phényle disubstitué dans lequel lesdits substituants sont choisis indépendamment parmi les groupes alkyle $C_{1-4}$ ou alcoxy $C_{1-3}$, ou les disubstituants forment ensemble un groupe méthylènedioxy; ou

5)- 3,4,5-triméthoxyphényle;

à condition que:

- (a) lorsque l'un ou l'autre des groupes $R^a$ ou $R^b$ est un 4-fluorophényle, l'autre soit obligatoirement choisi parmi les groupes pyridyle, ou phényle substitué en position 4 par un groupe 2,2,2-trihaloéthoxy, ou prop-2-èn-1-oxy;
- (b) $R^a$ et $R^b$ ne soient pas tous les deux un groupe phényle substitué en position 2, 3, 5, ou 6 par un groupe alkyl $C_{1-3}$ amino, dialkyl $C_{1-3}$, ou N-(azacycloalkyle $C_{5-6}$);
- (c) lorsque l'un des groupes $R^a$ ou $R^b$ est un groupe phényle disubstitué, l'autre soit obligatoirement un groupe 4-pyridyle;
- (d) lorsque l'un des groupes $R^a$ ou $R^b$ est un groupe cyanophényle, l'autre soit obligatoirement un groupe cyanophényle ou 4-pyridyle;
- (e) que $R^a$ et $R^b$ ne soient pas tous deux des groupes 3-pyridyle ou 2-pyridyle, ou

II.- un des groupes $R^a$ ou $R^b$ est un groupe pyridyle et l'autre est choisi parmi les groupes:

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(alkyle $C_{1-3}$)-(alcan $C_{1-3}$ amido), N-(azacycloalkyle $C_{5-6}$), prop-2-èn-1-oxy, ou 2,2,2-trihaloéthoxy;

(b) phényle disubstitué dans lequel lesdits substituants sont identiques, et choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou hydroxy, ou les disubstituants forment ensemble un groupe méthylènedioxy;

(c) phényle disubstitué dans lequel lesdits substituants ne sont pas identiques et sont choisis indépendamment parmi les groupes halo, alkyl $C_{1-3}$ amino, nitro, N-(alkyle $C_{1-3}$)-(alcan $C_{1-3}$ amido), dialkyl $C_{1-3}$ amino, amino, ou N-(azacycloalkyle $C_{5-6}$); ou

(d) phényle disubstitué dans lequel un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que

- (1) lorsque $R^a$ est un groupe 2-, ou 3-pyridyle et que $R^b$ est un groupe phényle monosubstitué, le substituant soit choisi parmi des groupes autres que bromo, iodo, N-(alkyle $C_{1-3}$)-(alcan $C_{1-3}$ amido), alkyl $C_{1-3}$ thio, alkyl $C_{1-3}$ sulfinyle, ou alkyl $C_{1-3}$ sulfonyle;
- (2) lorsque $R^a$ est un groupe 2-, ou 3-pyridyle et que $R^b$ est un groupe phényle disubstitué, les disubstituants soient tous deux choisis parmi des groupes autres que bromo, iodo, amino, nitro, hydroxy, ou N-(alkyle $C_{1-3}$)-(alcan $C_{1-3}$ amido);
- (3) lorsque $R^b$ est un groupe 2-, 3-, ou 4-pyridyle, et que $R^a$ est un groupe phényle monosubstitué, les substituants soient choisis parmi des groupes autres que bromo, iodo, N-(alkyle $C_{1-3}$)-(alcan $C_{1-3}$ amido), alkyl $C_{1-3}$ thio, alkyl $C_{1-3}$ sulfinyle, ou alkyl $C_{1-3}$ sulfonyle; et
- (4) lorsque $R^b$ est un groupe 2-, 3-, ou 4-pyridyle, et que $R^a$ est un groupe phényle disubstitué, les substituants soient choisis tous deux parmi des groupes autres que bromo, iodo, amino, nitro, hydroxy, N-(alkyle $C_{1-3}$)-N-(alcan $C_{1-3}$ amido),

ou un sel de celui-ci, acceptable du point de vue pharmaceutique,

A.- lorsqu'il est nécessaire de préparer un composé de formule (IB) dans lequel $R^a$ est un groupe 4-pyridyle et $R^b$ est un groupe autre que pyridyle, lequel procédé comprend la réaction d'un composé de formule (E) représentée par la structure:

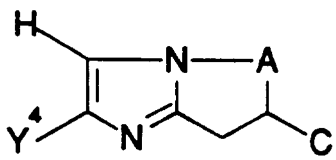

Formule (E)

dans laquelle

A et C sont tels que définis plus haut;

$Y^4$ est choisi parmi les groupes:

(a) phényle

(b) phényle monosubstitué dans lequel ledit substituant est choisi parmi les groupes halo, alcoxy $C_{1-3}$, alkyl $C_{1-3}$ thio, alkyle $C_{1-4}$, cyano, N-(alkyle $C_{1-3}$)-(alcan $C_{1-3}$ amido), alcan $C_{1-3}$ amido, alkyl $C_{1-3}$ amino, dialkyle $C_{1-3}$ amino, $CF_3$, N-(azacycloalkyle $C_{5-6}$), prop-2-èn-1-oxy, ou 2,2,2-trihaloéthoxy;

(c) phényle disubstitué dans lequel lesdits substituants sont identiques et sont choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, hydroxy, ou les disubstituants forment ensemble un groupe méthylènedioxy;

(d) phényle disubstitué dans lequel lesdits substituants ne sont pas identiques et sont choisis indépendamment parmi les groupes halo, alkyl $C_{1-3}$ amino, dialkyl $C_{1-3}$ amino, amino, N-(azacycloalkyle $C_{5-6}$), nitro, ou N-(alkyle $C_{1-3}$)-(alcan $C_{1-3}$ amido), ou

(e) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que, lorsque A est un groupe $CH_2CH_2$, C soit un atome d'hydrogène, et que $Y^4$ soit autre qu'un groupe 2,4-diméthoxyphényle ou 4-aminophényle;

ou un sel de celui-ci, acceptable du point de vue pharmaceutique, avec la pyridine et un halogénure d'acyle ou un ester haloacyle pour donner un composé de formule (F) représentée par la structure:

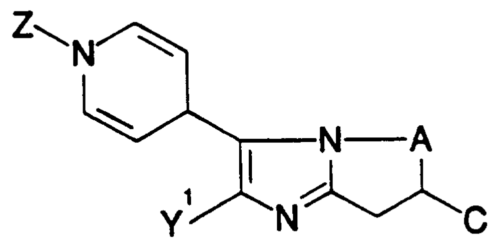

Formule (F)

dans laquelle:

A et C sont tels que définis plus haut; et

$Y^1$ est choisi parmi les groupes:

(a) phényle ou phényle monosubstitué dans lequel ledit substituant est choisi parmi les groupes halo, alcoxy $C_{1-3}$, alkyle $C_{1-3}$ thio, alkyle $C_{1-4}$, N-(alkyle $C_{1-3}$)-(alcan $C_{1-3}$ amido), dialkyl $C_{1-3}$ amino, $CF_3$, N-(azacycloalkyle $C_{5-6}$), alcan $C_{1-3}$ amido, alkyl $C_{1-3}$ amino, prop-2-èn-1-oxy, ou 2,2,2-trihaloéthoxy;

(b) phényle disubstitué dans lequel lesdits substituants sont identiques et sont choisis parmi les groupes halo, alcoxy $C_{1-3}$, dialkyl $C_{1-3}$ amino, alkyl $C_{1-3}$ amino, hydroxy, N-(azacycloalkyle $C_{5-6}$), 2,2,2-trihaloéthoxy, ou prop-2-èn-1-oxy, ou les disubstituants forment ensemble un groupe méthylènedioxy;

(c) phényle disubstitué dans lequel lesdits substituants ne sont pas identiques et sont choisis indépendamment parmi les groupes halo, nitro, dialkyl $C_{1-3}$ amino, ou N-(alkyle $C_{1-3}$)-(alcan $C_{1-3}$ amido), N-(azacycloalkyle $C_{5-6}$); ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

et Z est un groupe alcanoyle $C_{1-8}$, alcoxy $C_{1-8}$ carbonyle, benzoyle, phénoxycarbonyle, phénylacétyle, ou benzyloxycarbonyle;

suivie d'une désacylation/oxydation d'un composé de formule (F) pour donner un composé de formule (IB); et

B.- lorsqu'il est nécessaire de préparer un composé hydroxy de formule (IB), ledit procédé comprend la déméthylation d'un composé méthoxy de formule (I); la formule I étant représentée par la structure:

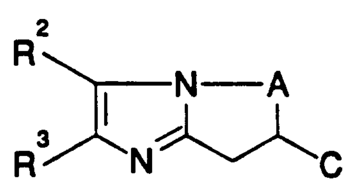

Formule (I)

dans laquelle:

A et C sont tels que définis plus haut; $R^2$ ou $R^3$ sont choisis indépendamment parmi les groupes:

a) - pyridyle

b) - phényle ou phényle monosubstitué dans lequel ledit substituant est choisi parmi les groupes dialkyl $C_{1-3}$ amino, alkyl $C_{1-3}$ amino, N-(azacycloalkyle $C_{5-6}$), cyano, 2,2,2-trihaloéthoxy, prop-2-èn-1-oxy, N-(alkyle $C_{1-3}$)-(alcan $C_{1-3}$ amido), alcan $C_{1-3}$ amido, amino, hydroxy, alkyl $C_{1-3}$ thio, alkyle $C_{1-4}$, halo, $CF_3$, alcoxy $C_{1-3}$, alkyl $C_{1-3}$ sulfinyle, ou alkyl $C_{1-3}$ sulfonyle,

c) - phényle disubstitué dans lequel lesdits substituants sont choisis indépendamment parmi les groupes alkyle $C_{1-4}$, alcoxy $C_{1-3}$, hydroxy, nitro, amino, halo, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou N-(alkyle $C_{1-3}$)-(alcan $C_{1-3}$ amido), ou les disubstituants forment ensemble un groupe méthylènedioxy; ou

d) - 3,4,5-triméthoxyphényle;

à condition que:

- (1) lorsque $R^3$ est un groupe cyanophényle, $R^2$ soit obligatoirement, ou bien un groupe cyanophényle, ou bien un groupe 4-pyridyle;

ou un sel de celui-ci, acceptable du point de vue pharmaceutique; avec HBr dans l'acide acétique, ou $BBr_3$ dans le dichlorométhane pour donner le composé hydroxy de formules (IB) correspondant, et

C.- lequel procédé comprend une O-alkylation du composé hydroxy de formule (I) tel que défini plus haut, lorsque A est un groupe $CH_2$ et au moins un des groupes $R^2$ ou $R^3$ est un groupe hydroxyphényle, pour donner le composé de formule (IB) correspondant, substitué par un groupe 2,2,2-trihaloéthoxyphényle, ou prop-2-èn-1-oxyphényle; et

D.- lequel procédé comprend l'acylation d'un composé aminophényle ou (alkyl $C_{1-3}$ amino)phényle de formule (IB) ou de formule (I);

lesquelles formule (IB) et formule (I) sont telles que définies ci-dessus, et dans lesquelles A est un groupe $CH_2CH_2$, et $R^2$ et $R^3$ de la formule (I) ne sont pas des groupes pyridyle, ou lorsque A est un groupe $CH_2$ et que l'un des groupes $R^2$ ou $R^3$ est un groupe pyridyle, avec l'halogénure d'acyle ou l'anhydride substitué en conséquence, dans la pyridine, pour donner le composé (alcan $C_{1-3}$ amido)phényle correspondant, ou le composé N-(alkyle $C_{1-3}$)-(alcan $C_{1-3}$ amido)phényle correspondant, de formules (IB); et

E.- lequel procédé comprend l'alkylation d'un composé alcan ($C_{1-3}$ amido)phényle de formules (I) telles que définies plus haut, au moyen d'un agent alkylant, en présence d'une base, pour donner le composé N-(alkyle $C_{1-3}$)-(alcan $C_{1-3}$ amido)phényle correspondant, de formule (IB); et

F.- lequel procédé comprend l'hydrolyse d'un composé (alcan $C_{1-3}$ amido)phényle ou N-(alkyle $C_{1-3}$)-(alcan $C_{1-3}$ amido)phényle de formules (I), telles que définies plus haut, pour donner le composé aminophényle ou (alkyl $C_{1-3}$ amino)phényle correspondant, de formule (IB); et

G.- lequel procédé comprend l'oxydation d'un composé de formule (IB) dans laquelle l'un ou les deux groupes $R^a$ et $R^b$ sont des groupes alkyle $C_{1-3}$ thio, au moyen de l'acide métachloroperbenzoïque pour donner le composé alkyl $C_{1-3}$ sulfinyle, alkyl $C_{1-3}$ sulfonyle correspondant, de formule (IB); et

H.- lequel procédé comprend la réduction d'un composé de formule (I) telle que définie plus haut, dans laquelle l'un, ou les deux groupes $R^2$ et $R^3$ de la formule (I), sont des groupes nitrophényle, au moyen de l'hydrogène gazeux sur catalyseur au palladium pour donner le composé aminophényle correspondant, de formule (IB); et

I.- lequel procédé comprend la cycloalkylation d'un composé de formule (I), telle que définie plus haut, dans laquelle l'un, ou les deux groupes $R^2$ et $R^3$ sont des groupes aminophényle, au moyen du dihalobutane ou du dihalopentane, selon le cas, en présence d'une base, pour donner le composé N-(azacycloalkyle $C_{5-6}$)phényle correspondant, de formule (IB); et

J.- lorsque doivent être préparés des composés de formules (IB) telles que définies plus haut, dans lesquelles les groupes $R^a$ et $R^b$ sont chacun un groupe (alkyl $C_{1-3}$ amino)phényle, lequel procédé comprend la réduction des composés alcan $C_{1-3}$ amido correspondants, au moyen du borane ou du complexe borane/sulfure de diméthyle, dans le tétrahydrofurane, pour donner les composé désirés, de formule (IB); et

K.- lorsque doivent être préparés des composés de formules (IB) telles que définies plus haut, dans lesquelles les groupes $R^a$ et $R^b$ sont chacun un groupe (dialkyl $C_{1-3}$ amino)phényle, lequel procédé comprend la réduction des composés N-(alkyle $C_{1-3}$)-(alcan $C_{1-3}$ amido) correspondants, au moyen du borane ou du complexe borane/sulfure de diméthyle, dans le tétrahydrofurane, pour donner les composé désirés, de formule (IB); et

L.- lequel procédé comprend:

(i) la réaction d'un composé de formule (G):

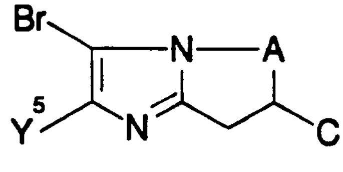

Formule (G)

dans laquelle:

A et C sont tels que définis plus haut;

$Y^5$ est choisi parmi les groupes:

(a) phényle ou phényle monosubstitué dans lequel ledit substituant est choisi parmi les groupes fluoro, chloro, alcoxy $C_{1-3}$, alkyle $C_{1-4}$, dialkyl $C_{1-3}$ amino, $CF_3$, alkyl $C_{1-3}$ amino, N-(azacycloalkyle $C_{5-6}$), prop-2-èn-1-oxy, ou 2,2,2-trihaloéthoxy;

(b) phényle disubstitué dans lequel lesdits substituants sont identiques et sont choisis parmi les groupes fluoro, chloro, alcoxy $C_{1-3}$, dialkyl $C_{1-3}$ amino, N-(azacycloalkyle $C_{5-6}$), 2,2,2-trihaloéthoxy, ou prop-2-èn-1-oxy, ou les disubstituants forment ensemble un groupe méthylènedioxy;

(c) phényle disubstitué dans lequel lesdits substituants ne sont pas identiques et sont choisis indépendamment parmi les groupes fluoro, chloro, alkyl $C_{1-3}$ amino, dialkyl $C_{1-3}$ amino, ou N-(azacycloalkyle $C_{5-6}$); ou

(d) phényle disubstitué dans lequel l'un desdits substituants doit être un groupe alcoxy $C_{1-3}$, 2,2,2-trihaloéthoxy, ou prop-2-èn-1-oxy, et l'autre substituant est choisi indépendamment parmi les groupes fluoro, chloro, alkyl $C_{1-3}$ amino, dialkyl $C_{1-3}$ amino, ou N-(azacycloalkyle $C_{5-6}$); ou un sel de celui-ci, acceptable du point de vue pharmaceutique,

avec un réactif alkyle $C_{1-5}$ lithium, pour donner, par échange lithium/halogène, le réactif lithien correspondant;

(ii) l'addition au réactif lithien, d'éthèrate d'halogénure magnésium en excès, pour donner, par transmétallation, le réactif de Grignard correspondant;

(iii) l'addition du réactif de Grignard à un sel de N-acylpyridium pour donner le composé de formule (F) correspondant;

(iv) la désacylation/oxydation du composé de formule (F) pour donner le composé de formule (IB) correspondant;

et ensuite, éventuellement, la conversion d'un composé de formule (IB) en un autre composé de formule (IB).

**2.** Procédé de préparation d'un composé de formule (E) suivant la revendication 1 ci-dessus;

A.- lequel procédé comprend le chauffage au reflux, dans l'eau, ou dans un solvant aqueux, d'un composé de formule (H), représentée par la structure:

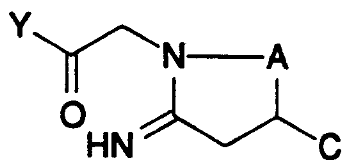

Formule (H)

dans laquelle:

A et C sont tels que définis dans la revendication 1;

Y est choisi parmi les groupes:

(a) phényle ou phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ amino, alcan $C_{1-3}$ amido, dialkyl $C_{1-3}$ amino, $CF_3$, N-(azacycloalkyle $C_{5-6}$), ou N-(alkyle $C_{1-3}$)-(alcan $C_{1-3}$ amido);

(b) phényle disubstitué dans lequel lesdits substituants sont identiques et sont choisis parmi les groupes halo, dialkyl $C_{1-3}$ amino, N-(azacycloalkyle $C_{5-6}$), ou alcoxy $C_{1-3}$, ou les disubstituants forment ensemble un groupe méthylènedioxy;

(c) phényle disubstitué dans lequel lesdits substituants ne sont pas identiques et sont choisis indépendamment parmi les groupes halo, nitro, alcan $C_{1-3}$ amido, dialkyl $C_{1-3}$ amino, ou N-(azacycloalkyle $C_{5-6}$); ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, N-(alkyle $C_{1-3}$)-(alcan $C_{1-3}$ amido), dialkyl $C_{1-3}$ amino, ou N-(azacycloalkyle $C_{5-6}$);

à condition que, lorsque A est un groupe $CH_2CH_2$ et que C est un atome d'hydrogène, Y soit un groupe autre que 2,4-diméthoxyphényle ou 4-aminophényle;

ou un sel de celui-ci, acceptable du point de vue pharmaceutique; et

B.- lequel procédé comprend l'alkylation d'un composé alcan ($C_{1-3}$ amido)phényle de formules (E) telles que définies plus haut, au moyen d'un agent alkylant, en présence d'une base, pour donner le composé N-(alkyle $C_{1-3}$)-(alcan $C_{1-3}$ amido)phényle correspondant, de formules (E); et

C.- lequel procédé comprend l'hydrolyse d'un composé (alcan $C_{1-3}$ amido)phényle ou N-(alkyle $C_{1-3}$)-(alcan $C_{1-3}$ amido)phényle de formules (E), telles que définies plus haut, pour donner le composé aminophényle ou (alkyl $C_{1-3}$ amino)phényle correspondant, de formules (E); et

D.- lequel procédé comprend la réduction d'un composé de formule (E) telle que définie plus haut, dans laquelle le groupe $Y^4$ de la formule (E), est un groupe nitrophényle, au moyen de l'hydrogène gazeux sur catalyseur au palladium pour donner le composé aminophényle correspondant, de formule (E); et

E.- lequel procédé comprend la cycloalkylation d'un composé de formules (E), telles que définies plus haut, dans lesquelles le groupe $Y^4$ est un groupe aminophényle, au moyen du dihalobutane ou du dihalopentane, selon le cas, en présence d'une base, pour donner le composé N-(azacycloalkyle $C_{5-6}$)phényle correspondant, de formule (E); et

F.- lequel procédé comprend une O-alkylation du composé hydroxy de formule (E) dans laquelle A est un groupe $CH_2$ et $Y^4$ est un groupe hydroxyphényle, ou A est un groupe $CH_2CH_2$ et $Y^4$ est un groupe hydroxyphényle, pour donner le composé correspondant de formule (E) substitué par un groupe 2,2,2-trihaloéthoxyphényle, ou prop-2-èn-1-oxyphényle; et

3. Procédé de préparation d'un composé de formule (H) suivant la revendication 2, comprenant la réaction d'un composé de formule (D):

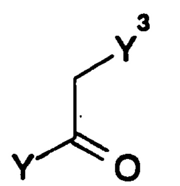

Formule (D)

dans laquelle Y est tel que défini pour la formule (H), et $Y^3$ est un groupe halo, avec un composé de formule (C):

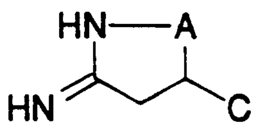

Formule (C)

dans laquelle A et C sont tels que définis pour la formule (H).

4. Procédé de préparation d'un composé de formule (G) suivant la revendication 1 ci-dessus:

A.- lequel procédé comprend la réaction d'un composé de formule (E) suivant la revendication 1, avec le brome; et

B.- lequel procédé comprend l'hydrolyse d'un composé (alcan $C_{1-3}$ amido)phényle ou N-(alkyle $C_{1-3}$)-(alcan $C_{1-3}$ amido)phényle de formule (G), telle que définie plus haut, pour donner le composé aminophényle ou (alkyl $C_{1-3}$ amino)phényle correspondant, de formule (G).

5. Procédé de préparation d'un composé de formule (F) suivant la revendication 1, qui comprend la réaction d'un composé de formule (E) suivant la revendication 1, avec la pyridine et un halogénure d'acyle ou un haloacyl ester.

6. Procédé suivant la revendication 1, de préparation d'un composé de formule (IB) dans laquelle:

(a) $R^a$ est un groupe 4-pyridyle, $R^b$ est un groupe 4-fluorophényle, A est un groupe $CH_2$, et C est un atome d'hydrogène; et

(b) $R^b$ est un groupe 4-méthoxyphényle, $R^a$ est un groupe 4-pyridyle, A est un groupe $CH_2$ et C est un atome d'hydrogène.

**7.** Procédé suivant la revendication 1, de préparation d'un composé de formule (IC)

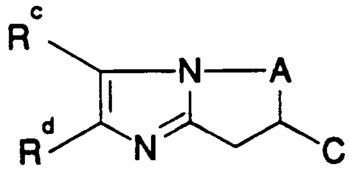

Formule (IC)

dans laquelle:

A et C sont tels que définis dans la revendication 1;

un des groupes $R^c$ ou $R^d$ doit être choisi parmi les groupes 2-pyridyle, 3-pyridyle, 4-pyridyle, phényle monosubstitué, ledit substituant étant choisi parmi les groupes dialkyl $C_{1-3}$ amino, alkyl $C_{1-3}$ amino, N-(azacycloalkyle $C_{5-6}$), cyano, 2,2,2-trihaloéthoxy, alcan $C_{1-3}$ amido, N-(alkyle $C_{1-3}$)-(alcan $C_{1-3}$ amido), ou prop-2-èn-1-oxy, ou phényle disubstitué dans lequel lesdits substituants sont choisis indépendamment parmi les groupes alkyle $C_{1-4}$, ou alcoxy $C_{1-3}$, ou les disubstituants forment ensemble, un groupe méthylèneoxy; et l'autre groupe $R^c$ ou $R^d$est choisi parmi les groupes:

1)- pyridyle;

2)- phényle;

3)- phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);

4)- phényle disubstitué dans lequel lesdits substituants sont choisis indépendamment parmi les groupes alkyle $C_{1-4}$ ou alcoxy $C_{1-3}$, ou les disubstituants, ensemble, forment un groupe méthylènedioxy; ou

5)- 3,4,5-triméthoxyphényle;

à condition que:

- (a) lorsque l'un ou l'autre des groupes $R^c$ ou $R^d$ est un groupe 4-fluorophényle, l'autre soit obligatoirement choisi parmi les groupes pyridyle, ou phényle substitué en position 4 par un groupe 2,2,2-trihaloéthoxy, ou prop-2-èn-1-oxy;
- (b) $R^c$ et $R^d$ ne soient pas tous les deux un groupe phényle substitué en position 2, 3, 5, ou 6 par un groupe alkyl $C_{1-3}$ amino, dialkyl $C_{1-3}$ amino, ou N-(azacycloalkyle $C_{5-6}$);
- (c) lorsque l'un des groupes $R^c$ ou $R^d$ est un groupe phényle disubstitué, l'autre soit obligatoirement un groupe 4-pyridyle;
- (d) lorsque l'un des groupes $R^c$ ou $R^d$ est un groupe cyanophényle, l'autre soit obligatoirement un groupe cyanophényle ou 4-pyridyle;
- (e) lorsque $R^d$ est un groupe alcan $C_{1-3}$ amido, ou N-(alkyle $C_{1-3}$)-(alcan $C_{1-3}$ amido), $R^c$ soit obligatoirement un groupe 4-pyridyle;
- (f) que $R^c$ et $R^d$ ne soient pas tous deux des groupes 3-pyridyle ou 2-pyridyle, ou

lorsque l'un des groupes $R^c$ ou $R^d$ est un groupe pyridyle, l'autre est choisi parmi les groupes:

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(azacycloalkyle $C_{5-6}$), prop-2-èn-1-oxy, ou 2,2,2-trihaloéthoxy;

(b) phényle disubstitué dans lequel lesdits substituants sont identiques et sont choisis parmi les groupes halo, alcoxy $C_{1-3}$, alkyl $C_{1-3}$ amino dialkyl $C_{1-3}$ amino, N-(azacycloalkyle $C_{5-6}$), 2,2,2-trihaloéthoxy, prop-2-èn-1-oxy, ou les disubstituants forment ensemble un groupe méthylènedioxy; ou

(c) phényle disubstitué dans lequel lesdits substituants ne sont pas identiques et sont choisis indépendamment parmi les groupes alkyl $C_{1-3}$ amino, dialkyl $C_{1-3}$ amino, ou N-(azacycloalkyle $C_{5-6}$); ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacyclo-alkyle $C_{5-6}$);

à condition que

- (1) lorsque $R^c$ est un groupe 2-, ou 3-pyridyle et que $R^d$ est un groupe phényle monosubstitué, le substituant soit choisi parmi des groupes autres que bromo iodo, alkyl $C_{1-3}$ thio, alkyl $C_{1-3}$ sulfinyle, ou alkyl $C_{1-3}$ sulfonyle;
- (2) lorsque $R^c$ est un groupe 2-, ou 3-pyridyle et que $R^d$ est un groupe phényle disubstitué, les disubstituants soient tous deux choisis parmi des groupes autres que bromo, ou iodo;
- (3) lorsque $R^d$ est un groupe 2-, 3-, ou 4-pyridyle, et que $R^c$ est un groupe phényle monosubstitué, les substituants soient choisis parmi des groupes autres que bromo, iodo, alkyl $C_{1-3}$ thio, alkyl $C_{1-3}$ sulfinyle, ou alkyl $C_{1-3}$ sulfonyle; et
- (4) lorsque $R^d$ est un groupe 2-, 3-, ou 4-pyridyle, et que $R^c$ est un groupe phényle disubstitué, les substituants soient choisis tous deux parmi des groupes autres que bromo, ou iodo.

8. Procédé suivant la revendication 1, de préparation d'un composé choisi parmi le groupe comprenant le composé dans lequel $R^a$ est un groupe pyridyle, $R^b$ est un groupe 4-fluorophényle, A est un groupe $CH_2$, et C est un atome d'hydrogène; et le composé dans lequel $R^a$ est un groupe 4-pyridyle, $R^b$ est un groupe 4-méthoxyphényle, A est un groupe $CH_2$ , et C est un atome d'hydrogène; et les sels de ceux-ci, acceptables du point de vue pharmaceutique.

9. Procédé de préparation d'une composition pharmaceutique contenant un composé de formule (IC) suivant la revendication 7, lequel procédé comprend la mise en association du composé et d'un véhicule, acceptable du point de vue pharmaceutique.

10. Utilisation d'un composé dans la préparation d'un médicament pour le traitement d'une maladie concernée par le mode d'action de la 5-lipoxygénase, autre que, (ou s'ajoutant à) l'arthrite rhumatoïde, le rhumatisme, l'inflammation, ou tout autre état morbide impliquant la cyclooxygénase, ledit composé étant de formule (IA):

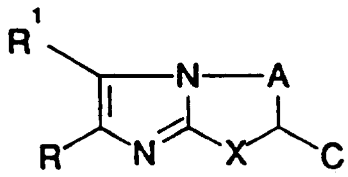

Formule (IA)

dans laquelle:
A et C sont tels que définis dans la revendication 1;

I.- lorsque X est un groupe $CH_2$ ou $S(O)_n$, et n est égal à 0, 1, ou 2: $R^1$ et R sont choisis indépendamment parmi les groupes
(a) pyridyle, à condition que, lorsque l'un ou l'autre des groupes R et $R^1$, ou les deux, sont des groupes pyridyle, X soit un autre groupe que $CH_2$ :
(b) phényle
(c) phényle monosubstitué, dans lequel ledit substituant est choisi parmi les groupes alcoxy $C_{1-3}$, halo, $CF_3$, alkyl $C_{1-3}$ thio, alkyle $C_{1-4}$, 2,2,2-trihaloéthoxy, prop-2-èn-1-oxy, alkyl $C_{1-3}$ amino, dialkyl $C_{1-3}$ amino, cyano, ou N-(azacycloalkyle $C_{5-6}$);
(d) phényle disubstitué dans lequel lesdits substituants sont choisis indépendamment parmi les groupes alkyle $C_{1-4}$ ou alcoxy $C_{1-3}$, ou les disubstituants forment ensemble un groupe méthylènedioxy;
(e) 3,4,5-triméthoxyphényle;
ou un sel de celui-ci, acceptable du point de vue pharmaceutique, à condition que:
- (1) lorsque X est un groupe $CH_2$, R et $R^1$ soient tous deux, autres qu'un groupe phényle substitué en position 2 ou 6 par un groupe alkyl $C_{1-3}$ amino; dialkyl $C_{1-3}$ amino, ou N-(azacycloalkyle $C_{5-6}$);
- (2) lorsque l'un des groupes R ou $R^1$ est un groupe cyanophényle, l'autre soit obligatoirement un groupe cyanophényle ou 4-pyridyle;
- (3) lorsque X est un groupe $S(O)_n$, et que l'un des groupes R ou $R^1$ est un groupe phényle substitué en position 2, 3, 5, ou 6 par un groupe alkyl $C_{1-3}$ amino, dialkyl $C_{1-3}$ amino, ou N-(azacycloalkyle $C_{5-6}$), l'autre soit obligatoirement un groupe 4-pyridyle; ou

II.- lorsque X est un groupe $CH_2$ et que l'un des groupes R ou $R^1$ est un groupe pyridyle, l'autre est choisi parmi les groupes:

(a) phényle monosubstitué dans lequel ledit substituant est choisi parmi les groupes halo, alcoxy $C_{1-3}$, alkyl $C_{1-3}$ thio, alkyle $C_{1-4}$, alkyl $C_{1-3}$ sulfinyle, alkyl $C_{1-3}$ sulfonyle, alkyl $C_{1-3}$ amino, dialkyl $C_{1-3}$ amino, $CF_3$, N-(azacycloalkyle $C_{5-6}$), prop-2-èn-1-oxy, ou 2,2,2-trihaloéthoxy;

(b) phényle disubstitué dans lequel lesdits substituants sont identiques et sont choisis parmi les groupes halo, alcoxy $C_{1-3}$, alkyl $C_{1-3}$ amino, dialkyl $C_{1-3}$ amino, N-(azacycloalkyle $C_{5-6}$), 2,2,2-trihaloéthoxy, prop-2-èn-1-oxy, ou les disubstituants forment ensemble un groupe méthylènedioxy; ou

(c) phényle disubstitué dans lequel lesdits substituants ne sont pas identiques et sont choisis indépendamment parmi les groupes alkyl $C_{1-3}$ amino, dialkyl $C_{1-3}$ amino, ou N-(azacycloalkyle $C_{5-6}$), ou

(d) phényle disubstitué dans lequel l'un desdits substituants doit être un groupe alcoxy $C_{1-3}$, hydroxy, 2,2,2-trihaloéthoxy, ou prop-2-èn-1-oxy, et l'autre substituant est choisi indépendamment parmi les groupes halo, alkyl $C_{1-3}$ amino, N-(alkyle $C_{1-3}$)-(alcan $C_{1-3}$ amido), dialkyl $C_{1-3}$ amino, amino, ou N-(azacycloalkyle $C_{5-6}$);

à condition que:

- (1) lorsque $R^1$ est un groupe 2-, ou 3-pyridyle, et que R est un groupe phényle monosubstitué, le substituant soit choisi parmi des groupes autres que bromo, iodo, alkyl $C_{1-3}$ thio, alkyl $C_{1-3}$ sulfinyle, ou alkyl $C_{1-3}$ sulfonyle;
- (2) lorsque $R^1$ est un groupe 2-, ou 3-pyridyle et que R est un groupe phényle disubstitué, les disubstituants soient tous deux choisis parmi des groupes autres que bromo ou iodo;
- (3) lorsque R est un groupe 2-, 3-, ou 4-pyridyle et $R^1$ est un groupe phényle monosubstitué, les substituants soient choisis parmi des groupes autres que bromo, iodo, alkyl $C_{1-3}$ thio, alkyl $C_{1-3}$ sulfinyle, ou alkyl $C_{1-3}$ sulfonyle; et
- (4) lorsque R est un groupe 2-, 3-, ou 4-pyridyle et $R^1$ est un groupe phényle disubstitué, les substituants soient tous deux choisis parmi des groupes autre que bromo ou iodo;

ou un hydrate de celui-ci ayant un groupe hydroxy attaché à l' atome de carbone auquel R est attaché, dans lequel R est un groupe autre que pyridyle, $R^1$ est un groupe phényle substitué en position 2 ou 4, par un groupe halo, $CF_3$ ou cyano, et X est un atome de soufre;

ou un sel de celui-ci, acceptable du point de vue pharmaceutique.

**11.** Utilisation d'un composé de formule (IA) suivant la revendication 10, dans la préparation d'un médicament destiné au traitement de l'asthme ou de la maladie inflammaroire de l'intestin.